# EUROPEAN PATENT APPLICATION

(11) **EP 4 516 893 A1**
(43) Date of publication of application: **05.03.2025**
(21) Application number: 22940132.8
(22) Date of filing: 27.04.2022
(51) Int. Cl.: C12N 5/00, C12M 1/34

(54) **SUBCULTURE TIMING DETERMINATION METHOD, SUBCULTURE TIMING DETERMINATION SYSTEM, AND CELL CULTURE SYSTEM**

(71) Applicant: Rorze Corporation, Fukuyama-shi, Hiroshima 720-2104 (JP); Rorze Lifescience Inc., Ibaraki 305-0854 (JP)
(72) Inventor: HARIMOTO Kenichi, Fukuyama-shi, Hiroshima 720-2104 (JP); YAMASAKI Yukito, Tsukuba-shi, Ibaraki 305-0854 (JP)
(74) Representative: Grünecker Patent- und Rechtsanwälte PartG mbB
(86) International application number: PCT/JP2022/019040
(87) International publication number: WO 2023/209853

(57) **Abstract**

It is required to perform subculture at an appropriate timing according to the actual state of cells. It is solved by the subculture timing determination method including an image-capturing step of photographing cells at a plurality of predetermined points of a culture vessel at a first photographing time point to obtain the cells as a cell image of each point of the plurality of predetermined points at the first photographing time point, a primary information obtaining step of obtaining primary information of the cells photographed in the cell image of each point at the first photographing time point, a statistical index value obtaining step of calculating a statistical index value of the primary information of the cells, and a determination step of performing determination that a photographing time point corresponding to a time point at which the statistical index value reaches a predetermined threshold value is a time point at which a subculture timing is reached.

## Description

### Technical Field

The present invention relates to a subculture timing determination method and a subculture timing determination system in cell culture, and a culture system using the same. The present invention particularly relates to a subculture timing determination method and a subculture timing determination system that determine the subculture timing of cells spreading or proliferating in a culture medium in a culture vessel, and a culture system using the same.

### Background Art

In the culture of cells represented by stem cells, cells are inoculated into a culture medium provided to a culture vessel such as a dish, and are cultured in an incubator. The inoculated cells adhere to a culture surface at a bottom surface portion of the culture medium in the culture vessel, and after a certain period of time, the inoculated cells cover the culture surface due to spreading and proliferation of the cells. Then, when contact between adjacent cells increases, spreading or proliferation stops. Thus, in order to compensate for the lack of space, the subculture of the cells are performed, that is, the cells are dispersed and re-inoculated into a fresh container, and the culture is continued. Therefore, in order to efficiently spread or proliferate cells, subculture at an appropriate timing is important. Generally, the timing is considered to be, for example, the time point at which the cell occupancy rate on a culture surface reaches approximately 80 percent of a confluent state. In addition, conventionally, a culture procedure has been adopted that performs subculture by empirically setting the stage expected to reach the time point as the stage for subculture.

### Citation List

### Patent Literature

PTL 1: Japanese Patent Application Laid-Open No. 2005-192485
PTL 2: Japanese Patent Application Laid-Open No. 2001-275659

### Summary of Invention

### Technical Problem

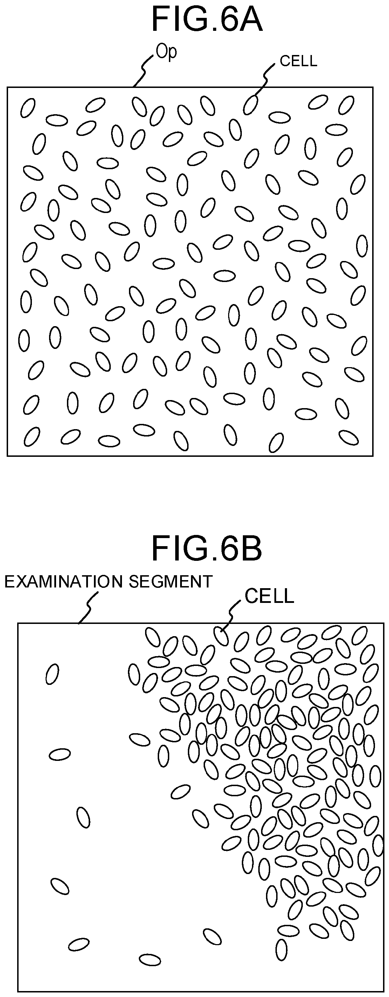

However, the rate of spreading or proliferation of cells varies from cell to cell, and due to, for example, differences in the states at the time when the cells are inoculated into a culture vessel, the subculture timing varies from culture vessel to culture vessel. FIG. 6A and FIG. 6B are diagrams illustrating distribution of cells in an observation position Op. For example, as illustrated in FIG. 6A, in the culture of cells, an ideal culture is to create a state in which there is little deviation in the inter-cell distance, and the inter-cell distance is gradually shortened on average so that there is no deviation in the inter-cell distance from the state in which the cells are inoculated. In such ideal culture, the time point at which the cell occupancy rate in a culture vessel reaches a predetermined value serves as an appropriate subculture timing. As an example of this time point, there is a time point at which the cell occupancy rate reaches approximately 80% of a confluent state. However, as described above, generally, the subculture timing has been defined to be the time point at which the cell occupancy rate is empirically estimated to reach a predetermined standard, without determining that the cell occupancy rate has reached the predetermined standard. This is due to the difficulty of easily recognizing the cell occupancy rate in a short period of time. As examples of technical procedures for solving this, PTL 1 and PTL 2 disclose procedures for objectively determining a subculture timing, which has been conventionally determined empirically, based on image data of cells in a culture medium.

PTL 1 discloses a culture state detection apparatus that calculates the cell density based on correlation between the run length of cells on a straight measurement line crossing image data of a culture medium and the cell density per unit area of the culture medium, and compares this with a predetermined determination value to determine the subculture timing. However, in PTL 1, although the image data as two dimensional data is used, since the straight measurement line crossing the image data is used, the data is one-dimensional data, and there is a problem in that the accuracy is reduced in accurately capturing the distribution of cells on a culture surface, and this reduction in accuracy has a problem in that recognition of a correct subculture timing is adversely affected.

On the other hand, PTL 2 discloses a culture method and a culture apparatus that calculate the number of cells, cell concentration, adherent cell occupancy area or adherent cell occupancy rate in a culture medium based on image data of cells on a culture surface in the culture medium, and determines the subculture timing based on the calculation result. However, in PTL 2, in determining the subculture timing, the image data of photographed cells is compared with the image data of cells that have been previously photographed to perform pattern recognition to find differences, thereby measuring the degree of spreading or proliferation of cells. Furthermore, in prediction of the timing of subsequent subculture, the subculture timing is predicted not only from image data, but also from culture medium consumption. That is, in PTL 2, since fixed point observation is not performed at a plurality of predetermined points in a culture surface, and the subculture timing is also predicted from the consumption of the culture medium, there are problems in that the calculation load of determination is increased, and the system configuration other than for images is increased. Therefore, it is required to determine an appropriate subculture timing only from image data of cells of a culture surface, with a simple configuration, and without using the state of a culture medium.

Furthermore, as illustrated in FIG. 6B, in a case where there is large deviation in the inter-cell distance in a culture surface, an appropriate subculture timing will come before the cell occupancy rate in the culture medium reaches approximately 80 percent. However, PTL 1 and PTL 2 do not involve recognizing the deviation of cells in the culture surface and using it for determination of the subculture timing. Therefore, it is required to recognize the deviation of cells in the culture surface to determine an appropriate subculture timing.

In addition, even if the time points at which culture is started are the same, the subculture timing is not necessarily consistent between culture vessels. Thus, if the subculture timing is uniformly defined to be a certain time, the optimal timing for subculture will be missed. Therefore, it is required to determine the subculture timing based on temporal changes of cells in a culture vessel at each predetermined time period, and to extract a plurality of predetermined points as elements for determining the timing of subculture to perform fixed point observation, so as to determine subculture at an appropriate subculture timing according to the actual state of cells in the culture surface.

### Solution to Problem

One aspect of the present invention is a subculture timing determination method of determining a subculture timing of cells spreading or proliferating in a culture medium in a culture vessel, the subculture timing determination method including an image-capturing step of photographing the cells at a plurality of predetermined points of the culture vessel at a first photographing time point to obtain the cells as a cell image of each point of the plurality of predetermined points at the first photographing time point, a primary information obtaining step of obtaining primary information of the cells photographed in the cell image of each point at the first photographing time point, a statistical index value obtaining step of calculating a statistical index value of the primary information of the cells, and a determination step of performing determination that a photographing time point corresponding to a time point at which the statistical index value reaches a predetermined threshold value is a time point at which the subculture timing is reached.

Another aspect of the present invention is a subculture timing determination method of determining a subculture timing of cells spreading or proliferating in a culture medium in a culture vessel, the subculture timing determination method including an image-capturing step of photographing the cells at a plurality of predetermined points of the culture vessel at a first photographing time point and a second photographing time point after the first photographing time point to obtain the cells as a cell image of each point of the plurality of predetermined points at each of the first photographing time point and the second photographing time point, a primary information obtaining step of obtaining primary information of the cells photographed in the cell image of the each point at each of the first photographing time point and the second photographing time point, a statistical index value obtaining step of calculating a statistical index value of the primary information at each of the first photographing time point and the second photographing time point, and a time-dependent change calculation step of calculating a time-dependent change index value from the statistical index value at the first photographing time point and the statistical index value at the second photographing time point, and the determination step includes a determination step of performing determination that a photographing time point corresponding to a time point at which the time-dependent change index value reaches a predetermined threshold value is a time point at which the subculture timing is reached.

A further aspect of the present invention is a subculture timing determination method of determining a subculture timing of cells spreading or proliferating in a culture medium in a culture vessel, the subculture timing determination method including an image-capturing step of photographing the cells at a plurality of predetermined points of the culture vessel at a first photographing time point and a second photographing time point after the first photographing time point to obtain the cells as a cell image of each point of the plurality of predetermined points at each of the first photographing time point and the second photographing time point, a primary information obtaining step of obtaining primary information of the cells photographed in the cell image of the each point at each of the first photographing time point and the second photographing time point, a time-dependent change calculation step of calculating a time-dependent change index value from the primary information at the first photographing time point and the primary information at the second photographing time point, and a statistical index value obtaining step of calculating a statistical index value of the time-dependent change index value of each of the first photographing time point and the second photographing time point, and the determination step includes a determination step of performing determination that a photographing time point corresponding to a time point at which the statistical index value reaches a predetermined threshold value is a time point at which the subculture timing is reached.

A further aspect of the present invention is a subculture timing determination method of determining a subculture timing of cells spreading or proliferating in a culture medium in a culture vessel, the subculture timing determination method including an image-capturing step of photographing the cells at a plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point, a primary information obtaining step of obtaining primary information of the cells photographed in the cell image of the each point at each of the first photographing time point and the second photographing time point and the third photographing time point, a statistical index value obtaining step of calculating a statistical index value of the primary information at each of the first photographing time point and the second photographing time point and the third photographing time point, a primary time-dependent change calculation step of calculating a primary time-dependent change index value of the statistical index value at the first photographing time point and the statistical index value at the second photographing time point, and a primary time-dependent change index value of the statistical index value at the second photographing time point and the statistical index value at the third photographing time point, a secondary time-dependent change calculation step of calculating a secondary time-dependent change index value from the primary time-dependent change index value of the first photographing time point and the second photographing time point, and the primary time-dependent change index value of the second photographing time point and the third photographing time point, and a determination step of performing determination that a photographing time point corresponding to a time point at which the secondary time-dependent change index value reaches a predetermined threshold value is a time point at which the subculture timing is reached.

A further aspect of the present invention is a subculture timing determination method of determining a subculture timing of cells spreading or proliferating in a culture medium in a culture vessel, the subculture timing determination method including an image-capturing step of photographing the cells at a plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point, a primary information obtaining step of obtaining primary information of the cells photographed in the cell image of the each point at each of the first photographing time point and the second photographing time point and the third photographing time point, a primary time-dependent change calculation step of calculating a primary time-dependent change index value of the primary information at the first photographing time point and the primary information at the second photographing time point, and a primary time-dependent change index value of the primary information at the second photographing time point and the primary information at the third photographing time point, a secondary time-dependent change calculation step of calculating a secondary time-dependent change index value from the primary time-dependent change index value of the first photographing time point and the second photographing time point, and the primary time-dependent change index value of the second photographing time point and the third photographing time point, a statistical index value obtaining step of calculating a statistical index value of the secondary time-dependent change index value at each of the first photographing time point and the second photographing time point and the third photographing time point, and a determination step of performing determination that a photographing time point corresponding to a time point at which the statistical index value reaches a predetermined threshold value is a time point at which the subculture timing is reached.

A further aspect of the present invention is a subculture timing determination method of determining a subculture timing of cells spreading or proliferating in a culture medium in a culture vessel, the subculture timing determination method including an image-capturing step of photographing the cells at a plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point, a primary information obtaining step of obtaining primary information of the cells photographed in the cell image of the each point at each of the first photographing time point and the second photographing time point and the third photographing time point, a primary time-dependent change calculation step of calculating a primary time-dependent change index value of the primary information at the first photographing time point and the primary information at the second photographing time point, and a primary time-dependent change index value of the primary information at the second photographing time point and the primary information at the third photographing time point, a statistical index value obtaining step of calculating a statistical index value of the primary time-dependent change index value at each of the first photographing time point and the second photographing time point and the third photographing time point, a secondary time-dependent change calculation step of calculating a secondary time-dependent change index value from the statistical index value of the first photographing time point and the statistical index value of the second photographing time point, and the statistical index value of the second photographing time point and the statistical index value of the third photographing time point, and a determination step of performing determination that a photographing time point corresponding to a time point at which the secondary time-dependent change index value reaches a predetermined threshold value is a time point at which the subculture timing is reached.

A further aspect of the present invention is a subculture timing determination system that determines a subculture timing of cells spreading or proliferating in a culture medium in a culture vessel, the subculture timing determination system including image-capturing means for photographing cells of a culture surface of each point of a plurality of predetermined points of the culture medium of the culture vessel to obtain the cells as cell images, and a processing apparatus configured to obtain primary information of the cells photographed in the cell images, wherein the image-capturing means photographs the cells at the plurality of predetermined points of the culture vessel at a first photographing time point to obtain the cells as a cell image of each point of the plurality of predetermined points at the first photographing time point, wherein the processing apparatus obtains primary information of the cells photographed in the cell image of the each point at the first photographing time point to calculate a statistical index value of the primary information of the cells, and wherein the processing apparatus performs determination that a photographing time point corresponding to a time point at which the statistical index value reaches a predetermined threshold value is a time point at which the subculture timing is reached.

A further aspect of the present invention is a subculture timing determination system that determines a subculture timing of cells spreading or proliferating in a culture medium in a culture vessel, the subculture timing determination system including image-capturing means for photographing cells of a culture surface of each point of a plurality of predetermined points of the culture medium of the culture vessel to obtain the cells as cell images, and a processing apparatus configured to obtain primary information of the cells photographed in the cell images, wherein the image-capturing means photographs the cells at the plurality of predetermined points of the culture vessel at a first photographing time point and a second photographing time point after the first photographing time point to obtain the cells as a cell image of each point of the plurality of predetermined points at each of the first photographing time point and the second photographing time point, wherein the processing apparatus obtains primary information of the cells photographed in the cell image of the each point at each of the first photographing time point and the second photographing time point to calculate a statistical index value of the primary information at each of the first photographing time point and the second photographing time point, and calculate a time-dependent change index value from the statistical index value at the first photographing time point and the statistical index value at the second photographing time point, and wherein the processing apparatus performs determination that a photographing time point corresponding to a time point at which the time-dependent change index value reaches a predetermined threshold value is a time point at which the subculture timing is reached.

A further aspect of the present invention is a subculture timing determination system that determines a subculture timing of cells spreading or proliferating in a culture medium in a culture vessel, the subculture timing determination system including image-capturing means for photographing cells of a culture surface of each point of a plurality of predetermined points of the culture medium of the culture vessel to obtain the cells as cell images, and a processing apparatus configured to obtain primary information of the cells photographed in the cell images, wherein the image-capturing means photographs the cells at the plurality of predetermined points of the culture vessel at a first photographing time point and a second photographing time point after the first photographing time point to obtain the cells as a cell image of each point of the plurality of predetermined points at each of the first photographing time point and the second photographing time point, wherein the processing apparatus obtains primary information of the cells photographed in the cell image of the each point at each of the first photographing time point and the second photographing time point to calculate a time-dependent change index value from the primary information at the first photographing time point and the primary information at the second photographing time point, and calculate a statistical index value of the time-dependent change index value at each of the first photographing time point and the second photographing time point, and wherein the processing apparatus performs determination that a photographing time point corresponding to a time point at which the statistical index value reaches a predetermined threshold value is a time point at which the subculture timing is reached.

A further aspect of the present invention is a subculture timing determination system that determines a subculture timing of cells spreading or proliferating in a culture medium in a culture vessel, the subculture timing determination system including image-capturing means for photographing cells of a culture surface of each point of a plurality of predetermined points of the culture medium of the culture vessel to obtain the cells as cell images, and a processing apparatus configured to obtain primary information of the cells photographed in the cell images, wherein the image-capturing means photographs the cells at the plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point, wherein the processing apparatus obtains primary information of the cells photographed in the cell image of the each point at each of the first photographing time point and the second photographing time point and the third photographing time point to calculate a statistical index value of the primary information at each of the first photographing time point and the second photographing time point and the third photographing time point, calculate a primary time-dependent change index value of the statistical index value at the first photographing time point and the statistical index value at the second photographing time point, and a primary time-dependent change index value of the statistical index value at the second photographing time point and the statistical index value at the third photographing time point, and calculate a secondary time-dependent change index value from the primary time-dependent change index value of the first photographing time point and the second photographing time point, and the primary time-dependent change index value of the second photographing time point and the third photographing time point, and wherein the processing apparatus performs determination that a photographing time point corresponding to a time point at which the secondary time-dependent change index value reaches a predetermined threshold value is a time point at which the subculture timing is reached.

A further aspect of the present invention is a subculture timing determination system that determines a subculture timing of cells spreading or proliferating in a culture medium in a culture vessel, the subculture timing determination system including image-capturing means for photographing cells of a culture surface of each point of a plurality of predetermined points of the culture medium of the culture vessel to obtain the cells as cell images, and a processing apparatus configured to obtain primary information of the cells photographed in the cell images, wherein the image-capturing means photographs the cells at the plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point, obtain primary information of the cells photographed in the cell image of the each point at each of the first photographing time point and the second photographing time point and the third photographing time point, calculate a primary time-dependent change index value of the primary information at the first photographing time point and the primary information at the second photographing time point, and a primary time-dependent change index value of the primary information at the second photographing time point and the primary information at the third photographing time point, calculate a secondary time-dependent change index value from the primary time-dependent change index value of the first photographing time point and the second photographing time point, and the primary time-dependent change index value of the second photographing time point and the third photographing time point, and calculate a statistical index value of the secondary time-dependent change index value at each of the first photographing time point and the second photographing time point and the third photographing time point, and wherein the processing apparatus performs determination that a photographing time point corresponding to a time point at which the statistical index value reaches a predetermined threshold value is a time point at which the subculture timing is reached.

A further aspect of the present invention is a subculture timing determination system that determines a subculture timing of cells spreading or proliferating in a culture medium in a culture vessel, the subculture timing determination system including image-capturing means for photographing cells of a culture surface of each point of a plurality of predetermined points of the culture medium of the culture vessel to obtain the cells as cell images, and a processing apparatus configured to obtain primary information of the cells photographed in the cell images, wherein the image-capturing means photographs the cells at the plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point, wherein the processing apparatus obtains primary information of the cells photographed in the cell image of the each point at each of the first photographing time point and the second photographing time point and the third photographing time point, calculates a primary time-dependent change index value of the primary information at the first photographing time point and the primary information at the second photographing time point, and a primary time-dependent change index value of the primary information at the second photographing time point and the primary information at the third photographing time point, calculates a statistical index value of the primary time-dependent change index value at each of the first photographing time point and the second photographing time point and the third photographing time point, and calculates a secondary time-dependent change index value from the statistical index value of the first photographing time point and the statistical index value of the second photographing time point, and the statistical index value of the second photographing time point and the statistical index value of the third photographing time point, and wherein the processing apparatus performs determination that a photographing time point corresponding to a time point at which the secondary time-dependent change index value reaches a predetermined threshold value is a time point at which the subculture timing is reached.

### Advantageous Effects of Invention

According to the present invention, an appropriate subculture timing can be determined in the culture of cells.

### Brief Description of Drawings

FIG. 1A illustrates an external schematic configuration of a cell culture system of the present invention.
FIG. 1B illustrates an example of the functional block diagram of the cell culture system of the present invention.
FIG. 1C illustrates another example of the functional block diagram of the cell culture system of the present invention.
FIG. 1D illustrates a further example of the functional block diagram of the cell culture system of the present invention.
FIG. 2A illustrates an outline of the flow of a culture process in the cell culture system of the present invention.
FIG. 2B illustrates an outline of an entire processing flow of a subculture determination process of the present invention.
FIG. 2C illustrates an external schematic configuration of a subculture timing determination unit of the present invention.
FIG. 2D illustrates a concept of a photographed image photographed by image-capturing means of the subculture timing determination unit of the present invention.
FIG. 2E illustrates a temporal concept of a photographing time point of an image obtained in the subculture timing determination unit of the present invention.
FIG. 2F illustrates a flow of a determination data generation process in a first embodiment of the present invention.
FIG. 3A illustrates a flow of a determination data generation process in a first example of a second embodiment of the present invention.
FIG. 3B illustrates a flow of a determination data generation process in a second example of the second embodiment of the present invention.
FIG. 3C illustrates an example of a concept of the selection of a notable photographing time point in the nearest comparison method of the second embodiment of the present invention.
FIG. 3D illustrates another example of the concept of the selection of the notable photographing time point in the nearest comparison method of the second embodiment of the present invention.
FIG. 3E illustrates an example of the concept of the selection of the notable photographing time point in the reference comparison method of the second embodiment of the present invention.
FIG. 3F illustrates an example of a concept of the generation of a time-dependent change index value in the second embodiment of the present invention.
FIG. 3G illustrates another example of a concept of the generation of a specific growth rate as a time-dependent change index value and a multiplying time in the second embodiment of the present invention.
FIG. 4A illustrates a flow of a determination data generation process in a first example of a third embodiment of the present invention.
FIG. 4B illustrates a flow of a determination data generation process in a second example of the third embodiment of the present invention.
FIG. 4C illustrates a flow of a determination data generation process in a third example of the third embodiment of the present invention.
FIG. 4D illustrates an example of a concept of the selection of a notable photographing time point in the nearest comparison method of the third embodiment of the present invention.
FIG. 4E illustrates another example of the concept of the selection of a notable photographing time point in the nearest comparison method of the third embodiment of the present invention.
FIG. 4F illustrates an example of a concept of the selection of a notable photographing time point in the reference comparison method of the third embodiment of the present invention.
FIG. 4G illustrates another example of the concept of the selection of a notable photographing time point in the reference comparison method of the third embodiment of the present invention.
FIG. 4H illustrates a concept of the generation of a difference change amount and a rate of amount of change as a primary time-dependent change index value and a secondary time-dependent change index value in the third embodiment of the present invention.
FIG. 4I illustrates a concept of the generation of a rate of change per unit time as a primary time-dependent change index value and a difference in rate of change as a secondary time-dependent change index value in the third embodiment of the present invention.
FIG. 4J illustrates a concept of a specific growth rate as a primary time-dependent change index value and a multiplying time in the third embodiment of the present invention.
FIG. 5A illustrates an example of a concept of variation of selection of a notable photographing time point in a fourth embodiment of the present invention.
FIG. 5B illustrates a concept of the reference value integrating method in the fourth embodiment of the present invention.
FIG. 5C illustrates a concept of the matrix aging method in the fourth embodiment of the present invention.
FIG. 5D illustrates a concept of the matrix aging method in a fifth embodiment of the present invention.
FIG. 5E is a concept of the matrix aging method in the fifth embodiment of the present invention.
FIG. 6A is a diagram schematically illustrating the distribution of cells in an observation position Op, and illustrates a case where there is no deviation in the distribution of cells.
FIG. 6B is a diagram schematically illustrating the distribution of cells in the observation position Op, and illustrates a case where there is a large deviation in the distribution of cells.

### Description of Embodiments

### [First embodiment]

### (Cell culture system)

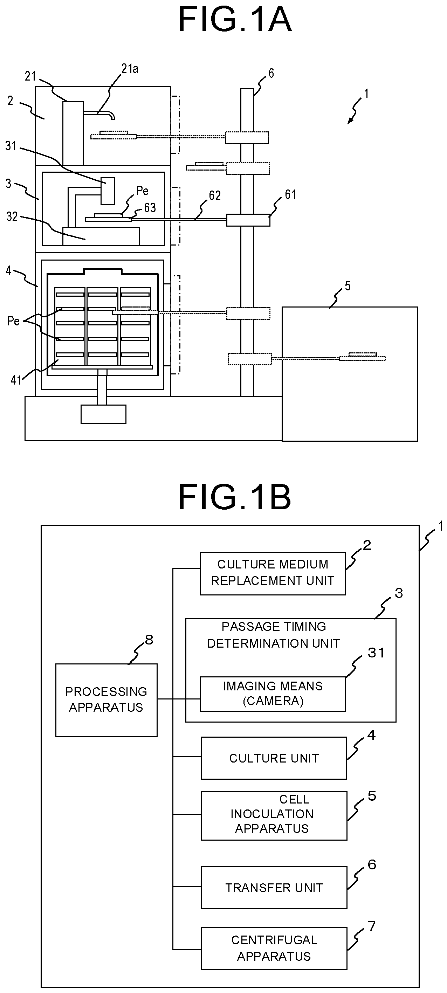

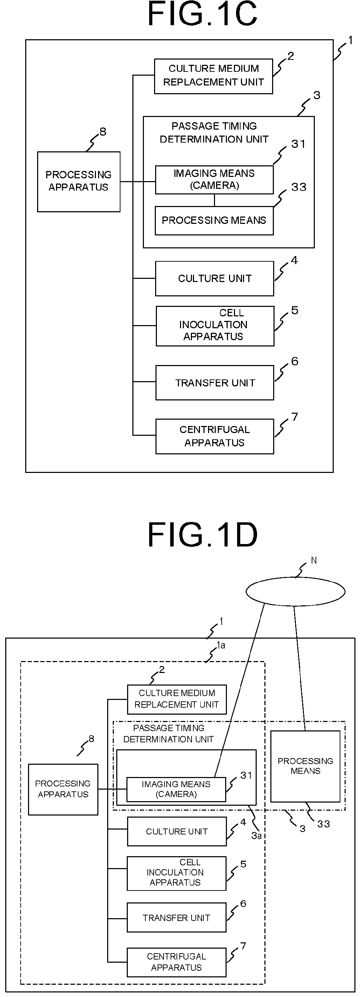

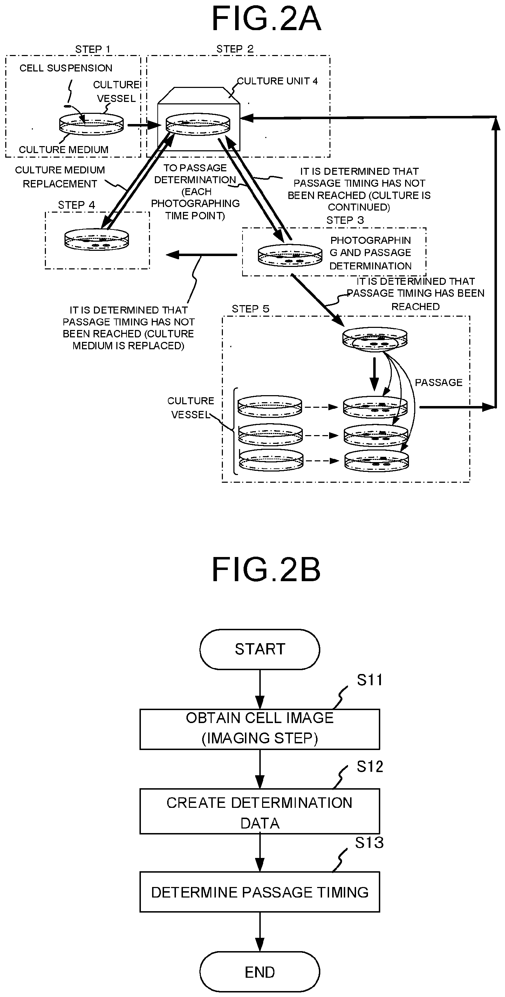

Referring to FIG. 1A to FIG. 2F, a subculture timing determination method and a subculture timing determination system in a first embodiment of the present invention will be described. Although the present invention is particularly suitable for subculture in culture of adherent cells in a culture vessel, the present invention is not necessarily limited to adherent cells. Timing of subculture is realized by a subculture timing determination unit 3, which is a subculture timing determination system, and a cell culture system 1 including the subculture timing determination unit 3. Hereinafter, subculture timing determination in the first embodiment will be described through the subculture timing determination unit 3 and the cell culture system 1. FIG. 1A is an external schematic diagram of the cell culture system 1. FIG. 1B illustrates an example of the functional block diagram illustrating electrical connections of the cell culture system 1. FIG. 1C and FIG. 1D illustrate other examples of the functional block diagram illustrating electrical connections of the cell culture system 1. FIG. 2A is a diagram illustrating an outline of the flow of a culture subculture process in the cell culture system 1 of the present invention. FIG. 2B is a diagram illustrating the flow of a subculture determination process in the first embodiment.

The cell culture system 1 includes, for example, as illustrated in FIG. 1B, an internal unit of a culture medium unit 2, a subculture timing determination unit 3, a culture unit 4, which is an incubator, a cell inoculation apparatus 5, a transfer unit 6, a centrifugal apparatus 7, and a processing apparatus 8. The cell culture system 1 does not necessarily need to include all of these units, and may include at least a part of these units.

In the cell culture system 1, a unit group (the culture medium unit 2, the subculture timing determination unit 3, the culture unit 4, the cell inoculation apparatus 5, the transfer unit 6, and the centrifugal apparatus 7) constituting the cell culture system 1 and the processing apparatuses 8 are electrically connected, so that the processing apparatus 8 can transmit and receive signals to and from these units. The culture medium unit 2 is an apparatus that provides a culture medium into a culture vessel, performs removal and collection of an old culture medium for culture medium replacement, and provides a fresh culture medium. The subculture timing determination unit 3 is an apparatus that performs determination of subculture timing of the culture vessel, and includes a camera 31, which is image-capturing means, inside the subculture timing determination unit 3. The culture unit 4 is an apparatus that stores a culture vessel in an internal chamber, and maintains the inside of the chamber at a predetermined temperature. The cell inoculation apparatus 5 is an apparatus that inoculates cells by, for example, dropping cell suspension into the culture vessel at an initial stage or at the time of subculture. The transfer unit 6 is an apparatus that moves the culture vessel in the unit group. The centrifugal apparatus is an apparatus that performs separation for collecting cells peeled from the culture vessel, at the time of subculture of the culture vessel.

For example, as shown in FIG. 1B, the processing apparatus 8 transmits a control signal to each of the culture medium unit 2, the subculture timing determination unit 3, the culture unit 4, the cell inoculation apparatus 5, the transfer unit 6, and the centrifugal apparatus 7 for controlling each of these, so as to control the operation of each of these. In addition, the processing apparatus 8 receives signals of respective states from the culture medium unit 2, the subculture timing determination unit 3, the culture unit 4, the cell inoculation apparatus 5, the transfer unit 6, and the centrifugal apparatus 7, respectively.

The processing apparatus 8 controls the unit group (the culture medium unit 2, the subculture timing determination unit 3, the culture unit 4, the cell inoculation apparatus 5, the transfer unit 6, the centrifugal apparatus 7) constituting the cell culture system 1 of the present invention, and performs reception and sending of signals required for the control. Additionally, in addition to this, the processing apparatus 8 can receive a signal of data detected in each unit. That is, the processing apparatus 8 can also receive data (for example, the number of cells, and the like) detected in the culture medium unit 2 and the subculture timing determination unit 3. In addition, as will be described later, the processing apparatus 8 can also perform processing of data of each unit, particularly in the subculture timing determination unit 3, to perform required determination.

Subsequently, referring to FIG. 2A, the outline functions of each unit and a culture subculture process of the present invention will be described. First, in a cell inoculation process, which is STEP1, a culture medium including cells is formed in an evaluation culture vessel Pe. That is, here, cell suspension containing cells is provided into the evaluation culture vessel Pe, or a culture medium is provided into the evaluation culture vessel Pe to inoculate cells by the cell inoculation apparatus 5. For example, the cell suspension is provided to a flat culture vessel, such as a dish or a petri dish, by using a dispensing tube 21a of a dispensing machine 21, or after providing the culture medium, the cell suspension is provided to the culture medium to produce the evaluation culture vessel Pe.

As a culture process, which is STEP2, the evaluation culture vessel Pe in which the cells have been inoculated in STEP1 is placed on a shelf 41 in the culture unit 4, and is stored at constant temperature for a predetermined time period. During this time period, the cells in the evaluation culture vessel Pe are attached to a bottom portion of the evaluation culture vessel Pe, and spread or proliferate within the evaluation culture vessel Pe including the culture medium. When a predetermined time period elapses, the evaluation culture vessel Pe performs a subculture timing determination process, which is STEP3. The processing apparatus 8 performs the timing count of the predetermined time period, picks out the evaluation culture vessel Pe, which is a target of determination, from the culture units 4 to the transfer unit 6, and sends a control signal so that the evaluation culture vessel Pe is transferred to the subculture timing determination unit 3.

In STEP3, the evaluation culture vessel Pe is picked out from the culture unit 4 and is transferred to the subculture timing determination unit 3, and it is determined whether a subculture timing is reached by photographing the culture medium of the evaluation culture vessel Pe in the subculture timing determination unit 3. Here, in a case where it is determined that the evaluation culture vessel Pe has not reached the subculture timing, it returns to the culture process in STEP2. STEP2 and STEP3 are repeatedly executed until it is determined that the subculture timing is reached. In the culture process in STEP2, in a case where it becomes necessary to replace the culture medium, although it is not yet time to determine the subculture timing, the culture medium is replaced by the culture medium unit 2 in STEP4 as needed, and it returns to the culture process in STEP2. In addition, as a result of determination of the subculture timing, also in a case where it is necessary to replace the culture medium, although the subculture timing is not reached, it returns to the culture process in STEP2 after replacing the culture media by the culture medium unit 2 in STEP4. On the other hand, in a case where it is determined that the evaluation culture vessel Pe has reached the subculture timing in the subculture timing determination process, it proceeds to a subculture process, which is STEP5. The subculture process in STEPS can be executed as, for example, the following process. First, the cells in the evaluation culture vessel Pe are removed and collected, the cell suspension containing the removed cells is adjusted, and the cell suspension is provided into a fresh evaluation culture vessel Pe by the cell inoculation apparatus 5 to seed the cells. At the time of adjustment of the cell suspension, the centrifugal apparatus 7 is utilized to separate cells as needed. The subculture will be completed when the cells are seeded to the fresh evaluation culture vessel Pe. After the subculture, it returns to the culture process in STEP2, and STEP2 to STEPS are repeated as needed.

During each STEP from STEP1 to STEP5, the transfer unit 6 performs transfer of the evaluation culture vessel Pe among the culture medium unit 2, the subculture timing determination unit 3, the culture units 4, and the cell inoculation apparatus 5. In the transfer unit 6, for example, an extendable arm 62 is attached to a moving table 61 that moves along a lifting rail, and the moving table 61 can move to the front of each unit and the evaluation culture vessel Pe can be removed and inserted in each unit. An actuator 63 capable of holding the evaluation culture vessel Pe is provided at a front end of the arm 62.

As shown in FIG. 1B, the processing apparatus 8 is arranged to, for example, the outside of each unit group, so as to be responsible for overall control of the unit group (the culture medium unit 2, the subculture timing determination unit 3, and the like) constituting the cell culture system 1. However, in addition to the processing apparatus 8, or instead of the processing apparatus 8, an internal processor can also be arranged in each unit for control and data processing of each unit. In this aspect, it becomes possible for a unit in which a processor is arranged to function independently of the other units. For example, in the case of the subculture timing determination unit 3, a device for performing control and data processing of the subculture timing determination unit 3 may be arranged in the subculture timing determination unit 3, so as to specialize in that processing. The subculture timing determination unit 3 can be used independently of the other units.

In other words, in this example, as shown in FIG. 1C, the processing apparatus 8 can be arranged in the subculture timing determination unit 3 as a processing apparatus 33, which is an internal processor, and the processing apparatus 33 can perform control and data processing of the subculture timing determination unit 3, instead of the processing apparatus 8. Accordingly, it becomes possible for the subculture timing determination unit 3 to operate independently of the other units.

Furthermore, as illustrated in FIG. 1D, the processing apparatus 33 can exist outside of a subculture timing determination apparatus 3a, which is physically and visually recognized as the subculture timing determination unit 3, and outside of a cell culture apparatus 1a, which is physically and visually recognized as the cell culture system 1. In this case, the processing apparatus 33 is communicably connected to the subculture timing determination apparatus 3a via communication means and a network N. For example, the subculture timing determination apparatus 3a is connected to the camera 31, which is the image-capturing means, via the communication means and the network N. This case includes a case where the network N and the processing apparatus 33 are configured as a cloud. This example is a mode in which the subculture timing determination unit 3 substantially (electrically, systemically) includes the processing apparatus 33, even if the processing apparatus 33 exists outside of the subculture timing determination apparatus 3a and the cell culture apparatus 1a in appearance (physically, visually). In addition, in a case where the cell culture system 1 includes the subculture timing determination unit 3, the cell culture system 1 has the same configuration as the configuration of FIG. 1C in a substantial system configuration. Accordingly, it becomes possible to arrange the processing apparatus 33 apart from the subculture timing determination apparatus 3a to which the camera 31 is arranged, and it becomes possible to perform processing and control in the subculture timing determination apparatus 3a from the outside.

Subsequently, hereinafter, the subculture timing determination performed by the subculture timing determination unit 3 will be described. Here, although an example (the configuration in FIG. 1C) will be described in which the processing apparatus 33 is arranged in the subculture timing determination unit 3, the same applies to a case where the processing is executed in the processing apparatus 8 arranged outside of the subculture timing determination unit 3, without arranging the processing apparatus 33 in the subculture timing determination unit 3 as described above. In this case, the processing apparatus 8 may have the following functions performed by the processing apparatus 33.

(Subculture timing determination unit) Subsequently, referring to FIG. 2B, a subculture determination process in the subculture timing determination unit 3 and the subculture timing determination method, which is the aforementioned STEP3, will be described. The subculture determination process includes, first, an image-capturing step (S11) of obtaining a cell image, a determination data creation step (S12) of creating determination data from the cell image, and a determination step (S13) of determining whether the subculture timing is reached.

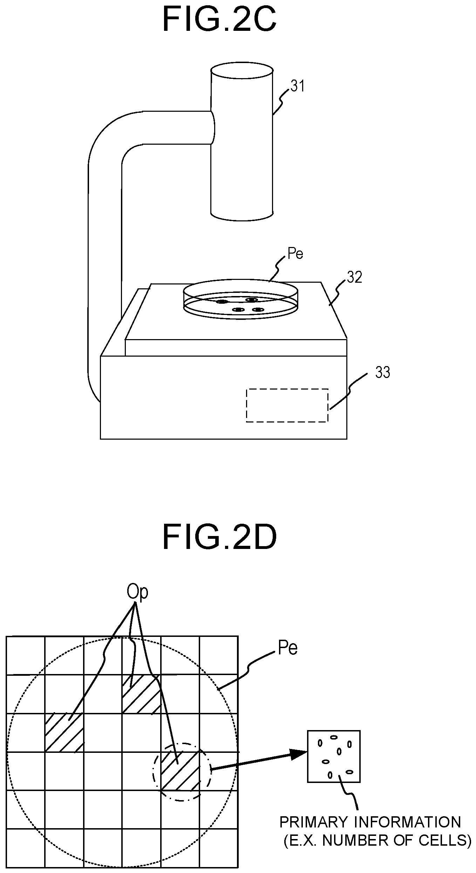

First, in the image-capturing step (S11), an image of cells in the evaluation culture vessel Pe is photographed. FIG. 2C illustrates an external schematic configuration of the subculture timing determination unit 3. The subculture timing determination unit 3 includes the camera 31, which is the image-capturing means, a photographing stage 32, and the processing apparatus 33. The evaluation culture vessel Pe can be arranged on the photographing stage 32, and the camera 31 can photograph the surface of the culture medium in the evaluation culture vessel Pe. A predetermined position of the surface of the culture medium in the evaluation culture vessel Pe transferred by the arm 62 of the transfer unit 6 is photographed with the camera 31, and is obtained as an image. The data of the obtained image is stored in a storage apparatus (not illustrated).

The processing apparatus 33 executes the determination data creation step (S12) and the determination step (S13). The processing apparatus 33 obtains the image obtained in the image-capturing step (S11), and processes the data obtained from the image to determine the subculture timing. These will be specifically described below with reference to FIG. 2C to FIG. 2F.

### (Image-capturing step)

First, in the image-capturing step (S11), the camera 31 executes an image-capturing step of photographing the cells as an image in a culture surface of the culture medium for all evaluation culture vessels Pe in the culture unit 4 of the cell culture system 1 to obtain the cells as cell images. In the specification, it is defined that "culture medium" refers to an in-plane direction of a bottom surface portion of evaluation culture vessel Pe and a culture surface refers to a portion in which cells are stuck in a depth direction in the culture medium in the vessel. One evaluation culture vessel Pe to be subjected to a determination is removed from the culture unit 4 by using the arm 62 of the transfer unit 6 and transferred into the subculture timing determination unit 3 by the transfer unit 6. The transferred evaluation culture vessel Pe is placed on the photographing stage 32 of the subculture timing determination unit 3. The culture medium in the placed evaluation culture vessel Pe is photographed by the camera 31. Upon completion of photographing, the evaluation culture vessel Pe is retrieved from the photographing stage 32 by the arm of the transfer unit 6 and forwarded into the culture unit 4 by the transfer unit 6. The image-capturing step includes repeating the above procedure to execute photographing the cells as an image in the culture surface for all evaluation culture vessels Pe to be subjected to a determination in the culture unit 4.

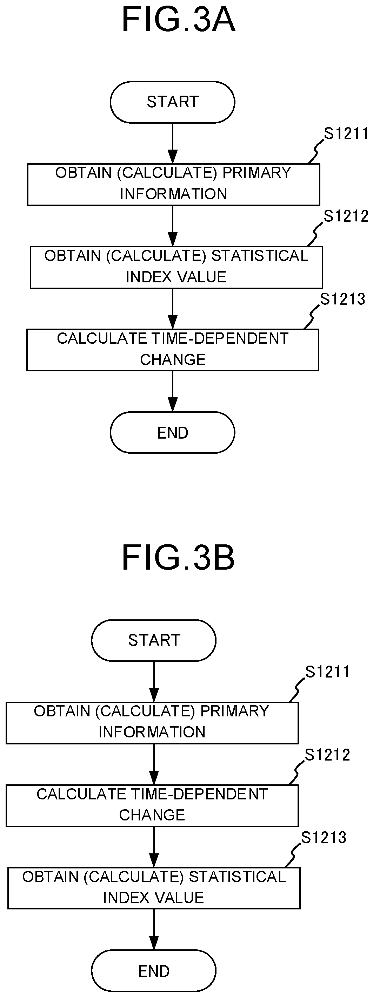

FIG. 2D is a conceptual diagram of an image when a culture surface of a culture medium in a certain evaluation culture vessel Pe is photographed by the camera 31. In image-capturing step, for example as illustrated in FIG. 2D, a culture surface of a culture medium in a certain evaluation culture vessel Pe is photographed by the camera 31 and put in a storage apparatus of the subculture timing determination unit 3 as a cell image. As an example, FIG. 2D illustrates a concept of, for example, 6×6 divisions (36 divisions) being photographed. In practice, however, finer divisions than as described above are photographed. Positions in the culture medium to be photographed are some regions selected from among entire regions of the evaluation culture vessel Pe and are culture medium positions that are a plurality of predetermined observation positions Op determined as typical of the state of evaluation culture vessel Pe such that they are suitable for evaluation of the culture surface in at least the evaluation culture vessel Pe. For example, in FIG. 3B, as an example of a position typical of the evaluation culture vessel Pe, regions in three images, which are hatched positions, are taken as observation positions Op. The observation position Op is determined in such a way that all regions of a culture medium in a certain evaluation culture vessel Pe are initially photographed, and then a position in the image region in which the number of cells and the like are sufficiently typical of the evaluation culture vessel Pe is selected, so that the position in the image region is determined as a fixed observation position. The observation positions Op are typically determined by selecting a plurality of positions such as three positions or ten positions. It is generally recognized that determination accuracy is improved by using a plurality of positions. In the process of subculture timing determination for observing time-dependent changes described below, the same observation position Op as the observation position Op that has once been determined is used in photographing at difference time points for any one evaluation culture vessel Pe. Hereinafter, while any one evaluation culture vessel Pe will be described as a representative example, the same applies to any of a plurality of evaluation culture vessels Pe.

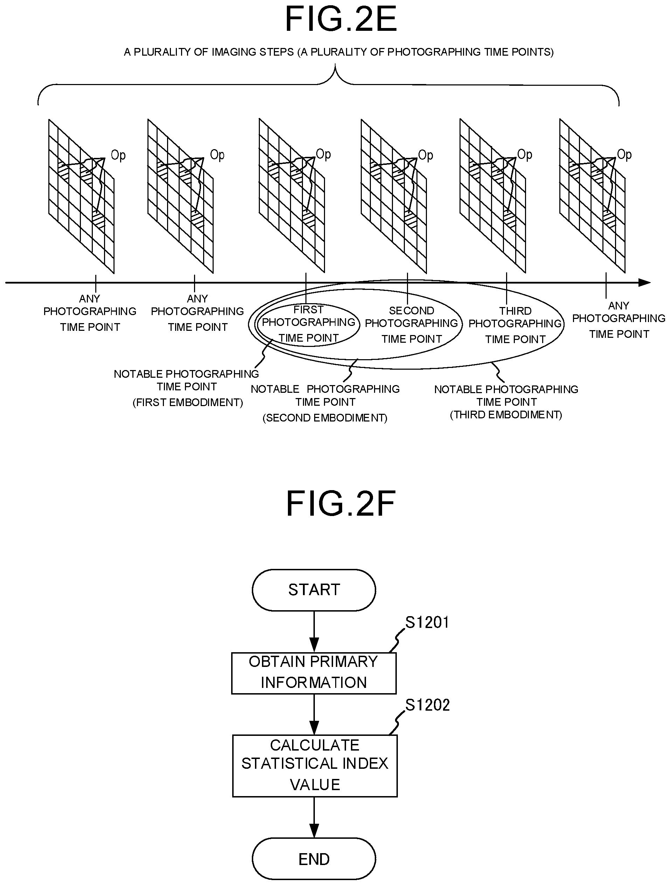

FIG. 2E illustrates a concept in which image-capturing steps are executed at a plurality of photographing time point. As illustrated, in the image-capturing step (S11), as the culture develops, images of the cells in the culture surface are photographed and obtained as cell images at a plurality of photographing time points with predetermined time intervals for the observation position Op in any one evaluation culture vessel Pe. At each photographing time point, cells in the culture surface are photographed to obtain cell images at a plurality of predetermined observation positions Op selected from the culture medium in the evaluation culture vessel Pe. At all photographing time points, the observation positions Op are selected at the same positions such that an observation is made at a fixed point. At each photographing time point, an evaluation culture vessel Pe to be subjected to a determination is removed from the culture unit 4 by the transfer unit 6 and transferred to the subculture timing determination unit 3, and the evaluation culture vessel Pe is transferred to the culture unit 4 after photographing completes. As the culture develops, when a new photographing time point has been reached, the image-capturing step (S11) is executed at the photographing time point, and in the representative example, the determination data creation step (S12) and the determination step (S13), which are described below, are executed each time the image-capturing step (S11) is executed.

In the first embodiment, the determination of the subculture timing is made by using only a cell image photographed at one photographing time point selected from a plurality of photographing time points. In the specification, the photographing time point used to determine the subculture timing is referred to as a notable photographing time point. The notable photographing time point of the first embodiment includes only the evaluation photographing time point. In the first embodiment, a cell image photographed at only the first photographing time point serving as the notable photographing time point in FIG. 2E is used in determining the subculture timing. As described later, in determining the subculture timing, cell images photographed at two notable photographing time points selected from a plurality of photographing time points are used in the second embodiment, and cell images selected from three or more of a plurality of notable photographing time points are used in the third embodiment. FIG. 2E represents that cell images selected from three notable photographing time points are used for the third embodiment notable photographing time point as a representative example.

### (Determination data creation)

Subsequent to the image-capturing step (S11), hereinafter, determination data for subculture determination is created at the determination data creation step (S12). FIG. 2F illustrates each step constituting the determination data creation step (S12) in the first embodiment. As illustrated in FIG. 2F, the determination data creation step (S12) in the first embodiment includes a primary information obtaining step (S1201) of obtaining primary information from a cell image obtained in the image-capturing step (S11), followed by a statistical index value obtaining step (S1202) of calculating a statistical index value from the primary information.

### (Obtaining primary information)

In the primary information obtaining step (S1201), the processing apparatus 33 obtains the primary information from the image obtained at one selected photographing time point (notable photographing time point). The primary information is information on the cells photographed in each cell image in the culture surface photographed and obtained at each photographing time point. The primary information is, for example, the number of cells, a cell density, a cell occupation area, a single cell area, and the relative distance between cells. The primary information can be obtained from images photographed in each image-capturing step by using commonly available, for example, commercially available, software and the like. In the primary information, the number of cells is the number of cells photographed in the photographed image and means the number of cells that is directly obtainable. The cell density is, for example, a density calculated by dividing the number of obtained cells by the area of the position at which photographing takes place. While various areas can be adopted, the area as used herein can be an area excluding a portion that is not contributable as information on cells, such as impurities such as bubbles or a portion of an image in which photographing is failed in the culture medium, the area being a competent effective area corresponding to a region in which cells can spread or proliferate. Furthermore, in those areas, the total area of the sizes of cells occupied by all the cells correspond to the occupation area, and the single cell area is the area of each cell photographed in the image. The occupation area of a single cell is the size of each cell photographed in the image, and the relative distance between cells in one cell image, for example, is a distance defined by two or more cells in the cells photographed in the image. The subculture timing can be determined from the primary information obtained from the image obtained at each photographing time point.

### (Obtaining statistical index value)

In the primary information obtaining step (S1201), the statistical index value obtaining step (S1202) is executed after the primary information obtained at one photographing time point is obtained. Here, from the primary information obtained at one photographing time point, a statistical index value corresponding to the photographing time point is calculated. Furthermore, a statistical index value of each primary information is calculated as basic data indicative of a tendency of the state of the primary information. The statistical index value is an index value defined as a representative value typically indicative of the primary information because a plurality of values is taken in each image, and is at least any of, for example, the number of sum, an average value, a variance, and a deviation such as a standard deviation. That is, the statistical index value of the primary information is a sum (value of total) of values obtainable from images photographed at a plurality of observation positions Op, an average value in each image, or a variance, a deviation. For example, in a case where the primary information is the number of cells and in a case where observation positions Op at a certain photographing time point are three positions, the statistical index value is any of a sum of the number of cells in images of the three positions, or an average value, a variance, or a standard deviation of the number of cells. The primary information and the statistical index value of the primary information are stored in storage means (not illustrated) as obtained data as needed.

Here, "an average value and a sum" and "a variance and a deviation" selected as statistical index values have distinct technical features as indexes for subculture of cells from their definitions. The sum and the average value serving as statistical index values are indexes indicative of an increase in the number of cells and spreading or proliferating of cells and their deceleration. For the sum and the average value, the sum and the average value in a plurality of observation positions Op can be taken as a sum and an average value serving as statistical index values for the same number of positions as the observation positions Op. Furthermore, it may be subjected to statistical processing such as averaging into one representative value or a handleable number of representative values, the number being smaller than the number of observation positions Op. On the other hand, the variance or the deviation serving as statistical index values have a feature of indicating uniformity of cells in the vessel. For the variance or the deviation, the variance or the deviation in a plurality of observation positions Op can be taken as a variance or a deviation serving as statistical index values for the same number of positions as the observation positions Op. Furthermore, it may be subjected to statistical processing such as averaging into one representative value or a handleable number of representative values, the number being smaller than the number of observation positions Op. In the culture of cells, generally, spreading or proliferating of cells causes the uniformity of cells to deteriorate. Subculture timing due to non-uniformity of the cells is then reached earlier before subculture timing due to limitation of the number of cells. That is, in a case where the sum or the average value is selected as a statistical index value, it is often the case that the cells in the culture vessel become non-uniform even though the subculture timing has not been reached yet. In this case, at least in part, it follows that some positions have already reached the limits in spreading or proliferating of cells, and it can be considered as a suitable timing for subculture. With the variance or the deviation serving as a statistical index value, which is an index for the uniformity of the cells in the culture vessel, a determination can be made on the subculture timing more suitably than selecting the sum or the average value as the statistical index value. Features of the sum and the average value and features of the variance and the deviation, which serve as statistical index values, are the same in the second and third embodiments described below.

### (Subculture determination)

After completion of the creation of the determination data, the determination step (S13) of determining whether the subculture timing has been reached is executed. In the determination step (S13), a determination is made on the subculture timing based on the statistical index value created in the determination data creation step (S12). The statistical index value quantitatively indicates the state of cells in culture surfaces at predetermined observation positions Op at a plurality of predetermined points selected from within the culture vessel. Accordingly, taking the number of cells as an example, each sum serving as a statistical index value at a position in the predetermined observation positions Op is the number of all cells in a region in the predetermined observation positions Op, and is a value typical of the total number of cells in the entire regions, including positions other than the observation positions Op in the culture surface in the culture vessel. Taking the number of cells as an example, the average value serving as a statistical index value can be the number of cells per unit area or the number of cells per one observation position Op, for example, and serves as a representative value indicative of potentiality of cells spreading or proliferating in the culture vessel, surpassing the sum.

In the determination step (S13), the statistical index value calculated in the statistical index value obtaining step (S1202) is compared to a predetermined threshold value. Here, when the determination data reaches the predetermined threshold value, it is determined that the subculture timing is reached. For the predetermined threshold value, a test as an examination is actually conducted in advance under the same conditions as determination of the subculture timing, and in the test, a statistical index value is obtained at a time point corresponding to the subculture timing to define the statistical index value as the predetermined threshold value.

The sum and the average value serving as statistical index values are highly effective in a case where the subculture timing is determined in an ideal culture state in which the distance between cells gradually decreases on average such that the distance between cells is kept consistent from a state in which the cells are inoculated. In a case where the sum and the average value serving as statistical index values are selected, a photographing time point corresponding to a time point at which the sum or the average value serving as a statistical index value reaches a predetermined threshold value is determined as a time point at which the subculture timing has been reached. A value at a timing at which subculture is naturally required as the sum or the average value of the primary information can be uniquely defined as an area portion of the observation position Op. The value may be taken as the predetermined threshold value. The sum and the average value, which are values that directly indicate spreading or proliferating of cells, increase over time in theory. The predetermined threshold value can be set as, for example, a value obtained by multiplying a substantial area in the direction in which cells can spread or proliferate in the culture vessel, for example, a base area by a predetermined percentage. For example, the predetermined percentage can be on the order of 70% to 90%. Furthermore, for example, a test as an examination is actually conducted in advance under the same conditions as determination of the subculture timing, and in the examination, the sum or the average value serving as a statistical index value at which spreading or proliferating of cells disappears can be defined as the predetermined threshold value. At this time, the predetermined threshold value can also be obtained in advance as the sum or the average value of the primary information of those reaching subculture timing in a plurality of culture vessels for examination.

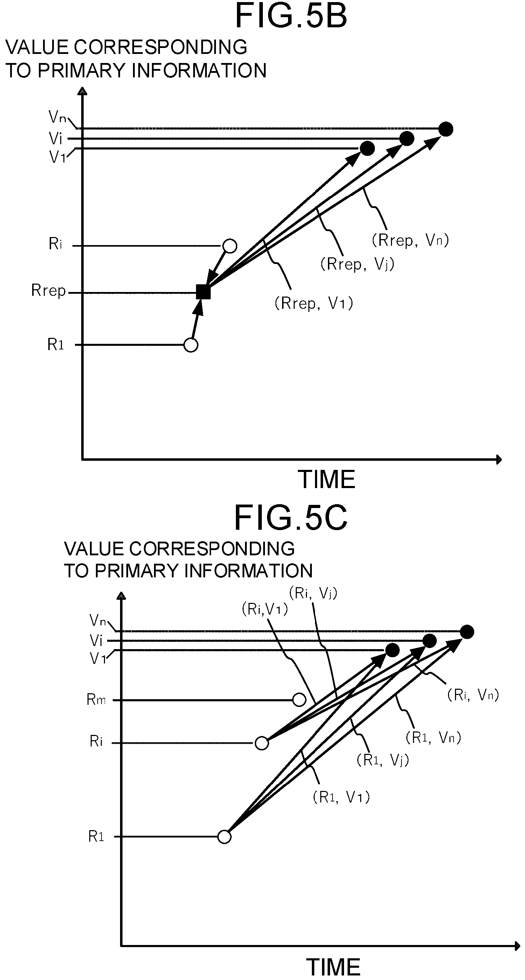

Furthermore, taking the number of cells as an example, a variance or a deviation serving as a statistical index value indicates how cells in an observation position Op are scattered as described above, and is a representative value indicating that cells can no longer spread or proliferate in some positions of the culture surface in the culture vessel even though the average values are the same and a situation where subculture is required has been reached (for example, FIG. 5B). A photographing time point corresponding to the time point at which the variance or the deviation serving as a statistical index value has reached the predetermined threshold value is determined as a time point at which the subculture timing has been reached. A value at a timing at which subculture is naturally required as the variance or the deviation of the primary information can be uniquely defined as an area portion of the observation position Op. The value may be taken as the predetermined threshold value. In the case of a variance or a deviation, the variance or the deviation temporarily decreases in theory because spreading or proliferating of cells occurs near each of the cells in an initial state of the culture.

Generally, then, due to development of spreading or proliferating of cells, a variance or a deviation hits a local minimum over time and then begins to increase. Then, after the subculture timing, since the cells cannot spread or proliferate, the variance or the deviation hits a local maximum and then begins to decrease. Accordingly, a test as an examination is actually conducted in advance under the same conditions as determination of the subculture timing, and in the examination, the predetermined threshold value can be defined as a local maximum at which the variance or the deviation serving as a statistical index value turns from increase to decrease. Furthermore, there may be an exceptional case where the variance or the deviation does not begin to increase due to locally abrupt spreading or proliferating of cells in an initial state of the culture. At that time, since an inflection point in time variation of the variance or the deviation is subculture timing, the inflection point in time variation can be taken as the predetermined threshold value. As described above, at the subculture timing, any inflection points in time variation appear, which are typified by extremal values such as a local minimum and a local maximum. The time point at which such an inflection point in time variation appears is set as the threshold value as a time point at which the subculture timing has been reached. In particular, in a case where the variance or the deviation does not begin to increase, which is the case where the cells spread or proliferate locally abruptly in an initial state of the culture as described above, it is also possible to take a value obtained by adding a margin value α to the local minimum as the threshold value. The margin value α is set such that the value obtained by adding the margin value α to the local minimum is at least smaller than the local maximum. Furthermore, the margin value α is 50% or less of |(local maximum) - (local minimum) | and the value is preferably zero. This value can also be obtained in advance as the variance or the deviation of the primary information of those reaching subculture timing in a plurality of culture vessels for examination.

In the present invention, the primary information on cells is obtained from an image in which the state of a culture medium containing the cells is photographed, and the statistical index value is calculated, so that the progress status of spreading or proliferating of the cells can directly be grasped and the subculture timing can be correctly grasped.

### [Second embodiment]

In the following, a method of subculture timing determination and a system thereof of the second embodiment will be described. The second embodiment is basically the same as the first embodiment but is an embodiment with a partially modified aspect. The configurations of the cell culture system 1 and the subculture timing determination unit 3 are the same as those in the first embodiment. Hereinafter, the second embodiment will be described in terms of different parts from the first embodiment, and description of the same parts as the first embodiment will not be repeated. The subculture determination process of the second embodiment is similar to the first embodiment illustrated in FIG. 2B in that the subculture determination process includes the image-capturing step (S11), the determination data creation step (S12), and the determination step (S13). However, the second embodiment has a difference in the determination data creation in the determination data creation step (S12) and the predetermined threshold value in the determination step (S13). The second embodiment is different from the first embodiment in that, in the determination data creation in the determination data creation step (S12), two photographing time points are selected as notable photographing time points from a plurality of photographing time points in the image-capturing step (S11), and subculture timing is determined by creating determination data from cell images at the two photographing time points. That is, in the second embodiment, a time-dependent element is added to the determination data.

### (Image-capturing step of culture surface)

As illustrated in FIG. 2E, the second embodiment is similar to the first embodiment in that cell images are photographed at a plurality of photographing time points. It is also similar in that observation positions Op are selected at the same positions such that an observation is made at a fixed point at each photographing time point, and the image-capturing step is executed when the culture develops and a new photographing time point has been reached.

### (Determination data creation)

FIG. 3A and FIG. 3B are flow diagrams of the determination data creation step (S12) in the subculture determination process of the second embodiment. In the determination data creation step (S12) of the second embodiment, a time-dependent change index value is calculated to add time-dependent element to the first embodiment. Here, FIG. 3A is a diagram illustrating a first example of the second embodiment. In the first example, after a primary information obtaining step (S1211) of obtaining primary information from a cell image and a statistical index value calculation step (S1212) of calculating an index value from the primary information are executed, a time-dependent change calculation step (S1213) of calculating a time-dependent change index value is executed.

On the other hand, FIG. 3B is a diagram illustrating a second example of the second embodiment. In the second example, the time-dependent change calculation step (S1213) of calculating a time-dependent change index value is executed between the primary information obtaining step (S1211) of obtaining primary information from a cell image and the statistical index value calculation step (S1212) of calculating an index value from the primary information. In the specification, two photographing time points used to calculate a time-dependent change index value in the time-dependent change calculation step (S1213) is referred to as notable photographing time points, and a temporally preceding photographing time point of the notable photographing time points is referred to as a reference photographing time point and a temporally following photographing time point as an evaluation photographing time point. The time-dependent change index value is a value of the amount of change for a statistical index value of the primary information at the notable photographing time point relative to a statistical index value of the primary information at a reference photographing time point.

In the second embodiment, two notable photographing time points selected from a plurality of photographing time points are used in determining subculture timing in both the first example and the second example. For example, the cell images photographed at the first photographing time point and the second photographing time point in FIG. 2E are used. The notable photographing time points in the second embodiment include the reference photographing time point and the evaluation photographing time point. The evaluation photographing time point is typically a new photographing time point that has been reached as the culture develops. For example, as illustrated in FIG. 2E, a first photographing time point serving as the reference photographing time point a second photographing time point serving as the evaluation photographing time point are selected. Hereinafter, description will be made with reference to notable photographing time points, in which the reference photographing time point is the first photographing time point and the evaluation photographing time point is the second photographing time point. As a method for selecting two notable photographing time points, there are two choices for both the first example and the second example: a nearest comparison method and a reference comparison method.

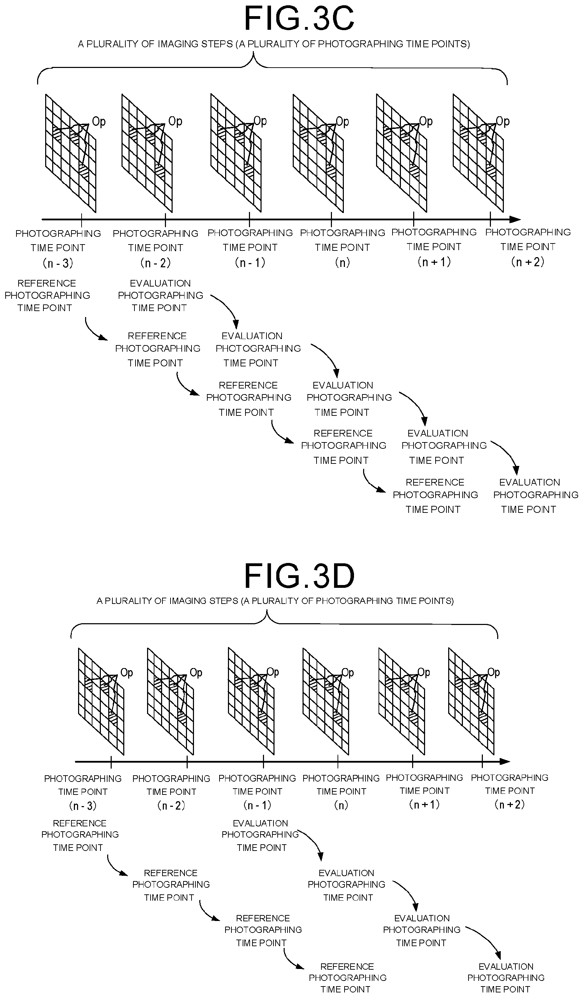

In the nearest comparison method, both the reference photographing time point and the evaluation photographing time point are updated as the latest notable photographing time points as new photographing time points increase. That is, in a case where determination of the subculture timing is conducted in the nearest comparison method by using any notable photographing time points (the reference photographing time point and the evaluation photographing time point) and when subculture timing is continued because the subculture has not been reached yet, the notable photographing time points (the reference photographing time point and the evaluation photographing time point) are updated to conduct the subculture determination. This will be specifically described with reference to FIG. 3C. FIG. 3C illustrates a concept of selecting notable photographing time points in the nearest comparison method, in which the notable photographing time points (the reference photographing time point and the evaluation photographing time point) are selected from a plurality of photographing time points (n-3) from a photographing time point (n-3) to a photographing time point (n+2). For example, after notable photographing time points in which the photographing time point (n-3) is the reference photographing time point and the photographing time point (n-2) is the evaluation photographing time point are selected and the determination of the subculture timing is conducted, and in a case where it is determined that the subculture timing has not been reached yet, notable photographing time points in which the photographing time point (n-2) is the reference photographing time point and the photographing time point (n-1) is the evaluation photographing time point are selected to conduct the determination of the subculture timing. Thereafter, in a similar way, until the determination of the subculture timing has been made, notable photographing time points are repeatedly updated to conduct the determination of the subculture timing, while the reference photographing time point is shifted from the photographing time point (n-3) to the photographing time point (n+1) and the evaluation photographing time point is also shifted from the photographing time point (n-2) to the photographing time point (n+2). When the reference photographing time point and the evaluation photographing time point are shifted for update, the reference photographing time point and the evaluation photographing time point must be regularly shifted in order. For example, in a case where the reference photographing time point is shifted in one step, the evaluation photographing time point must be regularly shifted such that it is also shifted in one step. Such an irregular way of shifting the evaluation photographing time point in two steps although the reference photographing time point is shifted only in one step is not allowed.

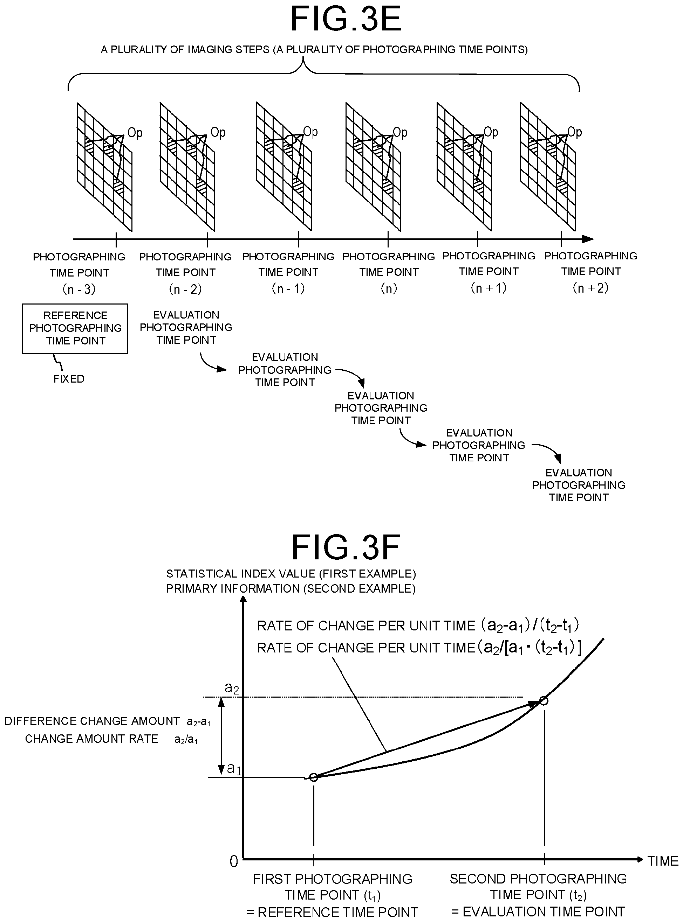

In the reference comparison method, notable photographing time points including the reference photographing time point and the evaluation photographing time point are selected as described below. FIG. 3E illustrates a concept of selecting notable photographing time points in the reference comparison method, in which the notable photographing time points (the reference photographing time point and the evaluation photographing time point) are selected from a plurality of photographing time points (n-3) from the photographing time point (n-3) to the photographing time point (n+2). In the reference comparison method, any initial photographing time point from a plurality of photographing time points is determined as the reference photographing time point, and any photographing time point after the reference photographing time point is selected as the evaluation photographing time point. For example, the photographing time point (n-3) is fixed as the reference photographing time point, and a notable photographing time point in which the photographing time point (n-2) is the evaluation photographing time point is selected to conduct the determination of the subculture timing. After the determination, in a case where it is determined that the subculture timing has not been reached yet, notable photographing time points are selected such that the photographing time point (n-2) remains unchanged as the reference photographing time point and the photographing time point (n-1) is the evaluation photographing time point to conduct the determination of the subculture timing. Thereafter, in a similar way, until the determination of the subculture timing has been made, the notable photographing time points are repeatedly updated while the reference evaluation photographing time point is kept as being the photographing time point (n-3) and only the evaluation photographing time point is shifted from the photographing time point (n-2) to the photographing time point (n+2) to conduct the determination of the subculture timing. That is, in the case of the reference comparison method, the reference photographing time point is used by being fixed each time the determination of the subculture timing is conducted and a new photographing time point is selected and changed as the evaluation photographing time point such that only the evaluation photographing time point is updated to the latest photographing time point, which are repeated for each subculture timing. Even in this case, when both the reference photographing time point and the evaluation photographing time point are shifted for update, each of the reference photographing time point and the evaluation photographing time point must be regularly shifted in order.

In the nearest comparison method, since the reference photographing time point and the evaluation photographing time point move together, it will be a relative comparison of the primary information at each photographing time point of the reference photographing time point and the evaluation photographing time point. On the other hand, in the reference comparison method, since the first photographing time point that is the reference photographing time point is fixed, it will be an absolute comparison of the primary information between the first photographing time point (fixed) as the reference photographing time point and the second photographing time point (moved) as the evaluation photographing time point. Furthermore, while any time interval can be set between photographing time points, in the nearest comparison method in which two consecutive photographing time points are selected in particular, it is preferable to set the time interval between photographing time points to be constant.

In the nearest comparison method, while a selection is typically made such that no photographing time point is present between the reference photographing time point and the evaluation photographing time point as illustrated in FIG. 3C, a selection may be made such that, as illustrated in FIG. 3D, any number of photographing time points that are not used for evaluation is set between the reference photographing time point and the evaluation photographing time point. FIG. 3D illustrates an example of one photographing time point that is not used for evaluation located between the reference photographing time point and the evaluation photographing time point. It is then possible to conduct the determination of the subculture timing as long as a selection is made such that the interval between the reference photographing time point and the evaluation photographing time point is constant and the reference photographing time point and the evaluation photographing time point are selected in order. Furthermore, in the reference comparison method, since the reference photographing time point is fixed and the evaluation photographing time point is updated, there are photographing time points between the reference photographing time point and the evaluation photographing time point.

Hereinafter, taking the nearest comparison method as an example, each of the first example and the second example will be described with reference to an example in which the reference photographing time point and the evaluation photographing time point are consecutive for certain notable photographing time points. Here, description will hereinafter be made with the first photographing time point being as the reference photographing time point and the second photographing time point being as the evaluation photographing time point for such notable photographing time points. Even in a case where a nearest comparison method in which the reference photographing time point and the evaluation photographing time point are not consecutive and the reference comparison method, the subculture determination process is the same as the example described below because data at photographing time points other than the notable photographing time points is simply ignored in creating data for the determination of the subculture timing.

### (Obtaining primary information)

The primary information obtaining steps are the same in the first example and the second example. In the primary information obtaining step (S1211), the processing apparatus 33 obtains the primary information at each photographing time point from an image obtained at each photographing time point. As in the first embodiment, the primary information obtaining step is, for example, the number of cells, a cell density, a cell occupation area, a single cell area, and the distance between cells. The description will not be repeated here. Hereinafter the first example will be described, and thereafter, the second example will be described.

### <First example>

### (Obtaining statistical index value)

In the first example, as described above, as illustrated in FIG. 3A, the primary information at each photographing time point is obtained in the primary information obtaining step (S1211), and thereafter, the statistical index value at each photographing time point is calculated from the primary information of each photographing time point in the statistical index value obtaining step (S1212). As in the first embodiment, the statistical index value is an index value defined as a representative value typically indicative of the primary information, and is at least any of, for example, a sum, an average value, a variance, and a deviation such as a standard deviation. The sum is a sum of the number of cells at a plurality of observation positions, a sum of cell occupation areas, a sum of single cell areas, and a sum of the distances between cells. The average value is an average value of the number of cells at a plurality of observation positions, an average value of cell densities, an average value of occupation areas of the number of cells, an average value of single cell areas, and an average value of the distances between cells. The variance and the deviation are a variance and a deviation of the number of cells of a plurality of observation positions, a variance and a deviation of cell densities, a variance and a deviation of cell occupation areas, a variance and a deviation of single cell areas, and a variance and a deviation of the distances between cells. Here, as in the first embodiment, the variance or the deviation serving as statistical index values, which are indexes of the uniformity of cells in the culture vessel, are more suitable for being able to determine the subculture timing than selecting the sum or the average value as a statistical index value.

### (Time-dependent change calculation step (obtaining time-dependent change index value))

Subsequently, in the time-dependent change calculation step (S1213), a time-dependent change index value is calculated from the statistical index value of each piece of primary information calculated at each photographing time point. One time-dependent change index value is created for values of pieces of primary information at two photographing time points. In the embodiment, the notable photographing time points are equal to the number of photographing time points used for determination. Since the primary information is obtained for each of notable photographing time points, the number of notable photographing time points is equal to the number of pieces of primary information. Accordingly, the number of time-dependent change index values is one fewer than the number of notable photographing time points. Furthermore, the time-dependent change index value is calculated such that the number of time-dependent change index values is equal to the number of observation positions Op, or, in a case where a plurality of observation positions Op is adjusted to be a smaller number of representative values, to the number of the representative values.

The time-dependent change calculation step (S1213) is a step in which a step of calculating a time-dependent change index value is added after the statistical index value obtaining step (S1212) of calculating the statistical index value of the first embodiment. This will be described with reference to FIG. 3F. FIG. 3F is a diagram illustrating a concept of a change in value (vertical axis) relative to time (horizontal axis) in the case of the first example and the second example. The vertical axis in the case of the first example indicates a statistical index value and the vertical axis in the case of the second example described later indicates a value of primary information. FIG. 3F illustrates, as an example, the nearest comparison method for two consecutive photographing time points that are notable photographing time points. However, in the case of the reference comparison method in which the first photographing time point and the second photographing time point are not selected as consecutive photographing time points, FIG. 3F may be read as there being photographing time points that are not illustrated between the first photographing time point and the second photographing time point.

In the reference comparison method in which, two photographing time points are selected such that the first photographing time point is fixed as the reference photographing time point and the second photographing time point that is the evaluation photographing time point is updated, the first photographing time point is always fixed. Then, the value of primary information is defined at the second photographing time points updated relative to the value of such fixed primary information, the primary information increases as cells spread or proliferate. Since the time-dependent change index value indicates increase or decrease in the percentage of increase of the value, the time-dependent change index value of the statistical index value indicates a tendency of increase or decrease in response to the tendency toward an increase in the primary information. On the other hand, in a case where the primary information is the relative distance between cells, the primary information decreases as cells spread or proliferate. In this case, since the time-dependent change index value also indicates increase or decrease in the percentage of decrease of the value, the time-dependent change index value of the statistical index value indicates a tendency of increase or decrease in response to the tendency toward a decrease in the primary information. Then, as the subculture timing approaches, the degree of change of increase or decrease is decelerated and indicates a trend such that the rate of change of increase or decrease is saturated towards a predetermined value. On the other hand, in the nearest comparison method in which, in two photographing time points, the first photographing time point and the second photographing time point are selected as consecutive photographing time points, the first photographing time point and the second photographing time point are updated all the time, resulting in a relative comparison between the first photographing time point and the second photographing time point. Consequently, at the initial stage of spreading or proliferating of cells, the primary information monotonically increases or monotonically decreases (monotonically decreases in a case where the primary information is the relative distance between cells), and the time-dependent change index value of the statistical index value also increases or decreases, while the degree of increase in the primary information at the first photographing time point and the degree of increase or decrease in the primary information at the second photographing time point vary as spreading or proliferating of cells progresses, and therefore, as the subculture timing approaches, the degree of increase or decrease in the time-dependent change index value tends to decelerate. Then, at the subculture timing, the time-dependent change index value that has exhibited increasing tendency inversely decreases at the predetermined value as an extremal value, and the time-dependent change index value that has exhibited decreasing tendency inversely increases. Hereinafter, description will be made here with reference to an example in which the primary information exhibits a trend toward an increase as cells spread or proliferate. However, in a case where the primary information exhibits a trend toward a decrease as cells spread or proliferate, it is possible to replace "increase" with "decrease" to read and vice versa, so that the example in which the primary information exhibits a trend toward an increase as cells spread or proliferate can be applied.

In calculating the time-dependent change index value, two photographing time points (the first photographing time point and the second photographing time point) are selected from a plurality of photographing time points. FIG. 3F illustrates an example in which the latest two consecutive photographing time points are selected as the first photographing time point (t₁) and the second photographing time point (t₂). FIG. 3F also indicates that the statistical index value (a₁) at the first photographing time point (t₁) is increased to the statistical index value (a₂) at the second photographing time point (t₂). Determination is made at the photographing time point whether the subculture timing has been reached, and if the subculture timing has not been reached, the latest two consecutive photographing time points including a new photographing time point are selected again to create determination data.

The time-dependent change index value can be taken as a difference change amount, which is the amount of difference in statistical index values of the primary information between the first photographing time point (reference photographing time point) and the second photographing time point (evaluation photographing time point) for the time interval between notable photographing time points (the first photographing time point and the second photographing time point). The time-dependent index value of the difference change amount is a difference change amount (a₂-a₁), which is the amount of difference of the statistical index value of the primary information at the second photographing time point relative to the statistical index value of the primary information at the first photographing time point. In other words, the difference change amount is the amount of increase. In an example in which the primary information is the number of cells, this is a value of the difference of increase between sums of the number of cells at the time points of the first photographing time point and the second photographing time point.

Furthermore, the time-dependent change index value may be taken as a percentage change amount of the statistical index value of the primary information at the second photographing time point (evaluation photographing time point) relative to the statistical index value of the primary information at the first photographing time point (reference photographing time point). The time-dependent index value of the percentage change amount is (a₂/a₁), which is a percentage change amount of the statistical index value of the primary information at the second photographing time point relative to the statistical index value of the primary information at the first photographing time point. The time-dependent change index value can also be taken as a difference-percentage change amount, which is the percentage of the amount of difference of the statistical index value of the primary information between the first photographing time point (reference photographing time point) and the second photographing time point (evaluation photographing time point) relative to the statistical index value of the primary information at the first photographing time point (reference photographing time point). The difference-percentage change amount can be taken as a difference-percentage change amount (a₂-a₁) /a₁), which is the percentage of difference of the amount of increase between the statistical index value of the primary information at the first photographing time point (reference photographing time point) and the statistical index value of the primary information at the second photographing time point (evaluation photographing time point) relative to the amount of increase of the statistical index value of the primary information at the first photographing time point. For example, in an example in which the primary information is the number of cells, the former percentage change amount is a value of the percentage of the sum of the number of cells at the second photographing time point relative to the sum of the number of cells at the first photographing time point, and the latter difference-percentage change amount is a value of the percentage of the sum of the increased number of cells from the first photographing time point to the second photographing time point relative to the sum of the number of cells at the first photographing time point.

The time-dependent change index value can also be taken as the difference change amount, the percentage change amount, and the difference-percentage change amount as described above for the statistical index value of the primary information at the first photographing time point and the statistical index value of the primary information at the second photographing time point relative to the time interval between the two photographing time points (the first photographing time point and the second photographing time point). Specifically, they are the difference change amount (a₂-a₁), the percentage change amount (a₂/a₁), and the difference-percentage change amount (a₂-a₁) /a₁), each of which can be divided by a time difference (t₂-t₁) between the first photographing time point and the second photographing time point to take it as a time-dependent change index value that is the rate of change per unit time. That is, the statistical index value of the primary information at two consecutive photographing time points can be taken as the rate of change per unit time of the difference change amount (a₂-a₁)/(t₂-t₁), the rate of change per unit time of the percentage change amount (a₂/[a₁•(t₂-t₁)]), and the rate of change per unit time of the difference-percentage change amount (a₂-a₁) / [a₁•(t₂-t₁)]). Any of these time-dependent change index values means an increasing rate per unit time of the statistical index value of the primary information at notable photographing time points, which is also the rate of increase. In an example in which the primary information is the number of cells, it is the increasing rate of the number of cells per unit time, namely, the rate of increase (growth rate). The rate of increase (growth rate) as used herein is an approximated rate (hereinafter, simple rate) obtained as a difference value by subjecting the statistical index values at two photographing time points to a linear approximation.

In addition to the examples described above, the rate of increase (growth rate) can be defined based on a wide variety of definitions as an increasing rate over time as long as it can represent a time-dependent change of the statistical index value of the primary information. In proliferation of cells, since the cause of increase is cell division, the rate of increase (growth rate) is not constant, and characterized in that, if conditions are met, multiples in particular increase theoretically by a factor of two such as twofold, fourfold, eightfold, and so on. In general, in a case where the rate of increase (growth rate) is to be indicated quantitatively, a specific growth rate is used. In the invention, not only in a case where the primary information is the number of cells but also in a case where the primary information is not the number of cells (the cell density, the cell occupation area, the single cell area, the relative distance between cells, and the like, as described above), the cause of increase is still cell division and the increase occurs substantially twofold. In practice, even in a case where the primary information is the number of cells, since division timing is different for each cell, it is unusual that the increase occurs exactly twofold per unit time. Accordingly, whether the primary information is the number of cells, multiplication factor of increase per unit time is a multiplication factor approximated to e^{µ}-fold. µ at this time is referred to as a specific growth rate. Accordingly, the specific growth rate µ obtained by subjecting the statistical index value at two photographing time points of the reference time point and the evaluation time point to exponential approximation (ₑ^{µt}) can be adopted as the rate of increase (growth rate).

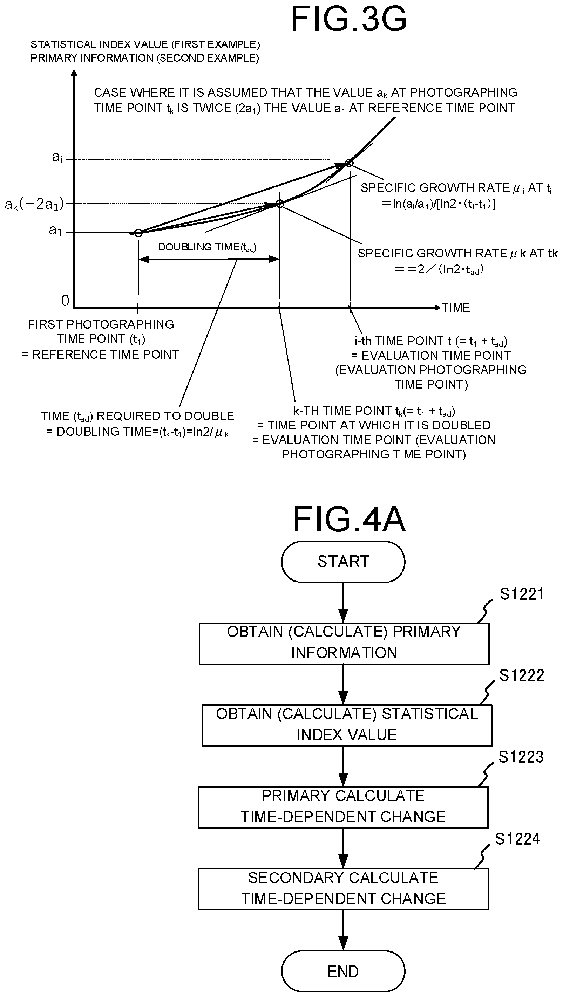

An example of applying the specific growth rate and a multiplying time t_{d} as the time-dependent change index values will be described with reference to FIG. 3G. Specifically, the reference time point is set to a first photographing time point t₁ as the reference photographing time point, and the statistical index value of the primary information at that time is a₁. Then, at each time point of an any photographing time point tᵢ [i=2 to n], a statistical index value of the primary information aᵢ [i=2 to n] is measured as the evaluation photographing time point. The statistical index value of the primary information aᵢ [i=2 to n] at each evaluation photographing time point can be approximated such that it varies, so that the relation of aᵢ/a₁=e^{[(µi)•(ti-t1)]} is established between the statistical index value of the primary information at the first photographing time point t₁ and a₁ as a generalized relation. Here, µᵢ is a specific growth rate at a photographing time point tᵢ [i=2 to n] and is µᵢ = [ln (aᵢ/a₁)]/(tᵢt₁).

Furthermore, a multiplying time (tad) can be adopted as the rate of increase (growth rate). The multiplying time is time required for the amount of evaluation time point to be twice the amount of reference time point, and the multiplying time t_{ad}=tₖ-t1 is established when it is assumed that multiplying occurs at i=k of photographing time points tᵢ [i=2 to n]. In relation to the specific growth rate, this time is a time at which e^{µ•tad}=2 is established assuming that the multiplication factor of increase per unit time is e^{µ}. That is, from the description above, if µᵢ is obtained as the time-dependent change index value of the primary information at each time point of an any photographing time point tᵢ[i=2 to n], for the time-dependent change index value µᵢ[i=2 to n] of the primary information at each evaluation photographing time point, the relation of e^{[(µi)•(tdi)]}=2 is established between the time-dependent change index value of the primary information at each evaluation photographing time point tᵢ and tdᵢ as a generalized relation. That is, multiplying time tdᵢ=ln2/µᵢ= (ln2) • (tᵢ-t₁) /ln (aᵢ/a₁). Here, as a comprehensible example, it is assumed that the statistical index value aₖ of the primary information at i=k[2<k<n] of photographing time points tᵢ[2<i<n] has become twice the statistical index value a₁ at the first photographing time point t₁ that is the reference photographing time point (aₖ=2a₁). At this time, time taken from the first photographing time point t₁ that is the reference photographing time point to the photographing time point tₖ[2<k<n] that is the evaluation photographing time point is what is called a multiplying time t_{ad} and the relation of ak/a1=e^{(µk•tad)} =2 is established. Based on this, specific growth rate µₖ= (ln2)/ (tₖ-t₁) at tₖ.

Furthermore, in addition to the multiplying time, time required for the amount of evaluation time points to reach a predetermined multiple of the amount of reference time points can be adopted as the rate of increase (growth rate). In the application, it is defined as a predetermined multiplying time (tx). The predetermined multiplying time is time required to reach X times the predetermined multiple, and if the multiplication factor of increase per unit time is e^{µ}, it is defined as e^{µ•tx}=X. That is, from the description above, if µᵢ is obtained as the time-dependent change index value of the primary information at each time point of any photographing time point tᵢ[i=2 to n], for the time-dependent change index value µᵢ[i=2 to n] of the primary information at each evaluation photographing time point, the relation of e^{[(µi)•(txi)]}=X is established between time-dependent change index value of the primary information at each evaluation photographing time point tᵢ and txᵢ as a generalized relation. That is, predetermined multiplying time txᵢ=lnX/µᵢ= (lnX) • (tᵢ-t₁) /ln (aᵢ/a₁). The predetermined multiplying time at photographing time point tₖ described above is tx=(lnX)•(tₖ-t1)/ln2, the multiplying time described above is a predetermined multiplying time at X=2.

In the determination data creation step (S12), the time-dependent change index values obtained above can be taken as determination data. As in the above, the time-dependent change index value may be calculated depending on the selection of each of the number of cells, a cell density, a cell occupation area, a single cell area, the distance between cells, and the like, as the primary information and the selection of each of a sum, an average value, a variance, and a deviation as the statistical index value. It can be considered that the time-dependent change index value of the first example is a representative value indicative of a time-dependent change of the statistical index values of the primary information. A plurality of time-dependent change index value may be taken as the same number of one or more pieces of determination data, or a plurality of time-dependent change index value may be reduced to one time-dependent change index value as an average value, a maximum value, a minimum value, and the like and it may be taken as one piece of determination data.

### (Subculture determination)

After completion of the creation of the determination data in the determination data creation step (S12), the determination step (S13) of determining whether the subculture timing has been reached is executed. In the determination step (S13), a determination is made on the subculture timing based on the determination data created in the determination data creation step (S12). In the first example, the determination data is a time-dependent change index value of the statistical index value. The statistical index value indicates quantitatively the state of cells in culture surfaces at predetermined observation positions Op at a plurality of predetermined points selected from within the culture vessel. Accordingly, in the first example, it is a time-dependent change index value of the sum serving as a statistical index value at a position of predetermined observation positions Op, and serves as a representative value that can indicate potentiality of cells spreading or proliferating in the culture vessel more accurately than the first embodiment.

In the determination step (S13), the determination data that is the time-dependent change index value calculated in the time-dependent change calculation step (S1213) is compared to a predetermined threshold value. Here, it is determined that the subculture timing has been reached when the determination data reaches the predetermined threshold value. The predetermined threshold value is the same as the first embodiment in a case where two photographing time points are selected such that the first photographing time point is fixed as the reference photographing time point and the second photographing time point is sequentially updated. As in the first embodiment, a test as an examination is actually conducted in advance under the same conditions as determination of the subculture timing, and in the test, a statistical index value is obtained at a time point corresponding to the subculture timing to determine the time-dependent change index value, which is defined as the predetermined threshold value. Even when a time-dependent change element is added, features of the statistical index value in determining the subculture timing will not be lost, which is as described in the first embodiment. That is, at the subculture timing, any inflection points in time variation in the time-dependent change index value appear, which are typified by extremal values such as a local minimum and a local maximum. The time point at which such an inflection point in time variation appears is set as the threshold value for determining the time point at which the subculture timing has been reached.

On the other hand, in a case where the first photographing time point and the second photographing time point are selected as consecutive photographing time points for two photographing time points, the predetermined threshold value is as follows. When the statistical index value increases as the primary information at the second photographing time point increases, the primary information at the first photographing time point increases and the statistical index value also increases. At the initial stage of spreading or proliferating of cells, the amount of increase of the statistical index value at the second photographing time point is larger than the amount of increase of the statistical index value at the first photographing time point. However, the amount of increase of each value gradually decreases as cells spread or proliferate, and at a stage at which the subculture timing of cells has been reached, the amount of increase of the statistical index value at the second photographing time point becomes smaller than the amount of increase of the statistical index value at the first photographing time point. Consequently, at a stage at which the subculture timing of cells has been reached, the statistical index value takes an extremal value at an any threshold value and decreases. The extremal value is set as the predetermined threshold value. The extremal value is defined during a test as an examination actually conducted in advance under the same conditions as determination of the subculture timing. In the test, a time-dependent change index value is determined from a statistical index value at a time point corresponding to the subculture timing and when the time-dependent change index value takes an extremal value, the extremal value is set as the predetermined threshold value. Furthermore, when the time-dependent change index value reaches the predetermined threshold value, the subculture timing has already been reached. Accordingly, a time point slightly earlier can be considered as timing best suited for the subculture. Accordingly, the predetermined threshold value can be set as a value obtained by subtracting a margin value α from an extremal value of the time-dependent change index value ("extremal value of time-dependent change index value" - "margin value α"). In this way, determination of whether or not the subculture timing has been reached can be made at a time point extremely close to the subculture timing and in the state in which deceleration of spreading or proliferating of cells is still imperceptible and cells are in good condition. The "margin value α" is also set during a test as an examination conducted in advance.

In the case of one piece of determination data, comparison is made to find whether the one piece of determination data has reached the predetermined threshold value, and in a case where it has reached the predetermined threshold value, it is determined that the subculture timing has been reached. In the case of one or more pieces of determination data, comparison is made to find whether a predetermined number of pieces of determination data of the one or more pieces of determination data have reached the predetermined threshold value, and in a case where they have reached the predetermined threshold value, it can be determined that the subculture timing has been reached. Furthermore, it may be determined that the subculture timing has been reached in a case where all the pieces of determination data of the one or more pieces of determination data have reached the predetermined threshold value. In this case, since time is required until all the pieces of determination data have reached the predetermined threshold value, it follows that determination of whether the subculture timing has been reached is made relatively late.

In contrast, it may be determined that the subculture timing has been reached in a case where at least one of the pieces of determination data of one or more pieces of determination data has reached the predetermined threshold value. In this case, since determination can be made only when one piece of determination data has reached the predetermined threshold value, determination of whether the subculture timing has been reached can be made earlier.

### <Second example>

In the following, the second example will be described. In the first example, the primary information is obtained at each photographing time point in the primary information obtaining step (S1211), followed by the statistical index value obtaining step (S1212) in which the statistical index value of each photographing time point is calculated from the primary information at each photographing time point, and thereafter, in the time-dependent change calculation step (S1213), the time-dependent change index value is calculated as the determination data. However, as illustrated in FIG. 3B, the second example is different in that, after the primary information of each photographing time point is obtained in the primary information obtaining step (S1211), the time-dependent change calculation step (S1213) is executed to calculate the time-dependent change index value of the primary information, and thereafter, the statistical index value of the time-dependent change index value is calculated as the determination data in the statistical index value obtaining step (S1212). The primary information and the primary information obtaining step (S1211) are the same as those in the first embodiment and the first example, and therefore, the description will not be repeated.

### (Time-dependent change calculation step (obtaining time-dependent change index values))

In the time-dependent change calculation step that follows (S1213), time-dependent change index values are calculated for the primary information calculated at each photographing time point. Each time-dependent change index value is the amount of change in the primary information between photographing time points. As in the first example, the time-dependent change index value in the second example is the difference change amount, the rate of amount of change, the rate of change per unit time, the change speed, or the time of change. The only difference regarding this value between the first and second examples is as follows. In the first example, the time-dependent change index value is the difference change amount, the rate of amount of change, the rate of change per unit time, the change speed, or the time of change of statistical index values of the primary information. By contrast, the time-dependent change index value in the second example is the difference change amount, the rate of amount of change, the rate of change per unit time, the change speed, or the time of change of the primary information. Therefore, the above-described calculation of the time-dependent change index value in the first example may be applied to the second example simply by substituting "the primary information" for "the statistical index values of the primary information" in calculating the time-dependent change index value from the statistical index values of the primary information in the first example. As such, in the second example, detailed description will not be provided regarding the calculation of the difference change amount, the rate of amount of change, the rate of change per unit time, the change speed, or the time of change of the primary information. In the second example, as in the first example, the change speed may be applied as the specific growth rate, and the time of change may be applied as the multiplying time.

With reference to FIG. 3F, the time-dependent change index values in the second example will be described. FIG. 3F is a diagram illustrating a concept of changes in the primary information (vertical axis) along time (horizontal axis) in the first and second examples. For the second example, the values of the primary information obtained in the primary information obtaining step (S1211) in the second example may be substituted for the statistical index values in the description of the time-dependent change calculation step (S1213) in the first example, that is, a₁ and a₂ corresponding to the time-dependent change index value. Here, time-dependent change index values as many as the pieces of primary information are obtained. It can be said that the time-dependent change index values in the second example directly indicate the tendency of time-dependent change in the primary information.

### (Obtaining statistical index values)

Then, as illustrated in FIG. 3B, after the time-dependent change index values of the primary information between the first photographing time point and the second photographing time point are obtained in the time-dependent change calculation step (S1213), a statistical index value of the time-dependent change index values is calculated in the statistical index value obtaining step (S1212). The statistical index value in the second example is an index value defined as a representative value typically indicative of the time-dependent change index values of the primary information, for example at least any of the average value, the variance, and the deviation such as the standard deviation. In the second example, which involves calculating the statistical index value of the time-dependent change index values, the statistical index value may be the average value, the variance, or the deviation of the time-dependent change index value of the primary information at each of the plurality of observation positions Op. The obtained statistical index value is used as determination data to perform the subculture timing determination. Multiple statistical index values may be simply used as two or more pieces of determination data, or a single statistical index value obtained from multiple statistical index values, such as the average value, maximum value, or minimum value, may be used as one piece of determination data.

### (Subculture determination)

After the determination data is created in the determination data creation step (S12), the determination step (S13) is performed for determining whether the subculture timing has been reached. In the determination step (S13), the subculture timing determination is performed based on the statistical index value created in the determination data creation step (S12). The determination data in the second example is the statistical index value of the time-dependent change index values. Therefore, as in the determination data in the first example that is the time-dependent change index value of the statistical index values, the elements of the determination data include the primary information, the time-dependent change, and the statistical index value. Thus, as in the first example, the determination data is effective for performing the subculture timing determination.

In the determination step (S13), the statistical index value calculated in the statistical index value obtaining step (S1202) is compared with a predetermined threshold value. The predetermined threshold value is the same as that in the first embodiment. As in the first embodiment, a test as an examination is actually conducted in advance under the same condition as the subculture timing determination. In this test, a statistical index value at the time point corresponding to the subculture timing is obtained to determine a time-dependent change index value, which is then defined as the predetermined threshold value. Even with the additional time-dependent change element, the characteristics of the statistical index value for the subculture timing determination are still maintained and effective as described in first embodiment.

As in the first example, if the determination data is created as a single representative value, that is, one piece of determination data, comparison is made to find whether the piece of determination data reaches the predetermined threshold value. If so, it is determined that the subculture timing has been reached. For two or more pieces of determination data, comparison is made to find whether a predetermined number of pieces of the two or more pieces of determination data reach the predetermined threshold value. If so, it may be determined that the subculture timing has been reached. It may also be determined that the subculture timing has been reached if all of the two or more pieces of determination data reach the predetermined threshold value. In this case, determination that the subculture timing has been reached is made relatively late, because the determination requires the time for all the pieces of determination data to reach the predetermined threshold value.

Conversely, it may be determined that the subculture timing has been reached if at least one of the two or more pieces of determination data reaches the predetermined threshold value. In this case, determination that the subculture timing has been reached can be made relatively early, because the determination requires only a piece of determination data to reach the predetermined threshold value.

### [Third embodiment]

Now, a subculture timing determination method and a system for the same in a third embodiment will be described. The third embodiment is an embodiment with a partially modified aspect of the first and second embodiments. The third embodiment differs from the first and second embodiments in that creating the determination data in the determination data creation step (S12) involves selecting at least three photographing time points as notable photographing time points from the plurality of photographing time points photographed in the image-capturing step (S11), and creating the determination data from the cell images at the notable photographing time points to perform the subculture timing determination. The determination data in the third embodiment also includes a time-dependent element. The configurations of the cell culture system 1 and the subculture timing determination unit 3 are the same as those in the first embodiment. The following description of the third embodiment will focus on the differences from the first and second embodiments, and what is the same as the first and second embodiments will not be described. As in the subculture determination process in the first embodiment illustrated in FIG. 2B, the subculture determination process in the third embodiment includes the image-capturing step (S11), the determination data creation step (S12), and the determination step (S13).

### (Culture surface image-capturing step)

As illustrated in FIG. 2E, in the third embodiment, cell images are photographed at multiple photographing time points in the image-capturing step (S11) as in the first and second embodiments. At each photographing time point, it is also similar in that the observation positions Op are selected at the same position such that an observation is made at a fixed point.

### (Creating determination data)

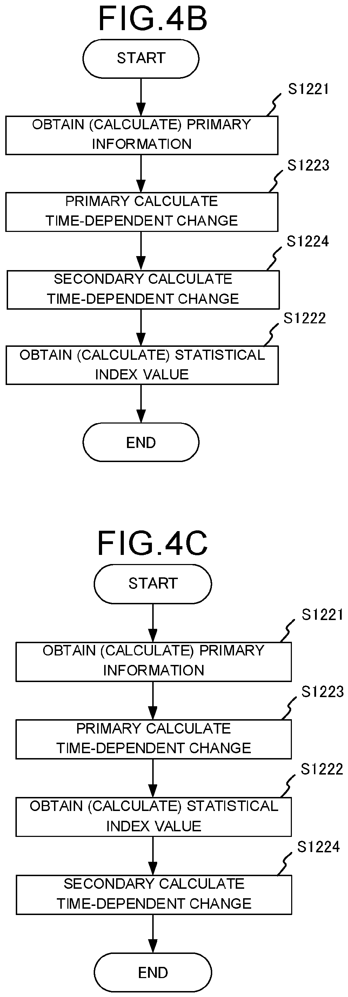

The determination data creation step (S12) is the same as that in the subculture determination process in the second embodiment illustrated in FIGS. 3A and 3B. In the third embodiment, creating the determination data in the determination data creation step (S12) includes a first example (FIG. 4A), a second example (FIG. 4B), and a third example (FIG. 4C). In the third embodiment, unlike in the second embodiment, the step of calculating the time-dependent change index values is performed in two stages: a primary time-dependent change step (S1223) and a secondary time-dependent change step (S1224). The first example (FIG. 4A) in this embodiment includes a primary information obtaining step (S1221) for obtaining primary information from cell images; a statistical index value calculation step (S1222) for subsequently calculating statistical index values from the primary information; a primary time-dependent change calculation step (S1223) for calculating primary time-dependent change index values of the primary information; and a secondary time-dependent change calculation step (S1224) for calculating a secondary time-dependent change index value from the primary time-dependent change index values. The second example (FIG. 4B) in this embodiment also includes a primary information obtaining step (S1221) for obtaining primary information from cell images; a primary time-dependent change calculation step (S1223) for calculating the amounts of change in primary time-dependent information of the primary information; a secondary time-dependent change calculation step (S1224) for calculating secondary time-dependent change index values from primary time-dependent change index values; and a statistical index value calculation step (S1222) for subsequently calculating a statistical index value of the secondary time-dependent change index values. Further, the third example (FIG. 4C) in this embodiment includes a primary information obtaining step (S1221) for obtaining primary information from cell images; a primary time-dependent change calculation step (S1223) for calculating the amounts of change in primary time-dependent information of the primary information; a statistical index value calculation step (S1222) for calculating statistical index values of primary time-dependent change index values; and a secondary time-dependent change calculation step (S1224) for calculating a secondary time-dependent change index value from the statistical index values of the primary time-dependent change index values.

In all the first to third examples in the third embodiment, the subculture timing determination uses cell images photographed at three or more photographing time points set as notable photographing time points. As in the second embodiment, the notable photographing time points include evaluation photographing time points and a reference photographing time point. For three notable photographing time points, cell images photographed at, for example, the first photographing time point, the second photographing time point, and the third photographing time point in FIG. 2E are used. As a representative example, the following description assumes using cell images photographed at three photographing time points. This embodiment is also applicable to four or more photographing time points used as the notable photographing time points, although such a case will be specifically described below as a fourth embodiment.

In both the first and second examples in the third embodiment, as in the second embodiment, the notable photographing time points may be selected in any of two manners: nearest comparison method and reference comparison method. In the nearest comparison method and the reference comparison method in the third embodiment, the notable photographing time points includes multiple evaluation photographing time points and/or multiple reference photographing time points. The following describes a case with one reference photographing time point and two evaluation photographing time points. In the representative example for the nearest comparison method, consecutive three photographing time points (a first photographing time point, a second photographing time point, and a third photographing time point) are selected as the notable photographing time points from multiple photographing time points. As in the second embodiment, in the nearest comparison method, the first, second, and third photographing time points are updated to the latest photographing time points as a new photographing time point appears.

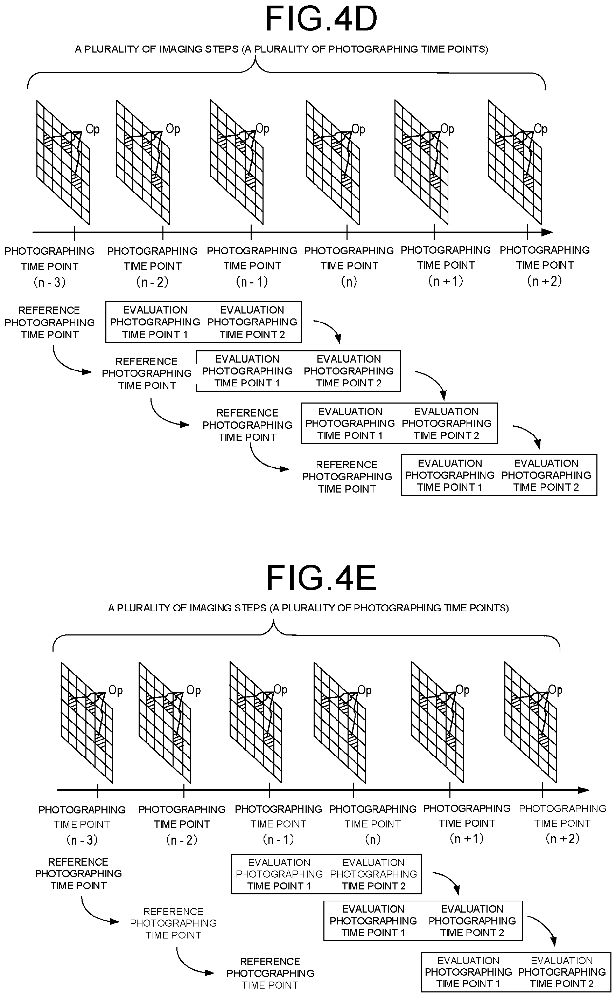

First, with reference to FIG. 4D, the nearest comparison method in the third embodiment will be specifically described. FIG. 4D illustrates a concept in which the notable photographing time points (the reference photographing time point and the evaluation photographing time points) for the nearest comparison method are selected from any photographing time points, **e.g.,** photographing time points (n-3) to (n+2). For example, the subculture timing determination is performed by selecting one reference photographing time point and two evaluation photographing time points as the notable photographing time points, including the photographing time point (n-3) set as the reference photographing time point, the photographing time point (n-2) set as a first evaluation photographing time point, and the photographing time point (n-1) set as a second evaluation photographing time point. If the determination indicates that the subculture timing has not been reached, the subculture timing determination is further performed by selecting the notable photographing time points that include the photographing time point (n-2) set as the reference photographing time point, the photographing time point (n-1) set as the first evaluation photographing time point, and the photographing time point n set as the second evaluation photographing time point. Similarly, the next notable photographing time points will include the photographing time point (n-1) set as the reference photographing time point, the photographing time point n set as the first evaluation photographing time point, and the photographing time point n+1 set as the second evaluation photographing time point. In this manner, subsequent subculture timing determination is performed while repeatedly updating the notable photographing time points by shifting these notable photographing time points as a new photographing time point arrives. As in the second embodiment, even in this case, when both the reference photographing time point and the evaluation photographing time point are shifted for update, each of the reference photographing time point and the evaluation photographing time point must be regularly shifted in order.

In the nearest comparison method, as illustrated in FIG. 4D, the notable photographing time points are typically selected to have no photographing time points intervening between the reference photographing time point and the first evaluation photographing time point. Alternatively, as in the second embodiment and as illustrated in FIG. 4E, the notable photographing time points may be selected to have any number of photographing time points not used for subculture timing determination intervening between the reference photographing time point and the first evaluation photographing time point. FIG. 4E illustrates an example of this. Further, in the nearest comparison method, the notable photographing time points may be set to have photographing time points not used for evaluation intervening between the reference photographing time point and the first evaluation photographing time point; or the notable photographing time points may be set to have photographing time points not used for evaluation intervening between the first evaluation photographing time point and the second evaluation photographing time point, irrespective of whether photographing time points not used for evaluation intervene between the reference photographing time point and the first evaluation photographing time point. Any time points may be used as the notable photographing time points for the subculture timing determination as long as the reference photographing time point, the first evaluation photographing time point, and the second evaluation photographing time point are spaced at fixed intervals and are selected in order in the same pattern. In the reference comparison method, which involves a fixed reference photographing time point and updated evaluation photographing time points, photographing time points may intervene between the reference photographing time point and the evaluation photographing time points. In this case, again, when the reference photographing time point and the first evaluation photographing time point and the second evaluation photographing time point are shifted for update, each of the reference photographing time point and the first evaluation photographing time point and the second evaluation photographing time point must be regularly shifted in order irrespective of whether photographing time points not used for evaluation intervene between the reference photographing time point, the first evaluation photographing time point, and the second evaluation photographing time point.

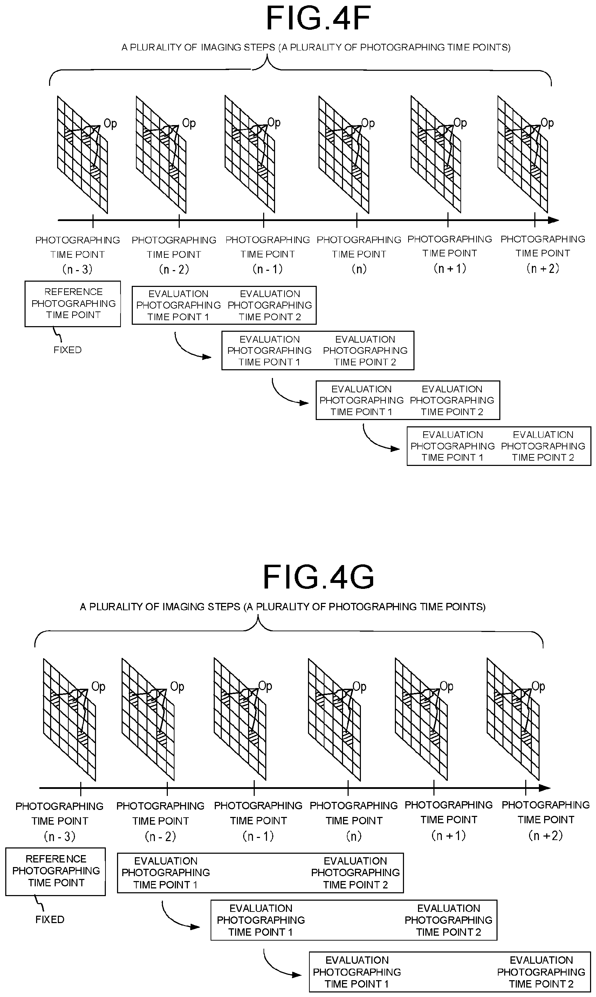

Next, with reference to FIG. 4F, the reference comparison method in the third embodiment will be specifically described. In the reference comparison method, the notable photographing time points including the reference photographing time point and the first and second evaluation photographing time points are selected as follows. FIG. 4F illustrates a concept selecting notable photographing time points in the reference comparison method, in which the notable photographing time points (the reference photographing time point and the evaluation photographing time points) are selected from a plurality of photographing time points from the photographing time points (n-3) to (n+2). In the reference comparison method, any initial one of the multiple photographing time points is determined as a fixed reference photographing time point, and any two of the photographing time points after the reference photographing time point are selected as the first and second evaluation photographing time points, respectively. If it is determined that the subculture timing has not been reached, the notable photographing time points (the reference photographing time point and the evaluation photographing time points) are determined by shifting only the first and second evaluation photographing time points and not shifting the reference photographing time point. For example, if the reference photographing time point is the photographing time point (n-3), the subculture timing determination is performed by selecting the notable photographing time points that include the photographing time point (n-3) fixed as the reference photographing time point, the photographing time point (n-2) set as the first evaluation photographing time point, and the photographing time point (n-2) set as the second evaluation photographing time point. After the determination, if the determination indicates that the subculture timing has not been reached, the subculture timing determination is further performed by selecting the notable photographing time points that include the photographing time point (n-2) still fixed as the reference photographing time point, the photographing time point (n-1) set as the first evaluation photographing time point, and the photographing time point (n-1) set as the second evaluation photographing time point. In this manner, subsequent subculture timing determination is performed until the subculture timing is reached while repeatedly updating the notable photographing time points by maintaining the reference evaluation photographing time point fixed at the photographing time point (n-3) and shifting the first and second evaluation photographing time points, as a photographing time point is added upon the arrival of new photographing timing. That is, the reference comparison method uses, at each occasion of subculture timing determination, the fixed reference photographing time point as well as the first and second evaluation photographing time points repeatedly updated as a new photographing time point is added. In the reference comparison method, as in the nearest comparison method, when the reference photographing time point and the first evaluation photographing time point and second evaluation photographing time point are shifted for update, each of the reference photographing time point and the first evaluation photographing time point and the second evaluation photographing time point must be regularly shifted in order.

Because the reference photographing time point in the reference comparison method is fixed, shifting the first evaluation photographing time point when a photographing time point is added upon the arrival of new photographing timing inevitably leaves any number of photographing time points not used for subculture timing determination intervening between the reference photographing time point and the first evaluation photographing time point. In this regard, the first evaluation photographing time point and the second evaluation photographing time point may also be set to have any number of photographing time points not used for subculture timing determination intervening between these time points. For example, FIG. 4G illustrates an example in which the subculture timing determination is performed by selecting the notable photographing time points that include the photographing time point (n-3) fixed as the reference photographing time point, the photographing time point (n-2) set as the first evaluation photographing time point, and the photographing time point (n) set as the second evaluation photographing time point. The photographing time point (n-1) is not selected as an evaluation photographing time point and therefore not used for the subculture timing determination. After the determination, if the determination indicates that the subculture timing has not been reached, the subculture timing determination is further performed by selecting the notable photographing time points that include the photographing time point (n-3) still fixed as the reference photographing time point, the photographing time point (n-1) set as the first evaluation photographing time point, and the photographing time point (n+1) set as the second evaluation photographing time point. In this manner, subsequent subculture timing determination is performed until the subculture timing is reached while repeatedly updating the notable photographing time points by maintaining the reference evaluation photographing time point fixed at the photographing time point (n-3) and shifting the first and second evaluation photographing time points, as a photographing time point is added upon the arrival of new photographing timing. Again, as described above, when the reference photographing time point and the first evaluation photographing time point and the second evaluation photographing time point are shifted for update, each of the reference photographing time point and the first evaluation photographing time point and the second evaluation photographing time point must be regularly shifted in order.

In the nearest comparison method, since the reference photographing time point and the evaluation photographing time point (the first and second evaluation photographing time points) move together, it will be a relative comparison of the primary information at each photographing time point of the reference photographing time point and the evaluation photographing time point. By contrast, in the reference comparison method, since the reference photographing time point is fixed, the comparison indicates the absolute difference between the primary information at the reference photographing time point (fixed over time) and the primary information at each evaluation photographing time point (moving over time). Although any time interval may be set between the photographing time points, a fixed time interval may be preferably set between the photographing time points especially in the nearest comparison method in which two consecutive photographing time points are selected.

The following describes the determination data creation step (S12) in the third embodiment separately for each of the first to third examples. The primary information obtaining step (S1221) in all the first to third examples is the same as the primary information obtaining step (S1211) in the second embodiment and therefore will not be described.

### <First example>

### (Obtaining statistical index value)

In the first example, as described above, as illustrated in FIG. 4A, the primary information at each photographing time point is obtained in the primary information obtaining step (S1221), and thereafter, the statistical index value at each photographing time point is calculated from the primary information of each photographing time point in the statistical index value obtaining step (S1222). As with the first embodiment, the statistical index value is an index value defined as a representative value typically indicative of the primary information, and is at least any of, for example, a sum, an average value, a variance, and a deviation such as a standard deviation. The sum is a sum of the number of cells at a plurality of observation positions, a sum of cell occupation areas, a sum of single cell areas, and a sum of the distances between cells. The average value is an average value of the number of cells at a plurality of observation positions, an average value of cell densities, an average value of occupation areas of the number of cells, an average value of single cell areas, and an average value of the distances between cells. The variance and the deviation are a variance and a deviation of the number of cells of a plurality of observation positions, a variance and a deviation of cell densities, a variance and a deviation of cell occupation areas, a variance and a deviation of single cell areas, and a variance and a deviation of the distances between cells. Here, as with the first embodiment, the variance or the deviation serving as statistical index values, which are indexes of the uniformity of cells in the culture vessel, are more suitable for being able to determine the subculture timing than selecting the sum or the average value as a statistical index value.

(Primary time-dependent change calculation step (obtaining primary time-dependent change index values)) Then, in the primary time-dependent change calculation step (S1223), primary time-dependent change index values are calculated from the statistical index values of the primary information calculated at the photographing time points. The primary time-dependent change index values are the amounts of change, or the rates of change, which are increases in the statistical index value of the primary information between the photographing time points, that is, between the first and second photographing time points and between the second and third photographing time points. One primary time-dependent change index value is created for every two values of the primary information; as a whole, primary time-dependent change index values one fewer than the number of statistical index values of the primary information are created. Primary time-dependent change index values one fewer than the number of observation positions Op or the representative values are calculated.

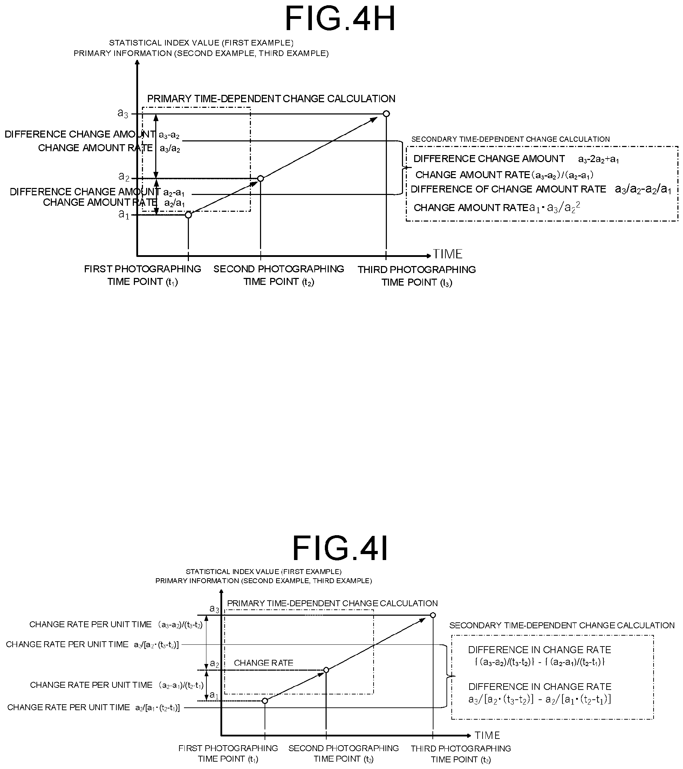

This will be described with reference to FIGS. 4H and 4I. FIGS. 4H and 4I are diagrams illustrating concepts of changes in value (vertical axis) along time (horizontal axis) in the first and second examples. The vertical axis in the first example indicates statistical index values, whereas the vertical axis in the second example to be described below indicates values of the primary information. FIGS. 4H and 4I illustrate examples of the nearest comparison method for three consecutive photographing time points (a first photographing time point, a second photographing time point, and a third photographing time point). For the above-described reference comparison method in which nonconsecutive photographing time points are selected as the first, second, and third photographing time points, FIGS. 4H and 4I may still be applied by assuming photographing time points not shown between the first, second, and third photographing time points. The characteristics of the tendency of increase in the primary time-dependent change index values in the reference comparison method and the nearest comparison method are the same as those in the second embodiment.

In the calculation of the primary time-dependent change index values, two photographing time points (the first and second photographing time points) are selected from multiple photographing time points. In the examples illustrated in FIGS. 4H and 4I, the latest two consecutive photographing time points are selected as the first photographing time point (t₁), the second photographing time point (t₂), and the third photographing time point (t₃). FIGS. 4H and 4I also show increases of the statistical index value (a₁) at the first photographing time point (t₁) to a statistical index value (a₂) at each of the second photographing time point (t₂) and the third photographing time point (t₃). At each of the photographing time points, it is determined whether the subculture timing has reached. If not, the latest three photographing time points including a new photographing time point are selected again to create the determination data.

In the first example, the calculation of the primary time-dependent change index values focuses on the two intervals between the three photographing time points selected for use in the determination, that is, pairs of photographing time points selected from the three photographing time points. Thus, two pairs of photographing time points are extracted from the three photographing time points to calculate a change of the value between the photographing time points of each pair as a primary time-dependent change index value. For example, the first example selects two photographing time points (the first and second photographing time points) as a first pair, and two photographing time points (the second and third photographing time points) as a second pair, to calculate primary time-dependent change index values between the photographing time points of the respective pairs. The two photographing time points extracted from the photographing time points for use in the determination are herein referred to as notable photographing time points. In the second embodiment, which uses a single combination of notable photographing time points, only one time-dependent change index value is obtained. By contrast, in this embodiment, one primary time-dependent change index value is obtained from two notable photographing time points; thus, a total of two primary time-dependent change index values are obtained, one from each of the two pairs of notable photographing time points. That is, for three photographing time points selected for use in the determination, two pairs of notable photographing time points are extracted, and two primary time-dependent change index values are calculated.

The primary time-dependent change index value between the two notable photographing time points in each pair is calculated in the same manner as in the second embodiment. For example, the primary time-dependent change index value may be the amount of difference between "the statistical index value of the primary information at the evaluation photographing time point among the notable photographing time points" and "the statistical index value of the primary information at the reference photographing time point among the notable photographing time points". That is, as illustrated in FIG. 4H, the primary time-dependent change index value may be the difference change amount, which is the value of the amount of difference in the statistical index value of the primary information, for each of the pairs of notable photographing time points, i.e., between the first photographing time point (the reference photographing time point) and the second photographing time point (the evaluation photographing time point) and between the second photographing time point (the reference photographing time point) and the third photographing time point (the evaluation photographing time point). The time-dependent index values in the form of the difference change amounts may be the difference change amounts (a₂-a₁) and (a₃-a₂), which are the amount of difference of the statistical index value of the primary information at the second photographing time point from the statistical index value of the primary information at the first photographing time point.

The primary time-dependent change index value may also be the percentage change amount of "the statistical index value of the primary information at the evaluation photographing time point among the notable photographing time points" to "the statistical index value of the primary information at the reference photographing time point among the notable photographing time points." The primary time-dependent change index values in the form of the percentage change amount are the rate of change a₂/a₁ of "the statistical index value of the primary information at the second photographing time point (the evaluation photographing time point)" with respect to "the statistical index value of the primary information at the first photographing time point (the reference photographing time point)", and the rate of change a₃/a₂ of "the statistical index value of the primary information at the third photographing time point (the evaluation photographing time point)" with respect to "the statistical index value of the primary information at the second photographing time point (the reference photographing time point)." These rates mean the value of the ratio of "the total sum of the numbers of cells at the second photographing time point" to "the total sum of the numbers of cells at the first photographing time point," and the ratio of "the total sum of the numbers of cells at the third photographing time point" to "the total sum of the numbers of cells at the second photographing time point," respectively.

The primary time-dependent change index value may also be the difference-percentage change amount of "the amount of difference between 'the statistical index value of the primary information at the evaluation photographing time point' and 'the statistical index value of the primary information at the reference photographing time point'" to "the statistical index value of the primary information at the reference photographing time point among the notable photographing time points." The primary time-dependent change index values in the form of the difference-percentage change amount may be represented as (a₂-a₁) /a₁, which is the ratio of "the amount of increase between the statistical index value of the primary information at the first photographing time point and the statistical index value of the primary information at the second photographing time point" to "the statistical index value of the primary information at the first photographing time point," and (a₃-a₂) /a₂, which is the ratio of "the amount of increase between the statistical index value of the primary information at the second photographing time point and the statistical index value of the primary information at the third photographing time point" to "the statistical index value of the primary information at the second photographing time point." In an example in which the primary information is the number of cells, these ratios mean the value of the ratio of "an increase in the total sum of the numbers of cells from the first photographing time point to the second photographing time point" to "the total sum of the numbers of cells at the first photographing time point," and the ratio of "an increase in the total sum of the numbers of cells from the second photographing time point to the third photographing time point" to "the total sum of the numbers of cells at the second photographing time point."

Further, as illustrated in FIG. 4I, for the time interval between notable photographing time points, the primary time-dependent change index value may be the difference change amount, the percentage change amount, and the difference-percentage change amount, as described above, between the statistical index value of the primary information at the reference photographing time point and the statistical index value of the primary information at an evaluation photographing time point. That is, for each of the time intervals of the two pairs of photographing time points (the first and second photographing time points, and the second and third photographing time points), the primary time-dependent change index value may be the difference change amount, the percentage change amount, and the difference-percentage change amount, between the statistical index value of the primary information at the first photographing time point (the reference photographing time point) and the statistical index value of the primary information at the second photographing time point (the evaluation photographing time point), and between the statistical index value of the primary information at the second photographing time point (the reference photographing time point) and the statistical index value of the primary information at the third photographing time point (the evaluation photographing time point). For the first photographing time point (the reference evaluation time point) and the second photographing time point (the evaluation photographing time point), the primary time-dependent change index value may be represented as (a₂-a₁)/(t₂-t₁), which is the rate of change per unit time of the difference change amount; (a₂/[a₁•(t₂-t₁)]), which is the rate of change per unit time of the percentage change amount; and (a₂-a₁)/[a₁•(t₂-t₁)]), which is the rate of change per unit time of the difference-percentage change amount. For the second photographing time point (the reference evaluation time point) and the third photographing time point (the evaluation photographing time point), the primary time-dependent change index value may be represented as (a₃-a₂)/(t₃-t₂), which is the rate of change per unit time of the difference change amount; (a₃/3₂•(t₃-t₂)]), which is the rate of change per unit time of the percentage change amount; and (a₃-a₂)/[a₂•(t₃-t₂)]), which is the rate of change per unit time of the difference-percentage change amount. These primary time-dependent change index values mean the increase rate per unit time of the statistical index value of the primary information between the first photographing time point (the reference photographing time point) and the second photographing time point (the evaluation photographing time point), and the increase rate per unit time of the statistical index value of the primary information between the second photographing time point (the reference photographing time point) and the third photographing time point (the evaluation photographing time point), respectively, which are also rates of increase. In an example in which the primary information is the number of cells, it is the increasing rate of the number of cells per unit time, namely, the rate of increase (growth rate). The rate of increase (growth rate) as used herein is an approximated simple rate obtained as a difference value by subjecting the statistical index values at two photographing time points to a linear approximation.

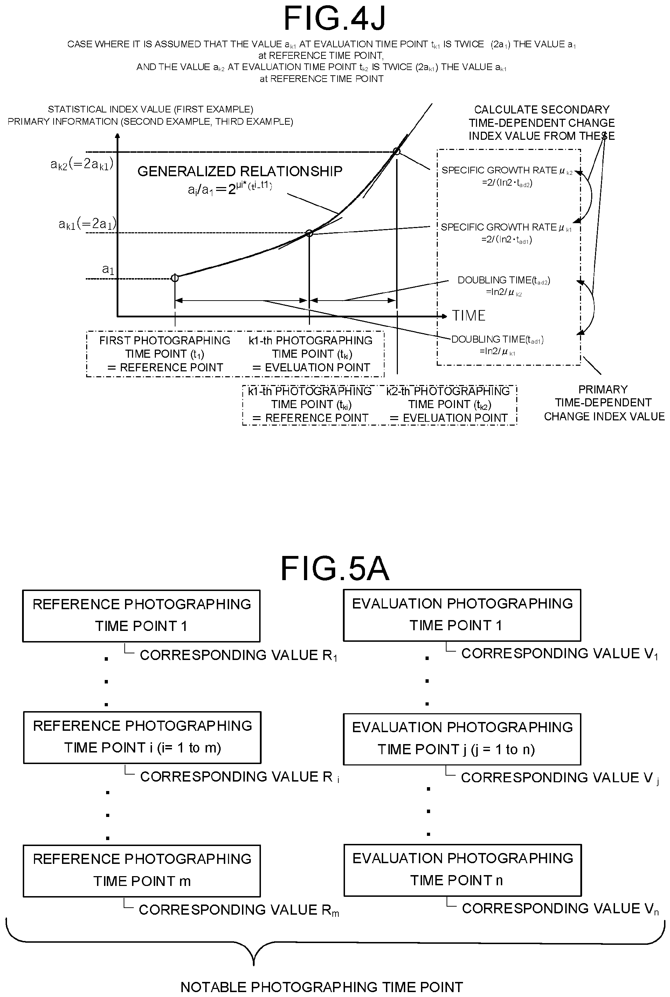

The ratio of increase (growth rate) can be defined based on a wide range of definitions as long as it can represent a time-dependent change in the statistical index value of the primary information, as in the second embodiment. That is, as illustrated in FIG. 4J, the rate of increase (growth rate) as a primary time-dependent change index value may be a specific growth rate rather than a simple speed defined as the amount of difference along time, as in the second embodiment. The specific growth rate may be defined as in the second embodiment, and therefore the description in the second embodiment is applicable. The following reiterates this for confirmation. The first photographing time point t₁ is the reference photographing time point serving as the reference time point in the calculation of the primary time-dependent change index value. The statistical index value aᵢ[i = 2 to n] of the primary information at a photographing time point tᵢ, which is each evaluation photographing time point serving as the evaluation time point in the calculation of the primary time-dependent change index value, can be approximated as a value changing so that the approximated generalized relationship aᵢ/a₁=e^{[(µi)•(ti-t1)]} holds true with respect to the statistical index value a₁ of the primary information at the first photographing time point t₁. Here, µᵢ is the specific growth rate at the photographing time point tᵢ [i = 2 to n], where µᵢ=[ln(aᵢ/a₁)]/(tᵢ-t₁).

Here, assuming that the statistical index value of the primary information is aₖ₁ for i=k₁[2<k₁<n] among the photographing time points tᵢ[2<i<n], the specific growth rate is µₖ₁= [ln(aₖ₁/a₁)]/(tₖ₁-t₁). Assuming that the statistical index value of the primary information is aₖ₂ at an evaluation photographing time point for i=k₂[k₁<k₂<n] among the photographing time points tᵢ[i<n], the specific growth rate is
µₖ₂= [ln(aₖ₂/aₖ₁)]/(tₖ₂-tₖ₁). These specific growth rates µₖ₂ and µₖ₁ are used to calculate a secondary time-dependent change index value to be described below, which is calculated from the two specific growth rates µₖ₂ and µₖ₁ calculated from the values at the two time points selected among the three time points in this manner.

Further, as in the second embodiment, a multiplying time can be used as the primary time-dependent change index value. That is, assume that the statistical index value aₖ₁ of the primary information for i = k₁ [2<k₁<n] among the photographing time points tᵢ [2<i<n] is twice the statistical index value a₁ at the first photographing time point t₁, which is the reference photographing time point (aₖ₁ = 2a₁). The time from the first photographing time point t₁ serving as the reference photographing time point to the k1-th photographing time point tₖ₁ [2<k₁<n] serving as an evaluation photographing time point is what is called a multiplying time t_{ad1} (= tₖ₁-t₁). For reference purposes, because the relationship aₖ₁/a₁ = e^{(µk1•tad1)} = 2 holds true, the specific growth rate in the multiplying time t_{ad1} is specifically µₖ₁ = ln2/t_{ad1} = ln2/(tₖ₁-t₁).

Similarly, assume that the statistical index value aₖ₂ of the primary information for i = k₂ [k₁<k₂<n] among the photographing time points tᵢ [i<n] is twice the statistical index value aₖ₁ at the k1-th photographing time point tₖ₁ when the reference photographing time point is the k1-th photographing time point tₖ₁ (aₖ₂ = 2ₐₖ₁). The time from the k1-th photographing time point tₖ₁ serving as the reference photographing time point to the k2-th photographing time point tₖ₂ serving as an evaluation photographing time point is what is called a multiplying time t_{ad2} (= tₖ₂-tₖ₁). For reference purposes, again, because the relationship aₖ₂/aₖ₁ = e^{(µk2•tad2)} = 2 holds true, the specific growth rate in the multiplying time t_{ad2} is specifically µₖ₂ = 2/ (ln2•t_{ad2}) = ln2/ (tₖ₂-tₖ₁). The secondary time-dependent change index value to be described below represented as a multiplying time is determined from the two multiplying times t_{ad1} (= tₖ₁-t₁) and t_{ad2} (= tₖ₁-t₁) calculated from the values at the two time points selected among the three time points in this manner.

In addition to the multiplying time, a predetermined multiplying time (txi) can be adopted as the rate of increase (growth rate), as in the second embodiment; the predetermined multiplying time (txi) is the time required for the amount at the evaluation time point to be a predetermined multiple of the amount at the reference time point. That is, in the above-described example, assume that the statistical index value aₖ₁ of the primary information for i = k₁ [2<k₁<n] among the photographing time points tᵢ [2<i<n] is a predetermined multiple (X times) of the statistical index value a₁ of the primary information at the photographing time point t₁, which is the reference time point, and the statistical index value aₖ₂ of the primary information at an evaluation photographing time point for i = k₂ [k₁<k₂<n] is a predetermined multiple (X times) of the statistical index value aₖ₁ of the primary information at the photographing time point tₖ₁, which is the reference time point. If the increase factor per unit time is e^{µi}, the relationship e^{[(µi)•(txi)]} = X holds true, and the predetermined multiplying times txₖ₁ = lnX/µₖ₁ = (lnX)•(tₖ₁-t₁)/ln(aₖ₁/a₁) and t_{Xk2} = lnX/µₖ₂ = (lnX)•(tₖ₂-tₖ₁)/ln(aₖ₂/aₖ₁) are obtained at i = k₁ and i = k₂, respectively. As in the second embodiment, the above-described multiplying time is the predetermined multiplying time for X = 2. These predetermined multiplying times are used to calculate the secondary time-dependent change index value to be described below, which are calculated from the two predetermined multiplying times calculated from the values at the two time points selected among the three time points in this manner.

(Secondary time-dependent change calculation step (obtaining primary time-dependent change index value)) Next, in the secondary time-dependent change calculation step (S1224), the secondary time-dependent change index value is calculated from the primary time-dependent change index value. To the secondary time-dependent change index value, the difference change amount as an index of the increment amount of the primary time-dependent change index value, the percentage change amount, and the rate of change per unit time per unit time can be applied, respectively. That is, the difference change amount or the percentage change amount between the primary time-dependent change index value (a pre-change value) for the time from the first photographing time point to the second photographing time point and each primary time-dependent change index value (a post-change value) for the time from the second photographing time point to the third photographing time point can be applied.

In the case of applying the difference change amount to the secondary time-dependent change index value, for example, as illustrated in FIG. 4H, when the difference change amount of the statistical index values is selected as the primary time-dependent change value and the difference change amounts a₂-a₁ and a₃-a₂ of the statistical index values are obtained respectively for the primary time-dependent change index value (pre-change value) for the time from the first photographing time point to the second photographing time point and the primary time-dependent change index value (post-change value) for the time from the second photographing time point to the third photographing time point, the secondary time-dependent change index value can be the difference change amount (a₃-a₂)-(a₂-a₁) = (a₃-2a₂+a₁) for which the pre-change value is subtracted from the post-change value.

In addition, in the case of applying the difference change amount to the secondary time-dependent change index value, when the percentage change amount of the statistical index values is selected as the primary time-dependent change value and the percentage change amounts a₂/a₁ and a₃/a₂ of the statistical index values are obtained for the primary time-dependent change index value (pre-change value) for the time from the first photographing time point to the second photographing time point and the primary time-dependent change index value (post-change value) for the time from the second photographing time point to the third photographing time point, the secondary time-dependent change index value can be (a₃/a₂)-(a₂/a₁) which is the difference change amount of the percentage change amounts.

In the case of applying the difference change amount to the secondary time-dependent change index value, when the difference percentage change amount of the statistical index values is selected as the primary time-dependent change value and (a₂-a₁)/a₁ and (a₃-a₂)/a₂ are obtained, the secondary time-dependent change index value can be [(a₃-a₂)a₂-(a₂-a₁)a₁] which is the difference change amount of the percentage change amounts.

Further, in the case of applying the difference change amount to the secondary time-dependent change index value, when the rate of change per unit time of the difference change amounts is selected as the primary time-dependent change index value and (a₂-a₁)/(t₂-t₁) and (a₃-a₂) /(t₃-t₂)are obtained, as illustrated in FIG. 4I, the secondary time-dependent change index value can be [(a₃-a₂)/(t₃-t₂)-(a₂-a₁) /(t₂-t₁)] which is the difference change amount of the rates of change per unit time, respectively.

In addition, in the case of applying the difference change amount to the secondary time-dependent change index value, when the rate of change per unit time of the percentage change amounts is selected as the primary time-dependent change index value and a_{3/} [a₂·(t₃-t₂)] and a₂/[a₁·(t₂-t₁)] are obtained, as illustrated in FIG. 4I, the secondary time-dependent change index value can be a₃/[a₂·(t₃-t₂)]-a₂/[a₁·(t₂-t₁)] which is the difference change amount of the rates of change per unit time as well.

In the case of applying the difference change amount to the secondary time-dependent change index value, when the rate of change per unit time of the difference percentage change amounts is selected as the primary time-dependent change index value and (a₂-a₁)/[a₁·(t₂-t₁)] and (a₃-a₂) / [a₂·(t₃-t₂)] are obtained, as illustrated in FIG. 4I, the secondary time-dependent change index value can be also (a₃-a₂) / [a₂·(t₃-t₂)]- (a₂-a₁)/a₁·(t₂-t₁)] which is the difference change amount of the rates of change per unit time, respectively.

In the case of applying the difference change amount to the secondary time-dependent change index value, when the specific growth rate is selected as the primary time-dependent change index value, as illustrated in FIG. 4J, the secondary time-dependent change index value is, as the difference change amount, (µₖ₂-µₖ₁) = [ln(aₖ₂/aₖ₁)]/(tₖ₂-tₖ₁)-[ln(aₖ₁/a₁)]/(tₖ₁-t₁).

In the case of applying the difference change amount to the secondary time-dependent change index value, when the multiplying time is selected as the primary time-dependent change index value, as illustrated in FIG. 4J, the secondary time-dependent change index value is, as the difference change amount, t_{ad2}-t_{ad1} = (tₖ₂-tₖ₁)-(tₖ₁-t₁)= tₖ₂-t₁.

In the case of applying the percentage change amount to the secondary time-dependent change index value, when the difference change amount which is the difference value of the statistical index values is selected as the primary time-dependent change index value and (a₂-a₁) and (a₃-a₂) are obtained, it can be (a₃-a₂)/(a₂-a₁) as a percentage of the primary time-dependent change index value (post-change value) for the time from the second photographing time point to the third photographing time point to the primary time-dependent change index value (pre-change value) for the time from the first photographing time point to the second photographing time point.

In the case of applying the percentage change amount to the secondary time-dependent change index value, when the percentage change amount of the statistical index values is selected as the primary time-dependent change index value and (a₂/a₁) and (a₃/a₂) are obtained, it can be (a₃/a₂)÷(a₂/a₁) = (a₁·a₃)/a₂² as the percentage of the primary time-dependent change index value (post-change value) for the time from the second photographing time point to the third photographing time point to the primary time-dependent change index value (pre-change value) for the time from the first photographing time point to the second photographing time point.

In the case of applying the percentage change amount to the secondary time-dependent change index value, when the difference percentage change amount of the statistical index values is selected as the primary time-dependent change index value and (a₂-a₁)/a₁ and (a₃-a₂)/a₂ are obtained, it can be (a₃-a₂) /a₂÷(a₂-a₁)/a₁, = [a₁·(a₃-a₂) ]/[a₂·(a₂-a₁)] as the percentage of the primary time-dependent change index value (post-change value) for the time from the second photographing time point to the third photographing time point to the primary time-dependent change index value (pre-change value) for the time from the first photographing time point to the second photographing time point.

In the case of applying the percentage change amount to the secondary time-dependent change index value, when the rate of change per unit time of the difference change amounts is selected as the primary time-dependent change index value and (a₃-a₂)/(t₃-t₂) and (a₂-a₁)/(t₂-t₁) are obtained, it can be [(a₃-a₂) /(t₃-t₂)]/[(a₂-a₁)/(t₂-t₁)] = [(a₃-a₂)·t₂-t₁)]/[(a₂-a₁)/(t₃-t₂)].

In the case of applying the percentage change amount to the secondary time-dependent change index value, when the rate of change per unit time of the percentage change amounts is selected as the primary time-dependent change index value and a₂/[a₁·(t₂-t₁)] and a₃/[a₂·(t₃-t₂)] are obtained, the secondary time-dependent change index value can be [(a₃-a₂) /(t₃-t₂)]÷[(a₂-a₁)/ (t₂-t₁)] = [(a₃-a₂)·(t₂-t₁)/(t₃-t₂)·(a₂-a₁)].

In the case of applying the percentage change amount to the secondary time-dependent change index value, when the rate of change per unit time of the difference percentage change amounts is selected as the primary time-dependent change index value and (a₂-a₁)/[a₁·(t₂-t₁)] and (a₃-a₂)/[a₂·(t₃-t₂)] are obtained, the secondary time-dependent change index value can be (a₃-a₂)/[a₂·(t₃-t₂)]÷(a₂-a₁)/a₁.(t₂-t₁)] as well.

In the case of applying the percentage change amount to the secondary time-dependent change index value, when the specific growth rate is selected as the primary time-dependent change index value, as illustrated in FIG. 4J, the secondary time-dependent change index value is, as the percentage change amount, µₖ₂/µₖ₁ = (tₖ₁-t₁)·[ln(aₖ₂/aₖ₁)]/(tₖ₂-tₖ₁)·[ln(aₖ₁/a₁)]/.

In the case of applying the percentage change amount to the secondary time-dependent change index value, when the multiplying time is selected as the primary time-dependent change index value, as illustrated in FIG. 4J, the secondary time-dependent change index value is, as the difference change amount, t_{ad2}/t_{ad1} = (tₖ₂-tₖ₁)/ (tₖ₁-t₁).

Similarly, in the case of applying the difference percentage change amount to the secondary time-dependent change index value, when the difference change amount which is the difference value of the statistical index values is selected as the primary time-dependent change index value and (a₂-a₁) and (a₃-a₂) are obtained, the secondary time-dependent change index value can be [(a₃-a₂)- (a₂-a₁) ]/(a₂-a₁) = (a₃-2a₂+a₁)/(a₂-a₁) .

In the case of applying the difference percentage change amount to the secondary time-dependent change index value, when the percentage change amount of the statistical index values is selected as the primary time-dependent change index value and (a₂/a₁) and (a₃/a₂) are obtained, the secondary time-dependent change index value can be [(a₃/a₂)- (a₂/a₁)]/ (a₂/a₁) . In the case of applying the difference percentage change amount to the secondary time-dependent change index value, when the difference percentage change amount of the statistical index values is selected as the primary time-dependent change index value and (a₂-a₁) /a₁ and (a₃-a₂) /a₂ are obtained, the secondary time-dependent change index value can be {[(a₃-a₂)/a₂]-[(a₂-a₁)/a₁]}/(a₂-a₁) /a₁.

In the case of applying the difference percentage change amount to the secondary time-dependent change index value, when the rate of change per unit time of the difference change amounts is selected as the primary time-dependent change index value and (a₃-a₂)/(t₃-t₂)and (a₂-a₁)/(t₂-t₁) are obtained, it can be {[(a₃-a₂)/(t₃-t₂)-(a₂-a₁)/ (t₂-t₁)]}/(a₂-a₁) /(t₂-t₁) = { [(a₃-a₂)/(t₃-t₂)]-[(a₂-a₁)/(t₂-t₁)]}·(t₂-t₁)/(a₂-a₁).

In the case of applying the difference percentage change amount to the secondary time-dependent change index value, when the rate of change per unit time of the percentage change amounts is selected as the primary time-dependent change index value and a₂/[a₁·(t₂-t₁)] and a₃/[a₂·(t₃-t₂)] are obtained, the secondary time-dependent change index value can be {a₃/[a₂·(t₃-t₂)]-a₂/[a₁·(t₂-t₁)]}/a₂/a₁. (t₂-t₁)] = {a₃/[a₂·(t₃-t₂)]-a₂/[a₁·(t₂-t₁)]}·[a₁·(t₂-t₁)]/a₂.

In the case of applying the difference percentage change amount to the secondary time-dependent change index value, when the rate of change per unit time of the difference percentage change amounts is selected as the primary time-dependent change index value and (a₂-a₁)/[a₁·(t₂-t₁) ] and (a₃-a₂)/[a₂·(t₃-t₂)] are obtained, the secondary time-dependent change index value can be also {[(a₃-a₂) /[a₂·(t₃-t₂)]- [(a₂-a₁)/[a₁·(t₂-t₁)]}/{(a₂-a₁)/[a₁·(t ₂-t₁)} which is the difference change amount of the rates of change per unit time, respectively.

In the case of applying the difference percentage change amount to the secondary time-dependent change index value, when the specific growth rate is selected as the primary time-dependent change index value, as illustrated in FIG. 4J, the secondary time-dependent change index value is, as the difference change amount, (µₖ₂-µₖ₁)/µₖ₁ == {[ln(aₖ₂/aₖ₁)]/(tₖ₂-tₖ₁)- [ln(aₖ₁/a₁)]/(tₖ₁-t₁)}/ [ln(aₖ₁-a₁)] /(tₖ₁-t₁)}.

In the case of applying the difference percentage change amount to the secondary time-dependent change index value, when the multiplying time is selected as the primary time-dependent change index value, as illustrated in FIG. 4J, the secondary time-dependent change index value is, as the difference change amount, (t_{ad2}- t_{ad1})/t_{ad1} = [(tₖ₂-tₖ₁)-(tₖ₁-t₁)]/(tₖ₁-t₁)= (tₖ₂-t₁)/(tₖ₁-t₁).

Further, when the specific growth rate and the multiplying time are selected as the primary time-dependent change index value, the rate of change per unit time can be applied to the secondary time-dependent change index value. Since the secondary time-dependent change index value is calculated similarly to the above, this case is omitted here.

Since one secondary time-dependent change index value is created for the two primary time-dependent change index values, as a whole, the secondary time-dependent change index values one fewer than the number of the primary time-dependent change index values are created and the secondary time-dependent change index values two fewer than the statistical index values of the primary information are created. The secondary time-dependent change index values two fewer than the number of the observation positions Op or the number of the representative values are obtained. With the above, the determination data creation step (S12) is ended, and the secondary time-dependent change index values obtained above are defined as the determination data. The plurality of secondary time-dependent change index values may be the same number of pieces of the determination data, or the plurality of secondary time-dependent change index values may be turned to one time-dependent change index value by the average value, the maximum value, or the minimum value, and it may be defined as one piece of the determination data.

Even though the primary time-dependent change index value is interposed, the secondary time-dependent change index value in the first example is information indicating a time-dependent element of the primary information similarly to the second embodiment, and quantitatively indicates the state of the cells on the culture surface at the predetermined observation positions Op at the plurality of predetermined points selected from the culture vessel. In the first example, the time-dependent change index value is of the sum as the statistical index value at the positions of the predetermined observation positions Op, and the representative value can accurately indicate the space where the cells can spread or proliferate in the culture vessel. The time-dependent change index value may be calculated according to each selection of the number of cells, the cell density, the cell occupation area, the single cell area, the distance between cells, and the like that are the primary information and each selection of the sum, the variance, and the deviation that are the statistical index values. The determination data in the first example means the representative value of acceleration of the change of the representative value of the primary information in the case where the sum or the average value is selected as the statistical index value, and means acceleration of the change of dispersion of the primary information in the case where the variance or the deviation is selected as the statistical index value.

### (Subculture determination)

After creation of the determination data is completed in the determination data creation step (S12), the determination step (S13) of determining whether or not the subculture timing is reached is executed. In the determination step (S13), the subculture timing is determined based on the determination data created in the determination data creation step (S12). In the determination step (S13), the determination data which is the secondary time-dependent change index value calculated in the time-dependent change calculation step (S1213) and the predetermined threshold value are compared. Here, it is also similar to the second embodiment that it is determined that the subculture timing is reached when the determination data reaches the predetermined threshold value. In the first example of the third embodiment as well, at the subculture timing, some inflection point represented by the extremal value such as the local minimum or the local maximum in the time variation of the secondary time-dependent change index value is observed. A time point at which such an inflection point of the time variation is observed is defined as a time point at which the subculture timing is reached, and is set as the threshold value.

Similarly to the second embodiment, in the case where the number of pieces of the determination data is one, whether or not the one piece of the determination data has reached the predetermined threshold value is compared, and when the predetermined threshold value is reached, it is determined that the subculture timing is reached. In the case where the number of pieces of the determination data is two or more, whether or not the predetermined number of pieces of the determination data among the two or more pieces of the determination data have reached the predetermined threshold value is compared, and when the predetermined threshold value is reached, it can be determined that the subculture timing is reached.

Further, it may be determined that the subculture timing is reached in the case where all the pieces of the determination data of the two or more pieces of the determination data have reached the predetermined threshold value. In this case, whether or not the subculture timing is reached can be determined early. In this case, whether or not the subculture timing is reached is determined relatively late.

Conversely, it may be determined that the subculture timing is reached in the case where at least one piece of the determination data among the two or more pieces of the determination data has reached the predetermined threshold value. In this case, whether or not the subculture timing is reached can be determined early.

The predetermined threshold value is similar to that in the first embodiment and the second embodiment in the case of the reference comparison method. Similarly to the first embodiment, a test as an examination is actually conducted in advance under the same condition as the determination of the subculture timing, the primary time-dependent change index value and the secondary time-dependent change index value for the statistical index value at the time point to be the subculture timing at which spreading or proliferation of cells is not observed are obtained in the test, and it is defined as the predetermined threshold value. Even if the time-dependent change element is added, the characteristics that the statistical index value has in the determination of the subculture timing are not lost, and it is similar to the form described in the first embodiment and the second embodiment.

In addition, in the case of the nearest comparison method of selecting three photographing time points as the three consecutive photographing time points, the predetermined threshold value is as follows. As described above, the secondary time-dependent change index value is the increment of the primary time-dependent change index value. As described in the second embodiment, since the primary time-dependent change index value is the increment of the statistical index value of the primary information, the primary time-dependent change index value gradually increases in the initial state and increases so that cells drastically spread or proliferate at a certain time point. Then, thereafter, the increase rate has a trend of gradually decreasing, taking the local maximum when the subculture timing is reached, and shifting to decrease. Therefore, the secondary time-dependent change index value gradually increases according to the increase of the increasing trend of the primary time-dependent change index value in the initial state, takes a positive local maximum, and gradually decreases according to attenuation of the secondary time-dependent change index value. Then, at the local maximum of the primary time-dependent change index value at which the subculture timing is reached, the secondary time-dependent change index value has a trend of inversely taking a negative local minimum and shifting to increase. Therefore, the local minimum is set as the predetermined threshold value. In addition, similarly to the second embodiment, since the subculture timing is already reached when the secondary time-dependent change index value reaches the predetermined threshold value, a time point slightly before that can be the timing most suitable for the subculture. Therefore, the predetermined threshold value can be set as a value for which a margin value α is added to "the local minimum of the secondary time-dependent change index value ("the local minimum of the secondary time-dependent change index value" + "margin value α"). Thus, it is possible to determine whether or not the subculture timing is reached in the excellent cell state where the spreading or the proliferation of the cells is not slowing down at a time point extremely close to the subculture timing. Similarly to the second embodiment, the "margin value α" is also set by the test as the examination conducted in advance.

### <Second example>

Next, the second example will be described with reference to FIG. 4B. In the first example (FIG. 4A), after the primary information is obtained at each photographing time point in the primary information obtaining step (S1221), the statistical index value at each photographing time point is calculated from the primary information at each photographing point in the statistical index value obtaining step (S1222). Then, after calculating the primary time-dependent change index value in the primary time-dependent change calculation step (S1223), the secondary time-dependent change index value is calculated to be the determination data in the secondary time-dependent change calculation step (S1224). However, in the second example, after the primary information at each photographing time point is obtained in the primary information obtaining step (S1221), the primary time-dependent change calculation step (S1223) is executed to calculate the time-dependent change index value of the primary information, and then the secondary time-dependent change index value is calculated. That is, it is different from the first example in that the primary time-dependent change calculation step (S1223) and the secondary time-dependent change calculation step (S1224) are combined and moved immediately after the primary information obtaining step (S1221). Then, thereafter, the statistical index value of the secondary time-dependent change index value is calculated to be the determination data in the statistical index value obtaining step (S1222). The primary information and the primary information obtaining step (S1221) are similar to the first embodiment and the first example and are therefore omitted.

(Primary time-dependent change calculation step (obtaining time-dependent change index value)) Following the primary information obtaining step (S1221), the primary time-dependent change index value of each primary information calculated at each photographing time point is calculated in the primary time-dependent change calculation step (S1223). While the primary time-dependent change index value of the statistical index value is obtained in the primary time-dependent change calculation step (S1223) in the first example, the primary time-dependent change index value of the primary information is obtained in the primary information obtaining step (S1221) in the second example. Therefore, when the statistical index value in the primary time-dependent change calculation step (S1223) in the first example is read as the primary information and the primary time-dependent change calculation step (S1223) in the first example is applied, the primary time-dependent change calculation step (S1223) in the second example is attained. The primary time-dependent change index value is the change amount of the primary information between the respective photographing time points.

In addition, the primary time-dependent change index value in the second example is, similarly to the first example, the difference change amount, the rate of amount of change, the rate of change per unit time, the change speed, or the time of change. The only difference is that, while it is the difference change amount, the rate of amount of change, the rate of change per unit time, the change speed, or the time of change of the statistical index value of the primary information in the first example, it is the difference change amount, the rate of amount of change, the rate of change per unit time, the change speed, or the time of change of the primary information in the second example. Therefore, while the primary time-dependent change index value is calculated from the statistical index value of the primary information in the calculation of the primary time-dependent change index value in the first example described above, "the statistical index value of the primary information" is simply read as "the primary information" and the first example may be applied to the second example. In the second example as well, similarly to the first example, the change speed can be applied as the specific growth rate, and the time of change can be applied as the multiplying time.

Further, this will be described with reference to FIG. 4H and FIG. 4I. FIG. 4H and FIG. 4I are diagrams illustrating the concept of the primary time-dependent change which is the time-dependent change of the primary information (vertical axis) to the time (horizontal axis) for the cases of the first example and the second example. FIG. 4H illustrates the case where the primary time-dependent change index value is the change amount or the rate of change, and FIG. 4I illustrates the case where the primary time-dependent change index value is the change amount or the rate of change per unit time. Both of FIG. 4H and FIG. 4I simultaneously illustrate the primary time-dependent change index value calculated in the primary time-dependent change calculation step (S1223) described here and the secondary time-dependent change index value calculated in the secondary time-dependent change calculation step (S1224) to be described next. In the second example, a₁ and a₂ as the statistical index values in the description of the primary time-dependent change calculation step (S1223) in the first example and FIG. 4H and FIG. 4I may be read as the value of the primary information obtained in the primary information obtaining step (S1221). Since one piece of the primary information is obtained from two pieces of the primary information, for the primary time-dependent change index value, the number of the primary time-dependent change index values corresponding to the number for which one is subtracted from the number of pieces of the primary information are obtained. The primary time-dependent change index value in the second example is a value directly indicating a time-dependent change trend of the primary information.

(Secondary time-dependent change calculation step (obtaining time-dependent change index value)) Following the primary time-dependent change calculation step (S1222), the secondary time-dependent change index value is calculated in the secondary time-dependent change calculation step (S1223). In the second example, the statistical index value in the first example is read as the primary information and a process similar to the first example is applied. The secondary time-dependent change index value is, similarly to the first example, the change amount or the rate of change which is the increment of the primary time-dependent change index value. That is, it is the change amount of the primary time-dependent change index value for the time from the first photographing time point to the second photographing time point and each primary time-dependent change index value for the time from the second photographing time point to the third photographing time point. This will be described with reference to FIG. 4H and FIG. 4I. FIG. 4H and FIG. 4I are the diagrams illustrating the concept of the change of the primary information (vertical axis) to the time (horizontal axis) for the cases of the first example and the second example. Both of FIG. 4H and FIG. 4I illustrate the secondary time-dependent change index value calculated in the secondary time-dependent change calculation step (S1224) together with the primary time-dependent change index value. In the secondary time-dependent change calculation step (S1224) in the second example, since the secondary time-dependent change index value is obtained by the primary time-dependent change calculation step (S1223), similarly to the primary time-dependent change calculation step (S1223), when the statistical index value in the primary time-dependent change calculation step (S1223) in the first example is read as the primary information and the secondary time-dependent change calculation step (S1224) in the first example is applied, the secondary time-dependent change calculation step (S1224) in the second example is attained. It is similar to the first example that one secondary time-dependent change index value is created for two primary time-dependent change index values so that the secondary time-dependent change index values one fewer than the number of the primary time-dependent change index values is created and the secondary time-dependent change index values two fewer than the statistical index values of the primary information are created as the whole.

### (Obtaining statistical index value)

After the secondary time-dependent change index value is calculated by the secondary time-dependent change calculation step (S1224), the statistical index value at each photographing time point is calculated from the primary information at each photographing time point in the statistical index value obtaining step (S1222). The statistical index value in the second example is the index value defined as a representative value typically indicative of the secondary time-dependent change index value. The statistical index value is, similarly to the first embodiment and the second embodiment, at least one of the average value, the variance and the deviation such as a standard deviation. In the second example, since the statistical index value of the secondary time-dependent change index value is calculated, it is the average value, the variance, or the deviation or the like of the secondary time-dependent change index value at each of the plurality of observation positions Op. With the statistical index value obtained here as the determination data, the subculture timing is determined. The plurality of statistical index values may be the same number of pieces of the determination data, or the plurality of statistical index values may be turned to one statistical index value by the average value, the maximum value, or the minimum value, and it may be defined as one piece of the determination data. The statistical index value of the secondary time-dependent change index value calculated in the statistical index value obtaining step (S1222) in the second example has a property and technical significance similar to that of the determination data which is the statistical index value of the time-dependent change index value in the second example of the second embodiment.

### (Subculture determination)

After the creation of the determination data is completed in the determination data creation step (S12), the determination step (S13) of determining whether or not the subculture timing is reached is executed. In the determination step (S13), the subculture timing is determined based on the statistical index value created in the determination data creation step (S12). Since the determination data in the second example includes the primary information, the time-dependent change element which is the primary time-dependent change index value and the secondary time-dependent change index value and the element of the statistical index value as the statistical index value of the secondary time-dependent change index value, it is the same as the second example of the second embodiment and the first example of the present embodiment as the determination data for determining the subculture timing, and is effective when the subculture timing is determined. The determination data in the second example means the representative value of the acceleration of the change of the primary information in the case where the sum or the average value is selected as the statistical index value, and means the dispersion of the acceleration of the change of the primary information in the case where the variance or the deviation is selected as the statistical index value.

In the determination step (S13), the statistical index value calculated in the statistical index value obtaining step (S1222) and the predetermined threshold value are compared. The predetermined threshold value is similar to that in the first example. Similarly to the first embodiment, a test as an examination is actually conducted in advance under the same condition as the determination of the subculture timing, the statistical index value at the time point to be the subculture timing is obtained in the test, the time-dependent change index value is obtained, and it is defined as the predetermined threshold value. Even if the time-dependent change element is added, the characteristics that the statistical index value has in the determination of the subculture timing are not lost, and it is just as described in the first embodiment.

In the case of the creation as the representative value of 1 for the number of pieces of the determination data, when the number of pieces of the determination data is defined as one, whether or not the one piece of the determination data has reached the predetermined threshold value is compared, and when the predetermined threshold value is reached, it is determined that the subculture timing is reached. In the case where the number of pieces of the determination data is two or more, whether or not the predetermined number of pieces of the determination data among the two or more pieces of the determination data have reached the predetermined threshold value is compared, and when the predetermined threshold value is reached, it can be determined that the subculture timing is reached. In the second example of the third embodiment as well, at the subculture timing, some inflection point represented by the extremal value such as the local minimum or the local maximum in the change trend of the statistical index value is observed. A time point at which such an inflection point of the change trend is observed is defined as the time point at which the subculture timing is reached, and is set as the threshold value.

Further, it may be determined that the subculture timing is reached in the case where all the pieces of the determination data of the two or more pieces of the determination data have reached the predetermined threshold value. In this case, since the time is needed for all the pieces of the determination data to reach the predetermined threshold value, whether or not the subculture timing is reached is determined relatively late.

Conversely, it may be determined that the subculture timing is reached in the case where at least one piece of the determination data among the two or more pieces of the determination data has reached the predetermined threshold value. In this case, since determination can be made when one piece of the determination data reaches the predetermined threshold value, whether or not the subculture timing is reached can be determined early.

### <Third example>

Next, the third example will be described with reference to FIG. 4C. In the third example (FIG. 4C), after the primary information is obtained at each photographing time point in the primary information obtaining step (S1221), the primary time-dependent change index value is calculated in the primary time-dependent change calculation step (S1223), and the statistical index value at each photographing time point is calculated from the primary information at each photographing time point in the statistical index value obtaining step (S1222) thereafter. Then, the secondary time-dependent change index value of the statistical index value is calculated to be the determination data in the secondary time-dependent change calculation step (S1224). That is, the statistical index value obtaining step (S1222) is executed to calculate the statistical index value at each photographing time point from the primary information between the primary time-dependent change calculation step (S1223) and the secondary time-dependent change calculation step (S1224), and the secondary time-dependent change index value of the statistical index value is calculated in the secondary time-dependent change calculation step (S1224). Hereinafter, this will be described. The primary information and the primary information obtaining step (S1221) are similar to the first embodiment and are therefore omitted.

(Primary time-dependent change calculation step (obtaining time-dependent change index value)) Following the primary information obtaining step (S1221), the primary time-dependent change index value of each primary information calculated at each photographing time point is calculated in the primary time-dependent change calculation step (S1223). Since processes in the primary information obtaining step (S1221) and the primary time-dependent change calculation step (S1223) are similar to that in the second example (FIG. 4B), the detailed description is omitted. In the third example as well, a₁ and a₂ as the statistical index values in the description of the primary time-dependent change calculation step (S1223) in the first example and FIG. 4H and FIG. 4I may be read as the value of the primary information obtained in the primary information obtaining step (S1221).

### (Obtaining statistical index value)

After the primary time-dependent change index value of the primary information is calculated by the primary time-dependent change calculation step (S1223), the statistical index value of the primary time-dependent change index value is calculated in the statistical index value obtaining step (S1222). The statistical index value in the third example is the index value defined as a representative value typically indicative of the primary time-dependent change index value. The statistical index value is, similarly to the first embodiment and the second embodiment, at least one of the average value, the variance and the deviation such as the standard deviation. In the third example, the statistical index value of the difference change amount, the rate of amount of change, the rate of change per unit time, the change speed or the time of change which is the primary time-dependent change index value is obtained. That is, in the third example, since the statistical index value of the primary time-dependent change index values is calculated, it is the average value, the variance or the deviation or the like of the difference change amount, the rate of amount of change, the rate of change per unit time, the change speed or the time of change of the primary information at each of the plurality of observation positions Op.

(Secondary time-dependent change calculation step (obtaining time-dependent change index value)) Following the statistical index value obtaining step (S1222), the secondary time-dependent change index value is calculated in the secondary time-dependent change calculation step (S1224). In the third example, the primary time-dependent change index value in the second example is read as the statistical index value, the process similar to the secondary time-dependent change calculation step (S1224) in the second example is applied, and the secondary time-dependent change obtaining step (S1224) is applied. In the third example, the secondary time-dependent change index value is the difference change amount, the rate of amount of change, the rate of change per unit time, the change speed or the time of change (secondary time-dependent change index value) of the average value, the variance and the deviation (statistical index values) of the difference change amount, the rate of amount of change, the rate of change per unit time, the change speed or the time of change (primary time-dependent change index value) of the primary information. With the statistical index value as the determination data, the subculture timing is determined. The plurality of statistical index values may be the same number of pieces of the determination data, or the plurality of statistical index values may be turned to one statistical index value by the average value, the maximum value, or the minimum value, and it may be defined as one piece of the determination data.

### (Subculture determination)

After the creation of the determination data is completed in the determination data creation step (S12), the determination step (S13) of determining whether or not the subculture timing is reached is executed. In the determination step (S13), the subculture timing is determined based on the statistical index value created in the determination data creation step (S12). Since the determination data in the third example is the secondary time-dependent change index value of the statistical index value, similarly to the first example and the second example, it is no different in that the primary information, the time-dependent change elements that are the primary time-dependent change index value and the secondary time-dependent change index value, and the element of the statistical index value are included, so that it is the same as the second embodiment and the first example and the second example of the present embodiment as the determination data for determining the subculture timing, and is effective when the subculture timing is determined. The determination data in the third example means the speed of the representative value of the speed of the change of the primary information in the case where the sum or the average value is selected as the statistical index value, and means the speed of the dispersion of the speed of the change of the primary information in the case where the variance or the deviation is selected as the statistical index value.

In the determination step (S13), the secondary time-dependent change index value calculated in the secondary time-dependent change calculation step (S1224) and the predetermined threshold value are compared. The predetermined threshold value is similar to that in the first example. Similarly to the first embodiment, a test as an examination is actually conducted in advance under the same condition as the determination of the subculture timing in a same manner as the process in the third example (FIG. 4C), the primary time-dependent change index value of the primary information at the time point to be the subculture timing is calculated in the test, the statistical index value is calculated, the secondary time-dependent change index value of the statistical index value is calculated, and it is defined as the predetermined threshold value. Even if the time-dependent change element is added, the characteristics that the statistical index value has in the determination of the subculture timing are not lost, and it is just as described in the first embodiment.

In the case of the creation as the representative value of 1 for the number of pieces of the determination data, when the number of pieces of the determination data is defined as one, whether or not the one piece of the determination data has reached the predetermined threshold value is compared, and when the predetermined threshold value is reached, it is determined that the subculture timing is reached. In the case where the number of pieces of the determination data is two or more, whether or not the predetermined number of pieces of the determination data among the two or more pieces of the determination data have reached the predetermined threshold value is compared, and when the predetermined threshold value is reached, it can be determined that the subculture timing is reached.

In the third example of the third embodiment as well, at the subculture timing, some inflection point represented by the extremal value such as the local minimum or the local maximum in the time variation of the secondary time-dependent change index value is observed. A time point at which such an inflection point of the time variation is observed is defined as the time point at which the subculture timing is reached, and is set as the threshold value. Further, it may be determined that the subculture timing is reached in the case where all the pieces of the determination data of the two or more pieces of the determination data have reached the predetermined threshold value. In this case, since the time is needed for all the pieces of the determination data to reach the predetermined threshold value, whether or not the subculture timing is reached is determined relatively late.

Conversely, it may be determined that the subculture timing is reached in the case where at least one piece of the determination data among the two or more pieces of the determination data has reached the predetermined threshold value. In this case, since determination can be made when one piece of the determination data reaches the predetermined threshold value, whether or not the subculture timing is reached can be determined early.

### [Fourth embodiment]

Next, as the fourth embodiment, the subculture timing determination method and the system will be described with reference to FIG. 5A to FIG. 5B. FIG. 5A is a diagram illustrating the concept of i pieces of the reference photographing time points (i = a natural number from 1 to m) and a value Ri (i = a natural number from 1 to m) corresponding to their primary information and j pieces of the evaluation photographing time points (j = a natural number from 1 to n) and a value Vj (j = a natural number from 1 to n) corresponding to their primary information, which are the notable photographing time points in the fourth embodiment.

In the third embodiment, the time-dependent change index value is calculated between one reference photographing time point and each of two or more evaluation photographing time points for the notable photographing time points. In contrast, in the fourth embodiment, the time-dependent change index value is calculated between the two or more reference photographing time points and two or more evaluation photographing time points respectively. The number of the reference photographing time points and the number of the evaluation photographing time points can be freely set to be one or more respectively. That is, as illustrated in FIG. 5A, i pieces of the reference photographing time points (i = a natural number from 1 to m) and j pieces of the evaluation photographing time points (j = a natural number from 1 to n) can be freely set as the notable photographing time points. Among them, an example in which one reference photographing time point and one evaluation photographing time point (j = a natural number from 1 to n) are set as the notable photographing time points is the second embodiment described above. In addition, an example in which one reference photographing time point and two evaluation photographing time points (j = a natural number from 1 to n) are set as the notable photographing time points is the third embodiment described above.

In the fourth embodiment, even though it is different from the third embodiment in that the plurality of reference photographing time points (i = a natural number from 1 to m) are used in particular, the third embodiment can be similarly applied except that the plurality of reference photographing time points (i = a natural number from 1 to m) are used. When applying the third embodiment in the fourth embodiment, there are two methods. One is a method (hereinafter, a reference value integration method) of attaining the representative values for the respective cases of the statistical index value (the case of the first example) based on the primary information obtained at the plurality of reference photographing time points (i = a natural number from 1 to m) and the primary information (the case of the second example and the third example) obtained at the plurality of reference photographing time points (i = a natural number from 1 to m), integrating them into one representative reference value, and applying the third embodiment when executing the primary time-dependent change index value (S1223). The other one is a method (hereinafter, a matrix time-dependent change method) of attaining a plurality of reference values without integrating them into one representative value for the respective cases of the statistical index value (the case of the first example) based on the primary information obtained at the plurality of reference photographing time points (i = a natural number from 1 to m) and the primary information (the case of the second example and the third example) obtained at the plurality of reference photographing time points (i = a natural number from 1 to m), and applying the third embodiment.

FIG. 5B illustrates the concept of the reference value integration method. In the reference value integration method, a representative reference value Rrep is created and the third embodiment described above is applied as follows. In the first example of the third embodiment, in the primary information obtaining step (S1221), the representative values are attained by a statistical method of an average value, a sum, or a weighting value or the like of the primary information obtained at the plurality of reference photographing time points (i = a natural number from 1 to m), and one representative reference value Rrep of the primary information is created. In addition, in the second example and the third example of the third embodiment, in the primary information obtaining step (S1221), the representative values are attained by the statistical method of the average value, the sum, or the weighting value or the like of the statistical index value of the primary information obtained at the plurality of reference photographing time points (i = a natural number from 1 to m), and one representative reference value Rrep of the primary information is created. At the time, statistical processing continues since the statistical index value of the primary information is also the average value, the sum, or the weighting value or the like, however, the representative value attaining for creating the representative reference value and the statistical index value of the primary information have technically different significances. The former is the processing for integrating the number of values, the number corresponding to the plurality of reference photographing time points (i = a natural number from 1 to m), into one representative value, and the latter is the processing for just creating the statistical index value indicating the state of the primary information of the cells. After two or more pieces of the value Ri (i = a natural number from 1 to m) based on the primary information obtained at the plurality of reference photographing time points (i = a natural number from 1 to m) are integrated into one representative reference value Rrep, the third embodiment may be applied as it is according to the respective descriptions of the first example to the third example so as to calculate the primary time-dependent change index values (Rrep, V1)...(Rrep, Vj) ... (Rrep, Vj) between j pieces of the evaluation photographing time points (j = a natural number from 1 to n) and the value Vj (j = a natural number from 1 to n) corresponding to their primary information.

In the matrix time-dependent change method, differently from the reference value integration method, the third embodiment may be applied as it is according to each of the first example to the third example, based on the number of pieces of the primary information, the number corresponding to the number of the reference photographing time points (i = a natural number from 1 to m), without attaining one reference value as the representative value.

FIG. 5C illustrates the concept of the matrix time-dependent change method. That is, the concept is illustrated in which the primary time-dependent change index value is calculated from the value Ri (i = a natural number from 1 to m) corresponding to the primary information at the respective reference photographing time points (i = a natural number from 1 to m) and the value Vj (j = a natural number from 1 to n) corresponding to the primary information at the respective evaluation photographing time points (j = a natural number from 1 to n).

In the matrix time-dependent change method, differently from the reference value integration method, the third embodiment may be applied as it is according to each of the first example to the third example, based on the number of pieces of the primary information, the number corresponding to the number of the reference photographing time points (i = a natural number from 1 to m), without attaining one representative reference value Rrep as the representative value. For example, it is assumed that a value derived from the primary information at each of the reference photographing time points (i = a natural number from 1 to m) is Ri (i = a natural number from 1 to m). The value Ri (i = a natural number from 1 to m) derived from the primary information means the statistical index value in the first example of the third embodiment, and the primary time-dependent change index value in the second example and the third example of the third embodiment. In the first example to the third example of the third embodiment, the third embodiment is applied so as to calculate sets (R1, V1)...(R1, Vj)...(R1, Vn) based on R1, which are m×n pieces of primary time-dependent change index values in the next step, sets (Ri, V1)...(Ri, Vj)...(Ri, Vn) based on Ri (i = 1 to m), and sets (Rm, V1)...(Rm, Vj)...(Rm, Vn) based on Rm, respectively, for each of m pieces of the value Ri (i = a natural number from 1 to m) with each of N pieces of the value Vj (j = a natural number from 1 to n), and to calculate the secondary time-dependent change index value from each of them. In the third embodiment, since there is one reference photographing time point, the primary time-dependent change index values are generated for the number of the evaluation photographing time points, and the secondary time-dependent change index values one fewer than them are generated. That is, in the case of the evaluation photographing time points (i = a natural number from 1 to n), n pieces of the primary time-dependent change index values and (n-1) pieces of the secondary time-dependent change index values are generated. In the matrix time-dependent change method in the fourth embodiment, index values for which they are multiplied with m pieces, that is (m×n) pieces of the primary time-dependent change index values and m·(n-1) pieces of the secondary time-dependent change index values are generated.

In other words, when it is assumed that the value derived from the primary information at each of the evaluation photographing time points (j = a natural number from 1 to n) is Vi (i = a natural number from 1 to m), the first example to the third example of the third embodiment are applied between the value Ri (i = a natural number from 1 to m) derived from the primary information at each of the reference photographing time points (i = a natural number from 1 to m) and the value Vj (j = a natural number from 1 to m) derived from the primary information at each of the evaluation photographing time points (j = a natural number from 1 to n). The value Vj (j = a natural number from 1 to n) derived from the primary information also means the statistical index value in the first example of the third embodiment and the primary time-dependent change index values in the second example and the third example of the third embodiment. That is, the case where the reference photographing time point is one point (i = 1) and the evaluation photographing time points are three points (j = 3) is the third embodiment, R₁=a₁ and V₁ = a₂ and R₁ = a₁ and V₂ = a₃ are defined in the case of the reference comparison method, and R₁ = a₁ and V₁ = a₂ and R₂ = a₂ and V₂ = a₃ are defined in the case of the nearest comparison method.

In the matrix time-dependent change method, in either of the first example and the third example of the third embodiment, the secondary time-dependent change index value is created based on the number of the value corresponding to the plurality of reference photographing time points. In the determination step (S13) of determining whether or not the subculture timing is reached, the predetermined threshold value is set as follows.

In the matrix time-dependent change method in the fourth embodiment as well, the pre-examination is conducted under the same condition in advance before actually determining the subculture timing, and the predetermined threshold value is determined from a result at the time point which is the subculture timing in the examination. In the examination, since it is conducted under the same condition as the actual subculture timing determination, the number of the reference photographing time points (i = a natural number from 1 to m) and the number of the evaluation photographing time points (j = a natural number from 1 to n) are also set to be the same. Therefore, when applying the first example or the third example of the third embodiment, in the examination as well, the sets (R1, V1)...(R1, Vj)... (R1, Vn) based on R1, the sets (Ri, V1)...(Ri, Vj) ... (Ri, Vn) based on Ri (i = 1 to m), and the sets (Rm, V1)...(Rm, Vj)... (Rm, Vn) based on Rm are calculated respectively as the primary time-dependent change index values, the secondary time-dependent change index values are calculated from each of them, and the determination data is created. Then, the determination data at the time point at which it is determined that the subculture timing is reached is created. In this, for the secondary time-dependent change index value (the first example and the third example) and the statistical index value (the second example) of the secondary time-dependent change index value as the determination data, the plurality of pieces of the determination data are created, respectively. However, even when there is the plurality of reference photographing time points, the inflection point is observed in each of the change trends of the secondary time-dependent change index value (the first example and the third example) and the statistical index value (the second example) of the secondary time-dependent change index value, and the vicinity thereof is defined as the predetermined threshold value when determining the subculture timing. In particular, in the case of setting the plurality of reference photographing time points, a pattern having the inflection point is observed also in the change trends themselves of the sets based on R1, the sets based on Ri (i = 1 to m) and the sets based on Rm so that more accurate determination than the second embodiment and the third embodiment having the time-dependent change element is possible.

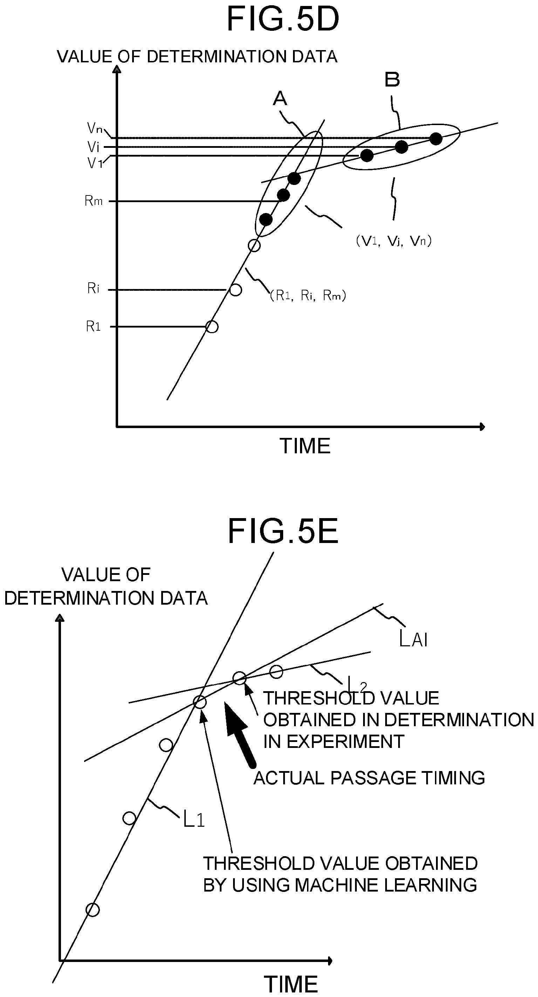

Next, another form which is a modification of the fourth embodiment will be described with reference to FIG. 5D. FIG. 5D illustrates a value change trend of a value (vertical axis) of the determination data (white circles and black circles) that changes with progress (subculture of "time" which is a horizontal axis) of the culture. In FIG. 5D, the white circle is the value corresponding to the reference photographing time point, and the black circle is the value corresponding to the evaluation photographing time point. When creating the determination data in the determination data creation step (S12), in a stage where the spreading or the proliferation of the cells is advancing before the subculture timing comes, the increasing trend of the value corresponding to the reference photographing time point and the increasing trend of the value corresponding to the evaluation photographing time point are within a range of fixed increasing trend dispersion (a group A of the values corresponding to the evaluation photographing time points in FIG. 5D). However, when the subculture timing comes, the increasing trend of the value corresponding to the reference photographing time point and the increasing trend of the value corresponding to the evaluation photographing time point are large and change in the increasing trend is observed. (A group B of the values corresponding to the evaluation photographing time points in FIG. 5D). Therefore, in the creation of the determination data, the evaluation photographing time point at which the trend of the group B of the values corresponding to the evaluation photographing time points in FIG. 5D starts to show is determined as the subculture timing. The time point at which the trend of the group B of the values corresponding to the evaluation photographing time points in FIG. 5D starts to show is the inflection point of the change trend of the determination data.

In this case, in the determination data creation step (S12), it is also possible to determine the subculture timing by the stage where the inflection point of the change trend of the determination data is observed. This can be applied to the first embodiment. On the other hand, the test as the examination may be actually conducted in advance under the same condition as the determination of the subculture timing, the predetermined threshold value may be defined by the stage where the inflection point of the change trend of the determination data is observed in the examination test, and the first embodiment to the fourth embodiment may be applied.

### [Fifth embodiment]

Next, the fifth embodiment will be described with reference to FIG. 5E. FIG. 5E illustrates the value change trend of the value (vertical axis) of the determination data (white circles) that changes with the progress (subculture of "time" which is the horizontal axis) of the culture. The fifth embodiment is the embodiment as a variation of the second embodiment to the fourth embodiment utilizing the time-dependent change element for the determination step (S13) of the subculture timing determination described in the first embodiment to the fourth embodiment. That is, in the fifth embodiment, the respective steps in the second embodiment to the fourth embodiment are applied as they are for the steps other than the determination step (S13), and the determination step (S13) in the fifth embodiment which is the present embodiment described below is applied for the determination step (S13) in each of the second embodiment to the fourth embodiment.

In the determination step (S13) in the fifth embodiment, the form is to utilize machine learning or deep learning in the determination of the predetermined threshold value. A mode itself of the machine learning and the deep learning is not limited, and all the widely general machine learning or deep learning can be used. Here, the machine learning and the deep learning themselves are not characteristic, and by using the machine learning and the deep learning in the determination of the subculture timing in the first embodiment to the fourth embodiment of the present claimed invention, the subculture timing can be further timely determined in addition to the second embodiment to the fourth embodiment.

FIG. 5E illustrates the change trend with the number of cells as the primary information and the sum as the statistical index value in the first embodiment to the fourth embodiment as examples. In this case, the increasing trend is shown until the subculture timing, and the inflection point of the change trend is provided at a position at which the subculture timing comes. Then, a saturated state of the change trend in which increase stops is illustrated after the subculture timing comes. The change trend of the other primary information and the other statistical index value in the first embodiment to the fourth embodiment is different from that illustrated in FIG. 5, but is the same in that the inflection point of the change trend is provided at the position at which the subculture timing comes. Hereinafter, as the description of the fifth embodiment, it should be understood as a common point that the inflection point of the change trend is observed at the position at which the subculture timing comes and the change trend becomes different before and after that, for the change trend illustrated in FIG. 5E.

The time point to be the actual subculture timing is around the part indicated with a thick arrow in FIG. 5E. Around this part, the change trend of the determination data (white circles) is the inflection point. However, the timing of the determination is conducted discretely at every predetermined time in the progress of the culture, the timing of the determination cannot be the time point coinciding with the inflection point. In the case of the second embodiment to the fourth embodiment utilizing the time-dependent change element, when defining the predetermined threshold, the test as the examination is actually conducted in advance under the same condition as the determination of the subculture timing and the predetermined threshold value is determined from the result of the test. In the fifth embodiment as well, the test as the examination is actually conducted in advance under the same condition as the determination of the subculture timing, the machine learning or the deep learning is applied to the result of the test, and the predetermined threshold value is determined.

In the second embodiment to the fourth embodiment, in the determination of the result of the test of the examination, pre-determination is made based on a feeling of an experienced user so to speak. There, in FIG. 5E, a line L₁ indicating the change trend in the stage where the culture is advancing and a line L₂ indicating the change trend after the culture advances and the subculture timing comes are instinctively determined, and the time point of the inflection point around an intersection of the line L₁ indicating the change trend in the stage where the culture is advancing is determined as the time point of the subculture timing. However, in the fifth embodiment in which the machine learning or the deep learning is applied, it is possible to capture the change trend that even the experienced user cannot recognize. For example, conceptionally, it is possible to derive the change trend that even the experienced user cannot recognize, such as a line L_{AI} indicating the change trend in FIG. 5E, and to derive the time point of the subculture timing in advance that cannot be obtained without using the machine learning or the deep learning. In the fifth embodiment, the time point is defined as the predetermined threshold value.

It is similar to the second embodiment to the fourth embodiment that it is determined that the subculture timing is reached when the determination data reaches the predetermined threshold value in the determination step (S13).

### Reference Signs List

1 cell culture system
2 culture medium unit
3 subculture timing determination unit
4 culture unit
5 cell inoculation apparatus
6 transfer unit
7 centrifugal apparatus
8 processing apparatus
31 camera (image-capturing means)
32 photographing stage
33 processing means
61 moving table
62 arm
63 actuator
Pe evaluation culture vessel

## Claims

1. A subculture timing determination method of determining a subculture timing of cells spreading or proliferating in a culture medium in a culture vessel, the subculture timing determination method comprising:
an image-capturing step of photographing the cells at a plurality of predetermined points of the culture vessel at a first photographing time point to obtain the cells as a cell image of each point of the plurality of predetermined points at the first photographing time point;
a primary information obtaining step of obtaining primary information of the cells photographed in the cell image of each point at the first photographing time point;
a statistical index value obtaining step of calculating a statistical index value of the primary information of the cells; and
a determination step of performing determination that a photographing time point corresponding to a time point at which the statistical index value reaches a predetermined threshold value is a time point at which the subculture timing is reached.

2. A subculture timing determination method of
determining a subculture timing of cells spreading or proliferating in a culture medium in a culture vessel, the subculture timing determination method comprising:
an image-capturing step of photographing the cells at a plurality of predetermined points of the culture vessel at a first photographing time point and a second photographing time point after the first photographing time point to obtain the cells as a cell image of each point of the plurality of predetermined points at each of the first photographing time point and the second photographing time point;
a primary information obtaining step of obtaining primary information of the cells photographed in the cell image of the each point at each of the first photographing time point and the second photographing time point;
a statistical index value obtaining step of calculating a statistical index value of the primary information at each of the first photographing time point and the second photographing time point; and
a time-dependent change calculation step of calculating a time-dependent change index value from the statistical index value at the first photographing time point and the statistical index value at the second photographing time point,
the subculture timing determination method comprising:
a determination step of performing determination that a photographing time point corresponding to a time point at which the time-dependent change index value reaches a predetermined threshold value is a time point at which the subculture timing is reached.

3. A subculture timing determination method of
determining a subculture timing of cells spreading or proliferating in a culture medium in a culture vessel, the subculture timing determination method comprising:
an image-capturing step of photographing the cells at a plurality of predetermined points of the culture vessel at a first photographing time point and a second photographing time point after the first photographing time point to obtain the cells as a cell image of each point of the plurality of predetermined points at each of the first photographing time point and the second photographing time point;
a primary information obtaining step of obtaining primary information of the cells photographed in the cell image of the each point at each of the first photographing time point and the second photographing time point;
a time-dependent change calculation step of calculating a time-dependent change index value from the primary information at the first photographing time point and
the primary information at the second photographing time point;
a statistical index value obtaining step of calculating a statistical index value of the time-dependent change index value of each of the first photographing time point and the second photographing time point; and
a determination step of performing determination that a photographing time point corresponding to a time point at which the statistical index value reaches a predetermined threshold value is a time point at which the subculture timing is reached.

4. A subculture timing determination method of
determining a subculture timing of cells spreading or proliferating in a culture medium in a culture vessel, the subculture timing determination method comprising:
an image-capturing step of photographing the cells at a plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point;
a primary information obtaining step of obtaining primary information of the cells photographed in the cell image of the each point at each of the first photographing time point and the second photographing time point and the third photographing time point;
a statistical index value obtaining step of calculating a statistical index value of the primary information at each of the first photographing time point and the second photographing time point and the third photographing time point;
a primary time-dependent change calculation step of calculating a primary time-dependent change index value of the statistical index value at the first photographing time point and the statistical index value at the second photographing time point, and a primary time-dependent change index value of the statistical index value at the second photographing time point and the statistical index value at the third photographing time point;
a secondary time-dependent change calculation step of calculating a secondary time-dependent change index value from the primary time-dependent change index value of the first photographing time point and the second photographing time point, and the primary time-dependent change index value of the second photographing time point and the third photographing time point; and
a determination step of performing determination that a photographing time point corresponding to a time point at which the secondary time-dependent change index value reaches a predetermined threshold value is a time point at which the subculture timing is reached.

5. A subculture timing determination method of
determining a subculture timing of cells spreading or proliferating in a culture medium in a culture vessel, the subculture timing determination method comprising:
an image-capturing step of photographing the cells at a plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point;
a primary information obtaining step of obtaining primary information of the cells photographed in the cell image of the each point at each of the first photographing time point and the second photographing time point and the third photographing time point;
a primary time-dependent change calculation step of calculating a primary time-dependent change index value of the primary information at the first photographing time point and the primary information at the second photographing time point, and a primary time-dependent change index value of the primary information at the second photographing time point and the primary information at the third photographing time point;
a secondary time-dependent change calculation step of calculating a secondary time-dependent change index value from the primary time-dependent change index value of the first photographing time point and the second photographing time point, and the primary time-dependent change index value of the second photographing time point and the third photographing time point;
a statistical index value obtaining step of calculating a statistical index value of the secondary time-dependent change index value at each of the first photographing time point and the second photographing time point and the third photographing time point; and
a determination step of performing determination that a photographing time point corresponding to a time point at which the statistical index value reaches a predetermined threshold value is a time point at which the subculture timing is reached.

6. A subculture timing determination method of
determining a subculture timing of cells spreading or proliferating in a culture medium in a culture vessel, the subculture timing determination method comprising:
an image-capturing step of photographing the cells at a plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point;
a primary information obtaining step of obtaining primary information of the cells photographed in the cell image of the each point at each of the first photographing time point and the second photographing time point and the third photographing time point;
a primary time-dependent change calculation step of calculating a primary time-dependent change index value of the primary information at the first photographing time point and the primary information at the second photographing time point, and a primary time-dependent change index value of the primary information at the second photographing time point and the primary information at the third photographing time point;
a statistical index value obtaining step of calculating a statistical index value of the primary time-dependent change index value at each of the first photographing time point and the second photographing time point and the third photographing time point;
a secondary time-dependent change calculation step of calculating a secondary time-dependent change index value from the statistical index value of the first photographing time point and the statistical index value of the second photographing time point, and the statistical index value of the second photographing time point and the statistical index value of the third photographing time point; and
a determination step of performing determination that a photographing time point corresponding to a time point at which the secondary time-dependent change index value reaches a predetermined threshold value is a time point at which the subculture timing is reached.

7. A subculture timing determination method
according to claim 2,
wherein the time-dependent change index value is a difference change amount, a rate of amount of change, a rate of change per unit time, a change speed, a time of change, a specific growth rate, or a predetermined multiplying time of the statistical index value.

8. A subculture timing determination method
according to claim 4,
wherein the primary time-dependent change index value is a difference change amount, a rate of amount of change, a rate of change per unit time, a change speed, a time of change, a specific growth rate, or a predetermined multiplying time of the statistical index value.

9. A subculture timing determination method
according to claim 3, wherein the time-dependent change index value is a difference change amount, a rate of amount of change, a rate of change per unit time, a change speed, a time of change, a specific growth rate, a predetermined multiplying time of the primary information.

10. A subculture timing determination method
according to claim 5, wherein the primary time-dependent change index value is a difference change amount, a rate of amount of change, a rate of change per unit time, a change speed, a time of change, a specific growth rate, a predetermined multiplying time of the primary information.

11. A subculture timing determination method
according to claim 6, wherein the primary time-dependent change index value is a difference change amount, a rate of amount of change, a rate of change per unit time, a change speed, a time of change, a specific growth rate, a predetermined multiplying time of the primary information.

12. A subculture timing determination method
according to any one of claims 1 to 6,
wherein the primary information is any of a number of cells, a cell density, a cell occupation area, a single cell area, and a relative distance between cells in the cell image.

13. A subculture timing determination method
according to any one of claims 1 to 6,
wherein the statistical index value is an average value.

14. A subculture timing determination method
according to any one of claims 1 to 6,
wherein the statistical index value is a sum.

15. A subculture timing determination method
according to any one of claims 1 to 6,
wherein the statistical index value is a variance or a deviation.

16. A subculture timing determination method
according to any one of claims 2 to 6,
wherein a pre-examination is conducted in advance, and the predetermined threshold value is defined as a statistical index value at which spreading or proliferation of the cells is not observed in the pre-examination.

17. A subculture timing determination method
according to claim 15,
wherein a pre-examination is conducted in advance, and the predetermined threshold value is defined as an inflection point in time variation of the variance or the deviation in the pre-examination.

18. A subculture timing determination method
according to claim 7,
wherein the rate of amount of change is calculated as a value at any one photographing time point, and wherein a pre-examination is conducted in advance, and the predetermined threshold value is defined as a secondary time-dependent change index value at a time point at which the time-dependent change index value no longer shows an increasing trend in the pre-examination.

19. A subculture timing determination method
according to claim 7,
wherein the rate of amount of change is calculated as a ratio to a value at an immediately preceding photographing time point, and
wherein a pre-examination is conducted in advance, and the predetermined threshold value is defined as an inflection point of time variation of the time-dependent change index value in the pre-examination.

20. A subculture timing determination method
according to claim 8,
wherein the rate of amount of change is calculated as a value at any one photographing time point, and wherein a pre-examination is conducted in advance, and the predetermined threshold value is defined as a secondary time-dependent change index value at a time point at which the secondary time-dependent change index value no longer shows an increasing trend in the pre-examination.

21. A subculture timing determination method
according to claim 8,
wherein the rate of amount of change is calculated as a ratio to a value at an immediately preceding photographing time point, and
wherein a pre-examination is conducted in advance, and the predetermined threshold value is defined as an inflection point of time variation of the secondary time-dependent change index value in the pre-examination.

22. A subculture timing determination method
according to claim 9 or 10,
wherein the rate of amount of change is calculated as a value at any one photographing time point, and wherein a pre-examination is conducted in advance, and the predetermined threshold value is defined as a secondary time-dependent change index value at a time point at which the statistical index value no longer shows an increasing trend in the pre-examination.

23. A subculture timing determination method
according to claim 9 or 10,
wherein the rate of amount of change is calculated as a ratio to a value at an immediately preceding photographing time point, and
wherein a pre-examination is conducted in advance, and the predetermined threshold value is defined as an inflection point of time variation of the statistical index value in the pre-examination.

24. A subculture timing determination method
according to claim 11,
wherein the rate of amount of change is calculated as a value at any one photographing time point, and wherein a pre-examination is conducted in advance, and the predetermined threshold value is defined as a secondary time-dependent change index value at a time point at which the secondary time-dependent change index value no longer shows an increasing trend in the pre-examination.

25. A subculture timing determination method
according to claim 11,
wherein the rate of amount of change is calculated as a ratio to a value at an immediately preceding photographing time point, and
wherein a pre-examination is conducted in advance, and the predetermined threshold value is defined as an inflection point of time variation of the secondary time-dependent change index value in the pre-examination.

26. A subculture timing determination method
according to any one of claims 4 to 6,
wherein a time interval between the first photographing time point and the second photographing time point and a time interval between the second photographing time point and the third photographing time point are a same time interval.

27. A subculture timing determination method
according to any one of claims 1 to 6,
wherein a pre-examination is conducted in advance, and the predetermined threshold value is defined through machine learning in the pre-examination.

28. A subculture timing determination system that
determines a subculture timing of cells spreading or proliferating in a culture medium in a culture vessel, the subculture timing determination system comprising:
image-capturing means for photographing cells of a culture surface of each point of a plurality of predetermined points of the culture medium of the culture vessel to obtain the cells as cell images; and
a processing apparatus configured to obtain primary information of the cells photographed in the cell images,
wherein the image-capturing means photographs the cells at the plurality of predetermined points of the culture vessel at a first photographing time point to obtain the cells as a cell image of each point of the plurality of predetermined points at the first photographing time point,
wherein the processing apparatus obtains primary information of the cells photographed in the cell image of the each point at the first photographing time point to calculate a statistical index value of the primary information of the cells, and
wherein the processing apparatus performs determination that a photographing time point corresponding to a time point at which the statistical index value reaches a predetermined threshold value is a time point at which the subculture timing is reached.

29. A subculture timing determination system that
determines a subculture timing of cells spreading or proliferating in a culture medium in a culture vessel, the subculture timing determination system comprising:
image-capturing means for photographing cells of a culture surface of each point of a plurality of predetermined points of the culture medium of the culture vessel to obtain the cells as cell images; and
a processing apparatus configured to obtain primary information of the cells photographed in the cell images,
wherein the image-capturing means photographs the cells at the plurality of predetermined points of the culture vessel at a first photographing time point and a second photographing time point after the first photographing time point to obtain the cells as a cell image of each point of the plurality of predetermined points at each of the first photographing time point and the second photographing time point;
wherein the processing apparatus obtains primary information of the cells photographed in the cell image of the each point at each of the first photographing time point and the second photographing time point to calculate a statistical index value of the primary information at each of the first photographing time point and the second photographing time point, and
calculate a time-dependent change index value from the statistical index value at the first photographing time point and the statistical index value at the second photographing time point, and
wherein the processing apparatus performs determination that a photographing time point corresponding to a time point at which the time-dependent change index value reaches a predetermined threshold value is a time point at which the subculture timing is reached.

30. A subculture timing determination system that
determines a subculture timing of cells spreading or proliferating in a culture medium in a culture vessel, the subculture timing determination system comprising:
image-capturing means for photographing cells of a culture surface of each point of a plurality of predetermined points of the culture medium of the culture vessel to obtain the cells as cell images; and
a processing apparatus configured to obtain primary information of the cells photographed in the cell images,
wherein the image-capturing means photographs the cells at the plurality of predetermined points of the culture vessel at a first photographing time point and a second photographing time point after the first photographing time point to obtain the cells as a cell image of each point of the plurality of predetermined points at each of the first photographing time point and the second photographing time point,
wherein the processing apparatus obtains primary information of the cells photographed in the cell image of the each point at each of the first photographing time point and the second photographing time point to calculate a time-dependent change index value from the primary information at the first photographing time point and the primary information at the second photographing time point, and calculate a statistical index value of the time-dependent change index value at each of the first photographing time point and the second photographing time point, and
wherein the processing apparatus performs determination that a photographing time point corresponding to a time point at which the statistical index value reaches a predetermined threshold value is a time point at which the subculture timing is reached.

31. A subculture timing determination system that
determines a subculture timing of cells spreading or proliferating in a culture medium in a culture vessel, the subculture timing determination system comprising:
image-capturing means for photographing cells of a culture surface of each point of a plurality of predetermined points of the culture medium of the culture vessel to obtain the cells as cell images; and
a processing apparatus configured to obtain primary information of the cells photographed in the cell images,
wherein the image-capturing means photographs the cells at the plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point,
wherein the processing apparatus obtains primary information of the cells photographed in the cell image of the each point at each of the first photographing time point and the second photographing time point and
the third photographing time point to calculate a statistical index value of the primary information at each of the first photographing time point and the second photographing time point and the third photographing time point, calculate a primary time-dependent change index value of the statistical index value at the first photographing time point and the statistical index value at the second photographing time point, and a primary time-dependent change index value of the statistical index value at the second photographing time point and the statistical index value at the third photographing time point, and
calculate a secondary time-dependent change index value from the primary time-dependent change index value of the first photographing time point and the second photographing time point, and the primary time-dependent change index value of the second photographing time point and the third photographing time point, and
wherein the processing apparatus performs determination that a photographing time point corresponding to a time point at which the secondary time-dependent change index value reaches a predetermined threshold value is a time point at which the subculture timing is reached.

32. A subculture timing determination system that
determines a subculture timing of cells spreading or proliferating in a culture medium in a culture vessel, the subculture timing determination system comprising:
image-capturing means for photographing cells of a culture surface of each point of a plurality of predetermined points of the culture medium of the culture vessel to obtain the cells as cell images; and
a processing apparatus configured to obtain primary information of the cells photographed in the cell images,
wherein the image-capturing means photographs the cells at the plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and
the third photographing time point, obtain primary information of the cells photographed in the cell image of the each point at each of the first photographing time point and the second photographing time point and
the third photographing time point, calculate a primary time-dependent change index value of the primary information at the first photographing time point and
the primary information at the second photographing time point, and a primary time-dependent change index value of the primary information at the second photographing time point and the primary information at the third photographing time point, calculate a secondary time-dependent change index value from the primary time-dependent change index value of the first photographing time point and the second photographing time point, and the primary time-dependent change index value of the second photographing time point and the third photographing time point, and calculate a statistical index value of the secondary time-dependent change index value at each of the first photographing time point and the second photographing time point and the third photographing time point, and
wherein the processing apparatus performs determination that a photographing time point corresponding to a time point at which the statistical index value reaches a predetermined threshold value is a time point at which the subculture timing is reached.

33. A subculture timing determination system that
determines a subculture timing of cells spreading or proliferating in a culture medium in a culture vessel, the subculture timing determination system comprising:
image-capturing means for photographing cells of a culture surface of each point of a plurality of predetermined points of the culture medium of the culture vessel to obtain the cells as cell images; and
a processing apparatus configured to obtain primary information of the cells photographed in the cell images,
wherein the image-capturing means photographs the cells at the plurality of predetermined points of the culture vessel at a first photographing time point and a second photographing time point after the first photographing time point and a third photographing time point after the second photographing time point to obtain the cells as a cell image of each point of the plurality of predetermined points at each of the first photographing time point and the second photographing time point and the third photographing time point,
wherein the processing apparatus obtains primary information of the cells photographed in the cell image of the each point at each of the first photographing time point and the second photographing time point and
the third photographing time point, calculates a primary time-dependent change index value of the primary information at the first photographing time point and the primary information at the second photographing time point, and a primary time-dependent change index value of the primary information at the second photographing time point and the primary information at the third photographing time point,
calculates a statistical index value of the primary time-dependent change index value at each of the first photographing time point and the second photographing time point and the third photographing time point, and calculates a secondary time-dependent change index value from the statistical index value of the first photographing time point and the statistical index value of the second photographing time point, and the statistical index value of the second photographing time point and the statistical index value of the third photographing time point, and
wherein the processing apparatus performs determination that a photographing time point corresponding to a time point at which the secondary time-dependent change index value reaches a predetermined threshold value is a time point at which the subculture timing is reached.

34. A subculture timing determination system
according to claim 29,
wherein the time-dependent change index value is a difference change amount, a rate of amount of change, a rate of change per unit time, a change speed, a time of change, a specific growth rate, or a predetermined multiplying time of the statistical index value.

35. A subculture timing determination system
according to claim 31,
wherein the primary time-dependent change index value is a difference change amount, a rate of amount of change, a rate of change per unit time, a change speed, a time of change, a specific growth rate, or a predetermined multiplying time of the statistical index value.

36. A subculture timing determination system
according to claim 30,
wherein the time-dependent change index value is a difference change amount, a rate of amount of change, a rate of change per unit time, a change speed, a time of change, a specific growth rate, or a predetermined multiplying time of the primary information.

37. A subculture timing determination system
according to claim 32,
wherein the primary time-dependent change index value is a difference change amount, a rate of amount of change, a rate of change per unit time, a change speed, a time of change, a specific growth rate, or a predetermined multiplying time of the primary information.

38. A subculture timing determination system
according to claim 33,
wherein the primary time-dependent change index value is a difference change amount, a rate of amount of change, a rate of change per unit time, a change speed, a time of change, a specific growth rate, or a predetermined multiplying time of the primary information.

39. A subculture timing determination system
according to any one of claims 28 to 33,
wherein the primary information is any of a number of cells, a cell density, a cell occupation area, a single cell area, and a relative distance between cells in the cell image.

40. A subculture timing determination system
according to any one of claims 28 to 33,
wherein the statistical index value is an average value.

41. A subculture timing determination system
according to any one of claims 28 to 33,
wherein the statistical index value is a sum.

42. A subculture timing determination system
according to any one of claims 28 to 33,
wherein the statistical index value is a variance or a deviation.

43. A subculture timing determination system
according to any one of claims 29 to 33,
wherein a pre-examination is conducted in advance, and the predetermined threshold value is defined as a statistical index value at which spreading or proliferation of the cells is not observed in the pre-examination.

44. A subculture timing determination system
according to claim 42,
wherein a pre-examination is conducted in advance, and
the predetermined threshold value is defined as an inflection point of time variation of the variance or the deviation in the pre-examination.

45. A subculture timing determination system
according to claim 34,
wherein the rate of amount of change is calculated as a value at any one photographing time point, and
wherein a pre-examination is conducted in advance, and the predetermined threshold value is defined as a secondary time-dependent change index value at a time point at which the time-dependent change index value no longer shows an increasing trend in the pre-examination.

46. A subculture timing determination system
according to claim 34,
wherein the rate of amount of change is calculated as a ratio to a value at an immediately preceding photographing time point, and
wherein a pre-examination is conducted in advance, and the predetermined threshold value is defined as an inflection point of time variation of the time-dependent change index value in the pre-examination.

47. A subculture timing determination system
according to claim 35,
wherein the rate of amount of change is calculated as a value at any one photographing time point, and
wherein a pre-examination is conducted in advance, and the predetermined threshold value is defined as a secondary time-dependent change index value at a time point at which the secondary time-dependent change index value no longer shows an increasing trend in the pre-examination.

48. A subculture timing determination system
according to claim 35,
wherein the rate of amount of change is calculated as a ratio to a value at an immediately preceding photographing time point, and
wherein a pre-examination is conducted in advance, and the predetermined threshold value is defined as an inflection point of time variation of the secondary time-dependent change index value in the pre-examination.

49. A subculture timing determination system
according to claim 36 or 37,
wherein the rate of amount of change is calculated as a value at any one photographing time point, and
wherein a pre-examination is conducted in advance, and the predetermined threshold value is defined as a secondary time-dependent change index value at a time point at which the statistical index value no longer shows an increasing trend in the pre-examination.

50. A subculture timing determination system
according to claim 36 or 37,
wherein the rate of amount of change is calculated as a ratio to a value at an immediately preceding photographing time point, and
wherein a pre-examination is conducted in advance, and the predetermined threshold value is defined as an inflection point of time variation of the statistical index value in the pre-examination.

51. A subculture timing determination system
according to claim 38,
wherein the rate of amount of change is calculated as a value at any one photographing time point, and
wherein a pre-examination is conducted in advance, and the predetermined threshold value is defined as a secondary time-dependent change index value at a time point at which the secondary time-dependent change index value no longer shows an increasing trend in the pre-examination.

52. A subculture timing determination system
according to claim 38,
wherein the rate of amount of change is calculated as a ratio to a value at an immediately preceding photographing time point, and
wherein a pre-examination is conducted in advance, and the predetermined threshold value is defined as an inflection point of time variation of the secondary time-dependent change index value in the pre-examination.

53. A subculture timing determination system
according to any one of claims 31 to 33,
wherein a time interval between the first photographing time point and the second photographing time point and a time interval between the second photographing time point and the third photographing time point are a same time interval.

54. A subculture timing determination system
according to any one of claims 28 to 33,
wherein a pre-examination is conducted in advance, and the predetermined threshold value is defined through machine learning in the pre-examination.

55. A subculture timing determination system
according to any one of claims 28 to 33,
wherein the processing apparatus is capable of communicating with the image-capturing means, and is arranged apart from the image-capturing means.

56. A cell culture system for cells spreading or
proliferating in a culture medium in a culture vessel, the cell culture system comprising:
a subculture timing determination system according to claim 28; and
at least one of a culture medium unit configured to replace the culture medium of the culture vessel, a culture unit configured to store the culture vessel at a predetermined temperature, and a transfer unit configured to transfer the culture vessel between the subculture timing determination system and the culture unit.

57. A cell culture system for cells spreading or
proliferating in a culture medium in a culture vessel, the cell culture system comprising:
a subculture timing determination system according to claim 29; and
at least one of a culture medium unit configured to replace the culture medium of the culture vessel, a culture unit configured to store the culture vessel at a predetermined temperature, and a transfer unit configured to transfer the culture vessel between the subculture timing determination system and the culture unit.

58. A cell culture system for cells spreading or
proliferating in a culture medium in a culture vessel, the cell culture system comprising:
a subculture timing determination system according to claim 30; and
at least one of a culture medium unit configured to replace the culture medium of the culture vessel, a culture unit configured to store the culture vessel at a predetermined temperature, and a transfer unit configured to transfer the culture vessel between the subculture timing determination system and the culture unit.

59. A cell culture system for cells spreading or
proliferating in a culture medium in a culture vessel, the cell culture system comprising:
a subculture timing determination system according to claim 31; and
at least one of a culture medium unit configured to replace the culture medium of the culture vessel, a culture unit configured to store the culture vessel at a predetermined temperature, and a transfer unit configured to transfer the culture vessel between the subculture timing determination system and the culture unit.

60. A cell culture system for cells spreading or
proliferating in a culture medium in a culture vessel, the cell culture system comprising:
a subculture timing determination system according to claim 32; and
at least one of a culture medium unit configured to replace the culture medium of the culture vessel, a culture unit configured to store the culture vessel at a predetermined temperature, and a transfer unit configured to transfer the culture vessel between the subculture timing determination system and the culture unit.

61. A cell culture system for cells spreading or
proliferating in a culture medium in a culture vessel, the cell culture system comprising:
a subculture timing determination system according to claim 33; and
at least one of a culture medium unit configured to replace the culture medium of the culture vessel, a culture unit configured to store the culture vessel at a predetermined temperature, and a transfer unit configured to transfer the culture vessel between the subculture timing determination system and the culture unit.
